(19) Europäisches Patentamt
European Patent Office
Office européen des brevets

(11) **EP 4 574 201 A2**

(12) **EUROPEAN PATENT APPLICATION**

(43) Date of publication:
**25.06.2025 Bulletin 2025/26**

(21) Application number: **25175734.0**

(22) Date of filing: **30.06.2016**

(51) International Patent Classification (IPC):
**A61N 1/36** (2006.01)

(52) Cooperative Patent Classification (CPC):
**A61B 18/14; A61N 2/002; A61N 2/004;**
**A61N 2/006; A61N 2/02; H01F 7/064;**
A61B 18/1206; A61B 2018/00452; A61N 1/06;
A61N 1/328; A61N 1/36014

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**

(30) Priority: **01.07.2015 US 201514789156**
**01.07.2015 US 201514789658**
**29.10.2015 US 201514926365**
**24.11.2015 US 201514951093**
**17.03.2016 US 201615073318**
**14.04.2016 US 201615099274**
**10.05.2016 US 201615151012**
**09.06.2016 US 201615178455**

(62) Document number(s) of the earlier application(s) in
accordance with Art. 76 EPC:
**21214041.2 / 3 988 166**
**16744527.9 / 3 316 962**

(71) Applicant: **BTL Medical Solutions a.s.**
**Staré Mesto**
**110 00 Praha 1 (CZ)**

(72) Inventors:
• **Mrazek, Jiri**
**150 00 Praha (CZ)**

• **Prouza, Ondra**
**251 01 Ricany u Prahy (CZ)**
• **Pribula, Ondrej**
**130 00 Praha (CZ)**
• **Schwarz, Tomas**
**169 00 Praha (CZ)**
• **Franta, Tomas**
**252 29 Dobrichovice (CZ)**
• **Ladman, Jakub**
**155 00 Praha (CZ)**
• **Hurych, Zdenek**
**153 00 Praha (CZ)**

(74) Representative: **DTS Patent- und Rechtsanwälte**
**PartmbB**
**Brienner Straße 1**
**80333 München (DE)**

Remarks:
This application was filed on 12.05.2025 as a
divisional application to the application mentioned
under INID code 62.

(54) **MAGNETIC STIMULATION DEVICES FOR THERAPEUTIC TREATMENTS**

(57)    A magnetic stimulation device, method of operating the magnetic stimulation device and methods of use are described. The magnetic stimulation device reaches high repetition rates and/or high values of magnetic flux density. The magnetic stimulation device provides various treatments at the same time and a plurality of treatment effects are achieved.

Figure 2

EP 4 574 201 A2

## Description

### FIELD OF THE INVENTION

The present invention relates to apparatus and methods for treating a patient by a magnetic field. The application of the magnetic field is provided by a plurality of magnetic field generating devices.

### BACKGROUND OF THE INVENTION

Magnet therapy originally used permanent magnets with a stationary magnetic field. Natural magnets were applied especially to acupuncture points, or to the location of pain. Thereafter natural magnets were replaced by synthetic magnets and electromagnets of stationary magnetic field of higher induction than permanent magnets. In the last few decades, therapeutic methods have used mainly a pulsed magnetic field.

Devices and methods generating magnetic impulses have been used for medical treatments and/or aesthetic treatment. A time-varying magnetic field induces electric currents in the patient's body, which may provide similar effect as electrotherapeutic treatment. The induced electrical currents may evoke an active response by sufficient intensity, impulse duration and/or repetition rate. The advantage of the methods using magnetic field compared with electrotherapeutical methods is that changing magnetic field therapy does not require contact with the patient and can be performed through clothing. With magnetic field treatments, the stimulating signal does not pass through the skin. Rather, the electrical currents are induced directly in the stimulated tissue. This increases stimulation focus and eliminates unwanted side effects of the therapy (e.g. skin irritation). Using a sufficiently large magnetic flux density and/or repetition rate, it is possible to stimulate various tissues without need of invasive methods.

The currently used magnetic stimulation devices mostly consist of one magnetic field generating device, a capacitor parallel to a power source and a switching device in series to the power source. Further such topology requires a snubbering device, such as diode or RC snubbering circuit, for protecting the energy source during the reverse polarity of resonance. The use of a snubbering device causes high energy losses. The magnetic stimulation device wastes a lot of energy because of low efficiency due to significant electric losses while generating the time-varying magnetic field. Therefore these devices generate magnetic impulses ineffectively.

Further there is a need to treat different areas by magnetic field to provide more efficient and faster treatment of large areas. The large areas may be at least one muscle group or region prone to cellulite.

Another disadvantage of currently used devices using one magnetic field generating device is the shape of the magnetic field. The shape of the magnetic field is dependent on the shape of the magnetic field generating device. The magnetic field may not be selectively focused during the treatment. The adjustable parameters are magnetic flux density, repetition rate and/or time duration of the treatment.

Still another disadvantage of currently used magnetic stimulation devices is the slow treatment in the case of large and/or a plurality of treated areas. The applicator has to be manually moved from one position to another position to stimulate the large and/or the plurality of treated areas and the treatment of the entire area takes a long time. Further the treatment is not homogenous since the magnetic field does not stimulate the entire large target area at the same time.

Furthermore magnetic stimulation devices using a plurality of magnetic field generating devices have limited effectiveness due to the serial connection of the switching device and magnetic field generating device which requires a snubbering device, causing high energy losses.

Currently used devices and methods of operating a magnetic stimulation device are strictly limited to generating a time-varying magnetic field. Any combined methods are applied by two separate devices generating two different types of treatment. There is a demand for a device providing combined treatments, e.g. contactless treatment by electromagnetic field, such as conventional treatment and treatment by time-varying magnetic field.

Presently, stimulation for treatment and improvement of patient's wellbeing and/or appearance is performed by magnet treatment methods reaching low repetition rates or direct current methods. In the case of more intensive treatments the magnetic stimulation device operation is limited by the factory settings and/or by the worst potential cases which are predefined by the dependence of the maximal values of magnetic flux densities and the repetition rates. The treatment is limited by the preset operation parameters.

Following the state of art the stimulation by a time-varying magnetic field is limited by key parameters of repetition rate, magnetic flux density and/or treatment duration. The applicator may exceed a predetermined temperature which may cause heat injury to the patient where the treatment of the biological structure requires high magnetic flux density. If the magnetic stimulation device produces more heat than the cooling system can dissipate, the treatment device is turned off based on feedback from a temperature sensor in the applicator.

In commercially available magnetic stimulation devices the limit may be set by the operator. However, the control system of the magnetic stimulation device may operate the magnetic stimulation device to reach the predetermined magnetic flux density which is determined empirically by the manufacturer during the most discriminating stimulation, e.g.

the case of the highest repetition rate. Therefore the stimulation is limited in the magnetic flux density domain and/or the depth of the stimulated target tissue is limited. Furthermore, the stimulation is also limited by the repetition rate and/or the treatment duration.

Additionally, no commercially available magnetic stimulation device is able to monitor the stimulation energy and protect the patient and/or the magnetic stimulation device from an unintended event by monitoring the current value of the operation parameter. Therefore, an unintended event may cause heat damage to the patient and/or the magnetic stimulation device without ceasing the treatment.

Unintended events may cause the operation parameter drop so the efficiency of the magnetic stimulation device decreases if an unintended event occurs. Power consumption may increase during the unchanged treatment parameters, placing the patient and/or the magnetic stimulation device at risk.

Magnet therapy uses the influence of magnetic flux on biological tissue. Electric current is induced in the tissue due to voltage change which causes a polarization of the cell membrane. One of fundamental phenomenon of electric current in biological tissue is a transfer of neural excitation or muscle contraction. The intensity of the effect is dependent on the magnetic flux density, repetition rate of the pulses, pulse time duration or envelope of the stimulation signal.

Water and biological molecules are diamagnetic substances. The magnetic field is not affected by diamagnetic substances. Therefore no loss of intensity or magnetic flux density occurs when passing through the biological structure or tissue. Therefore the deep biological structures may be stimulated by the time-varying magnetic field as well. One of the time-varying magnetic fields is the so called pulsed electromagnetic field (PEMF). PEMF is limited by the repetition rate and even by the magnetic flux density. PEMF repetition rates are in range of 5 to 100Hz and magnetic flux density is up to 600 Gauss (equivalent to 60 mT) based on Physikalische Medizin. [HEISEL, Jürgen. Physikalische Medizin. Stuttgart: Georg Thieme Verlag KG, 2005. ISBN 3-13-139881-7. p. 159]. Another source recites the highest magnetic flux density of 6 mT. [BRONZINO, Joseph, D. The Biomedical Engineering Handbook, Volume I. United States of America: CRC Press LLC, 2000. Second edition. ISBN 0-8493-0461-X. p. 91-1 - 91-8].

Existing methods of magnet therapy generally tend to be limited by the key parameters of repetition rate and/or magnetic flux density. These methods may use high values of magnetic flux density at low repetition rate or vice versa. These combinations limit the effectiveness of muscle therapy at higher repetition rates over 50 Hz. Therefore the stimulation of deep structures or stimulation by high repetition rates or the combination of both is limited. Existing designs do not provide any device and/or method for stimulating biological structure at repetition rate over 50 Hz and magnetic flux density sufficient to cause at least partial muscle contraction repetitively. Additionally existing methods do not disclose time duration of the therapy.

Existing methods are also not able to provide stimulation of biological structures by pulsed magnetic field at repetition rates which exceed the frequency resolution of the biological structure. Some systems also require making physical contact with the patient since the magnetic field is weak or the stimulation signal cannot be transferred without the electrical contact. Generally, these known methods are limited to repetition rates over 50 Hz in order to provide biological structure stimulation. Furthermore, repetition rates exceeding 100 Hz are not utilized. The therapeutic methods at higher repletion rates over 100 Hz are provided only by electrotherapeutic methods.

Existing methods are also not able to provide non-invasive stimulation of neural structures by time-varying magnetic field at repetition rates exceeding the frequency resolution of the neural structure. Some systems also require physical contact with the patient since the magnetic field is weak or the stimulation cannot be transferred without the electrical contact. Generally, these known methods are limited to repetition rates reaching 120 Hz in order to provide neural structure stimulation.

Presently, muscle contraction or neural structure stimulation for diagnosis or prognosis or causing improvement of patient's well-being, such as strengthening, training, myorelaxation or analgesic effect at higher repetition rates over 50 Hz and at sufficient intensity stimulus may be achieved only by direct current therapy. However, direct current methods require contact with the patient and even may be invasive. These methods can result in skin irritation, painful application especially for the high intensity stimulus, discomfort during the treatment, lack of deep tissue stimulation by non-invasive methods, and a lack of patient compliance with a prescribed therapy due to these factors.

Aesthetic medicine includes all treatments resulting in enhancing a visual appearance and satisfaction of the patient. Patients not only want to be in good health, they also want to minimize all imperfections including body shape and effects of natural aging. Indeed, patients request quick, non-invasive procedures providing satisfactory results with minimal risks.

The most common methods used for non-invasive aesthetic applications are based on application of mechanical waves, e.g. ultrasound or shock wave therapy; or electromagnetic waves, e.g. radiofrequency treatment or light treatment, such as intense pulsed light or laser treatment. The effect of mechanical waves on tissue is based especially on cavitation, vibration and/or heat inducing effects. The effect of applications using electromagnetic waves is based especially on heat production in the biological structure.

Skin tissue is composed of three basic elements: epidermis, dermis and hypodermis or so called subcutis. The outer and also the thinnest layer of skin is the epidermis. The dermis consists of collagen, elastic tissue and reticular fibres.

The hypodermis is the lowest layer of the skin and contains hair follicle roots, lymphatic vessels, collagen tissue, nerves and also fat forming a subcutaneous white adipose tissue (SWAT). The fat cells create lobules which are bounded by connective tissue, fibrous septa (retinaculum cutis).

Another part of adipose tissue, so called visceral fat, is located in the peritoneal cavity and forms visceral white adipose tissue (VWAT) located between parietal peritoneum and visceral peritoneum, closely below muscle fibres adjoining the hypodermis layer.

PEMF may be used to improve the conventional non-invasive aesthetic medicine applications, e.g. radiofrequency treatment, diathermy treatment, ultrasound treatment, etc. The enhanced visual results are achieved in combination with these methods as described in patent application US2015025299. The magnetic flux density of the recited magnetic field reaches 20 Gauss and the repetition rate varies from 16 to 25 Hz.

US2015157873 recites an applicator including stimulation coil for emitting magnetic field to the patient to induce muscle contraction for the purpose of fatty tissue breakdown. The magnetic flux density is in range of 0.01 to 0.1 T at distance of 5 cm from the coil surface, the recited repetition rate varies from 10 to 30 Hz and impulse duration is in range of 100 to 300 $\mu$s. However, the proposed stimulation by magnetic flux density 0.1 T is limited to stimulate only superficial muscle. Additionally, this stimulation may be time consuming and provide limited results. Magnetic flux density of 0.1 T is insufficient to intensively stimulate superficial muscle or deep biological structures since the magnetic flux density is low. The magnetic flux density is able to stimulate limited number of muscle fibres. Therefore the muscle contraction is weak and the method is insufficient to be applied for satisfactory enhancement of visual appearance. In order to achieve any results the treatment duration lasts long time period. Accordingly, patients with a high BMI cannot be well treated using existing methods.

Present aesthetic approaches do not propose any method sufficient for visual enhancement by contact and/or contactless use. The above-described methods are limited in key parameters which are repetition rate and/or magnetic flux density. All known methods use low values of magnetic flux density and/or low repetition rates which does not allow satisfactory enhancement of visual appearance. As a result, new methods are needed to enhance the visual appearance of the patient.

The present aesthetic approaches don't provide any treatment combining the effect of time-varying magnetic field treatment and conventional treatment, e.g. treatment by electromagnetic field such as radiofrequency treatment. The currently used radiofrequency treatment includes many adverse events such as non-homogenous thermal temperature, insufficient blood and/or lymph flow during and/or after the treatment. Additionally several adverse event such as panniculitis may occur after the treatment.

## SUMMARY OF THE INVENTION

A time varying magnetic field may be used to treat variety disorders and injuries of muscle, nerve and connective tissue. It may also be used also in physiotherapy, aesthetic therapy, urology, urogynecology, psychiatry, neurology and neurophysiology for therapy and diagnosis/prognosis.

A device for time variable magnetic field generation may include an energy source, an energy storage device, a switching circuit, a coil and possibly a core. The energy storage device accumulates tens of Joules of energy and the magnetic flux density induced by the coil is in the range of tenths of a Tesla to about one Tesla.

Existing devices have low efficiency and they waste energy, which limits their use. Eddy currents induced within the coil create engineering challenges. Existing devices contain coils which are made of metallic strips, electric wires or hollow conductors. Since the therapy requires large currents, significant losses are caused by induced eddy currents within the coil. Eddy currents lead to production of unwanted is heat and therefore there is need to sufficiently cool the coil. Also, the energy source must be protected during reverse polarity of resonance. This requires using protective circuits which consume significant amounts of energy.

Due to low efficiency, existing devices may not achieve repetition rates of magnetic pulses above one hundred Hertz, as may be needed to produce a magnetic flux density sufficient for acting on neurons, muscle fibers and/or endocrine cells (e.g. at least partial muscle contraction). Using existing devices, interruptions during therapy or between therapies are often necessary to avoid overheating the device.

The present methods and devices as described below produce a time varying magnetic field for patient treatment which better optimizes energy use, increases the effectiveness of the treatments and provide a new treatment. The magnetic impulses may be generated in monophasic, biphasic or polyphasic regimes. In a first aspect, the device has one or more coils; a switch; an energy storage device and a connection to an energy source. The coil may be made of insulated wires with a conductor diameter less than 3 mm even more preferably less than 0.5 mm and most preferably less than 0.05 mm. Smaller diameter and individual insulation of the wires significantly reduces self-heating of the coil and therefore increase efficiency of magnetic stimulation device. The coil may be flexibly attached in a casing of device. The casing may comprise a blower or blowers which ensure cooling of the coil.

Space between the insulated wires may be filled with a solid material so as to reduce the noise caused by vibrations. The coil is connected with an energy storage device which serves as a storage of energy.

The switch can be any kind of switch such as diode, MOSFET, JFET, IGBT, BJT, thyristor or a combination of them. The switch can be connected in parallel to the coil and the energy storage device, to eliminate reversal polarity of high voltage on the terminals of the energy source in the second half of the resonant effect. Therefore there is no need for additional protective circuits to protect the energy source from the negative voltage. Electric losses associated with such protective circuits are avoided. Energy use is reduced. The voltage drop in the energy storage device between first and second oscillation maximum during resonance is also reduced. Via the lower voltage drop, higher repetition rates of magnetic pulses and higher magnetic flux density may be achieved for treatment of the patient.

The coil of the magnetic stimulation device may be flexibly attached to casing of the device. The blower or blowers may be arranged to blow air on both sides of coil. Optionally, the coil may be a flat type coil.

As used here "continual therapy" and "continual magnetic stimulation" means therapy where the set of the magnetic flux density and repetition rate of magnetic pulses does not lead to exceeding of the operating temperature 43 °C on the casing of the device operating in an ambient temperature of 30 °C regardless of the duration of therapy.

The present invention provides a magnetic stimulation device including a radiofrequency treatment device and method of controlling the device.

The combined magnetic and radiofrequency treatment device includes at least one magnetic field generating device which is able to generate high power magnetic pulses and radiofrequency treatment as well.

The present invention provides a magnetic stimulation device and method of controlling the magnetic stimulation device using a plurality of magnetic field generating devices.

Using a plurality of magnetic field generating devices provides faster treatment. Large and/or different areas may be treated in shorter time. Using a plurality of applicators allows different areas and/or target biological structures to be stimulated at the same time. The movement of the at least one applicator may automatically follow a predetermined trajectory. Hence manual manipulation is not needed. Furthermore the shape of the generated magnetic field may be adjusted by an operator.

The treatment effect is more focused since the magnetic stimulation may be focused by interference of the magnetic fields generated by the plurality of magnetic field generating devices. The magnetic field may selectively stimulate small biological structures and the treatment effect may be more effective. The results may be achieved faster since the treatment is enhanced due to using the plurality of magnetic field generating devices.

The treatment is more effective with reference to the currently used magnetic stimulation devices due to use of a parallel switching device with reference to the energy source, since the effectivity is higher due to lower energy losses. Additionally there are no losses in snubbering devices no snubbering device is needed or used. Additionally the hardware components may be rated at the nominal working voltage of the power supply even during resonance. This provides a significant advantage relative to existing devices which use serial switch topology where during resonance the hardware components are loaded by a voltage approximately double the nominal working voltage.

The present invention provides a new approach in determining the parameters of biological structure treatment.

According to the first aspect of the invention the magnetic stimulation device monitors the stimulation energy based on the current value of an operation parameter and/or the operation parameter waveform of one period.

According to another aspect of the invention the magnetic stimulation device may include additional thermal protection based on mathematic and/or signal processing methods which determine the relation of the currently determined operation parameter with a reference and/or with the operation parameter measured in a different value of characteristic quantity.

According to still another aspect of the invention the magnetic stimulation device may determine, based on the transition thermal characteristic of the magnetic stimulation device, the maximal treatment parameters which can be sufficiently cooled by the cooling system.

According to still another aspect of the invention the control unit may calculate optimal flow of the cooling medium based on the treatment parameters, transition thermal characteristic of the magnetic stimulation device and/or the cooling medium temperature and significantly reduce the noise of the cooling system.

According to still another aspect of the invention the control unit may optimize the treatment parameters based on the current value of operation parameters and a transition thermal characteristic of the magnetic stimulation device.

The present invention provides an alternative treatment of neural diseases, diagnoses or prognosis, alleviating pain, myorelaxation, muscle stimulation, tissue healing, sleep improvement or edema reduction. Additionally, the present methods may be provided in combination with traditional medicament treatment.

The method may provide a non-invasive transfer of a stimulation signal from an applicator to biological structure to evoke the action potential of biological structure.

The method may use a peak to peak magnetic flux density on a coil surface at least 0.2 T, 0.4 T, 1.5 T, 2 T, or at least 3 T. The repetition rate may exceed 50 Hz, 80 Hz, 90 Hz, 100 Hz or 120 Hz, and up to 150 Hz, with preferable repetition rate up to 700 Hz. with initial or successive treatments lasting several seconds or longer, for example, for at least 5, 10, 30, 60, 120 or 240 seconds, or longer. The pulse width is in the range of tens to hundreds of microseconds.

In a first aspect, a method provides stimulation of biological structure using magnetic field at repetition rates exceeding 50 Hz for purpose of at least a partial muscle contraction.

In another application of the invention, the time-varying magnetic field at higher repetition rate exceeding the repetition rate resolution of the neural structure may be used for generating an envelope of lower repetition frequency than the repetition rate. The envelope may be generated by time-varying magnetic flux density and/or repetition rate and/or impulse duration. This method is effective for stimulation of denervated muscle.

According to the first application of the invention, the time-varying magnetic field at a magnetic flux density of at least 0.1 T and/or repetition rate at least 100 Hz may be used for stimulation of biological structure for diagnosis, prognosis or improving the patient's well-being.

In still another application of the invention, the time-varying magnetic field may be used for stimulation of biological structure to improve the patient's mental health or for influencing the hormonal production and/or balance.

In still another application of the invention, the time-varying magnetic field may also be used for stimulation of deep neural structures e.g., to treat psychiatric and/or neurodegenerative and/or psychiatric diseases, such as treatment of Parkinson's or Alzheimer's disease.

In still another application of the invention, the time-varying magnetic field may be used for stimulation of neural structure to determine whether the neural pathway is impaired or not, or the level of the impairment may be determined. The one approach is stimulating central neural system or peripheral neural structure and determining the feedback, e.g. muscle contraction. The conduction deficiency may be determined via maximal voluntary contraction.

In still another application of the invention, the time-varying magnetic field may also be used for stimulation of neural structure to alleviate pain via central neural system stimulation or via peripheral neural structure. In general, the peripheral neural structure may be stimulated by repetition rates exceeding 100 Hz and/or by envelopes of lower repetition frequencies. The repetition rate and magnetic flux density selectively stimulates different neural structures. The pain is alleviated at specific repetition rates, pulse shapes and/or current densities. The pain alleviation effect may be caused by high power time-varying magnetic field at different levels of neural system.

In still another application of the invention, the time-varying magnetic field may be used for stimulation of neural structure to cause myorelaxation effects. The myorelaxation effects may occur generally during stimulation by time-varying magnetic field of repetition rate at least 100 Hz. However, the neural structure may be stimulated by envelopes at lower repetition frequencies below 100 Hz.

In another aspect of the invention, a neuromuscular plate is stimulated causing an at least partial contraction of the muscle. The muscle is contracted at higher repetition rates and the contraction is stronger and more efficient for improving the muscle strength. The method is especially useful for deep muscles, major muscles, and for treatment of patients with high value of BMI. Deep muscle is the muscle underneath the superficial muscle. Muscle tissues may be selectively stimulated and the magnetic flux density of the stimulation may be adjusted based on patient characteristics or input. Treatment time can be shortened to a minimum due to selective stimulation of targeted muscles. Additionally, the treatment may be non-invasive or even contactless due to the high value of magnetic flux density. The patient may be treated without removing clothing, thereby reducing patient discomfort.

In still another application of the invention, the time-varying magnetic field may be used for stimulation of neural structure to cause muscle stimulation. The muscle stimulation may occur during stimulation by envelopes of repetition frequencies below 100 Hz. The application may be used for stimulation of denervated muscle treatment.

The target biological structure may be a joint. Due to the pulsed magnetic field, the dynamic fluid properties of synovial fluid are improved and muscle contraction is achieved, contributing to positioning of the joint by short movements of the joint compartments.

In still another application of the invention, the time-varying magnetic field may be used for stimulation of neural structure to cause healing tissue effect, improve the sleeping patterns or reduce the edema.

In a further aspect of the invention, the method stimulates the biological structure via a magnetic stimulation signal of at least 100 Hz, where the stimulation is intended for at least partial muscle contraction. The pulsed magnetic field induces the electric current which may provide myorelaxation. The stimulation signal repetition rate may be at least 120 Hz or at least 140 Hz.

Advantages of the present magnet therapy may include: stimulation of the deep neural structures which are problematically stimulated by superficial stimulation; non-invasive and/or non-contact application of magnetic flux density; treatment without removing clothing; absolute non-invasiveness of the stimulation and elimination of skin irritation in the place of time-varying magnetic field application; high rate of acceptability of the stimulation by patients; elimination of stimulation side effects; elimination of the necessity of applicator made of biocompatible materials; providing a clean and sterile applicator; and capability to provide local or area treatment.

The present invention provides new methods of aesthetic treatment of a patient.

In a first aspect, a method for stimulation of biological structure uses a time-varying magnetic field at a magnetic flux density of at least 0.15 T and/or repetition rate 30 Hz for the purpose of at least a partial muscle contraction and enhancing the visual appearance of the treated body area.

In another aspect, the stimulation utilizes non-invasive and/or contactless transfer of the stimulation signal from an applicator to biological structure to evoke the action potential of the biological structure to induce at least partial muscle contraction. The applicator may include a source of magnetic field e.g. a coil.

The present methods enable new treatments defined by a peak to peak magnetic flux density on the coil surface at least 0.15, 0.2, 0.8, 1.5, 2, 2.4 or up to 7 Tesla at repetition rate at least 1, 10, 30, 50, 55, 60 or up to 700 Hertz with treatment/successive treatments lasting several seconds or longer, for example, for at least 5, 10, 30, 60, 120 or 240 seconds, or longer. The pulse width is in the range of tens to hundreds of $\mu$s.

In further aspect, a neuromuscular plate and/or the nerve innervating the neuromuscular plate is stimulated and at least partial muscle contraction is provided. The muscle may be contracted at higher repetition rates and the contraction is stronger. Therefore the stimulation is more efficient for reducing the number and/or volume of adipocytes and enhancing the visual appearance of the treated body area via targeted muscle contraction. Additionally, strong muscle contractions at higher repetition rates cause mechanical movement of all the layers in proximity of the contracted muscle. This method therefore causes remodeling and/or neogenesis of the collagen and elastin fibers.

According to another embodiment the method may be applied for enhancing the visual appearance of body area including or proximate to major muscle structures. Further the method for enhancing the visual appearance is convenient for treatment of patients with high BMI.

The muscle tissue is selectively stimulated and the magnetic flux density of the stimulation may be adjusted based on the patient's feeling and/or needs. Treatment time is shortened due to selective stimulation of muscles. Additionally, the treatment may be non-invasive or even contactless due to the high value of magnetic flux density. Invasive methods may be used as well. The patient may be treated without the necessity of taking clothes off. The method reduces patient discomfort.

The present methods may be used for enhancing visual appearance of body areas including adipose tissue reduction, muscle toning, muscle shaping, body contouring, body shaping, skin tightening, cellulite treatment, circumferential reduction, breast enhancement and/or lip enhancement.

A method of treating a biological structure uses a combination of non-invasive methods for enhancing human appearance. The invention utilizes electromagnetic radiation. Methods may be used for targeted remodeling adipose tissue, focused treatment of cellulite, body contouring, skin tightening or skin rejuvenation. The invention relates to focused heating of the target tissue by electromagnetic waves, whereas the effect of focused heating of the target tissue is amplified by the effect of a pulsed magnetic field treatment.

**BRIEF DESCRIPTION OF THE DRAWINGS**

Fig. 1 is a cross section view of a coil winding.

Fig. 2 is an illustrative embodiment of cross-section of the magnetic applicator.

Fig. 3 is an illustrative embodiment of a casing of the magnetic applicator.

Fig. 4A and 4B illustrates circuit for providing high power impulses to a stimulating coil.

Fig. 5 is a graph showing voltage drop in the energy storage device.

Fig. 6 illustrates an operation mode when impulses are generated by the plurality of magnetic field generating devices at one time within a pulse

Fig. 7 illustrates an operation mode when impulses are generated by the plurality of magnetic field generating devices at plurality of different times within a pulse

Fig. 8 illustrates an exemplary embodiment of a magnetic stimulation device including a plurality of magnetic field generating devices generating time-dependent impulses

Fig. 9 illustrates an exemplary embodiment of a magnetic stimulation device including a plurality of magnetic field generating devices generating time-independent impulses

Fig. 10 illustrates an exemplary embodiment of a magnetic stimulation device including a plurality of magnetic field generating devices generating time-independent impulses

EP 4 574 201 A2

Fig. 11 illustrates a voltage calibration curve of one impulse measured in the time domain.

Figs. 12A-12G illustrates a difference of a voltage calibration curve in the case of a metal object in proximity of the magnetic stimulation device.

Fig. 13 illustrates a difference of voltage calibration curve in the case of a hardware error of the magnetic stimulation device.

Fig. 14 illustrates a diagram of a calculation algorithm operating with a plurality of inputs.

Fig. 15 illustrates an exemplary application of the calculation algorithm.

Figure 16 illustrates an action potential curve of a biological structure.

Figure 17 illustrates a perceived intensity of the stimulus.

Figures 18A and 18B illustrate an envelope generation by magnetic flux density modulation.

Figures 19A and 19B illustrate an envelope generation by repetition rate modulation.

Figures 20A and 20B illustrate an envelope generation by impulse duration modulation.

Figure 21 illustrates an exemplary application of pretreatment sequence.

Figure 22 illustrates a stimulation by exemplary bursts.

Figures 23A and 23B illustrate a generation of constant envelope.

Figures 24A, 24B and 24C illustrate a generation of FSE and SSE from OS.

Figure 25 illustrates a threshold value corresponding to different envelopes of the stimulation signal.

**LIST OF REFERENCE NUMBERS**

| 1 | magnetic field generating device |
|---|---|
| 2 | circuit wire |
| 3 | fastening point |
| 4 | blower |
| 5 | applicator |
| 6 | arrows |
| 7 | casing of magnetic applicator |
| 8 | outlet |
| 9 | connecting tube |
| 10 | fluid conduit |
| 11 | switch |
| 12 | coil |
| 13 | energy storage device |
| 14 | energy source |
| 15 | protection circuitry |
| 16 | impulse |
| 17 | pulse |
| 18 | energy source |
| 19 | energy storage device |
| 20 | magnetic field generating device |
| 21 | magnetic field generating device |
| 22 | magnetic field generating device |

| | |
|---|---|
| 23 | switching device |
| 24 | switching device |
| 25 | switching device |
| 26 | switching device |
| 27 | energy source |
| 28 | energy storage device |
| 29 | energy storage device |
| 30 | energy storage device |
| 31 | magnetic field generating device |
| 32 | magnetic field generating device |
| 33 | magnetic field generating device |
| 34 | switching device |
| 35 | switching device |
| 36 | switching device |
| 37 | switching device |
| 38 | switching device |
| 39 | switching device |
| 40 | energy source |
| 41 | energy storage device |
| 42 | magnetic field generating device |
| 43 | switching device |
| 44 | voltage calibration curve (full line) |
| 45 | measured voltage waveform (dashed line) |
| 46 | second maximum of measured voltage (incorrect) |
| 47 | second maximum of calibration curve (correct) |
| 48 | time shift |
| 49 | voltage drop (incorrect) |
| 50 | first maximum |
| 51 | voltage drop (correct) |
| 52 | resonance effect |
| 53 | calibration voltage value at same time |
| 53' | currently measured voltage value at same time |
| 54 | calibration voltage value at different time |
| 54' | currently measured voltage value at different time |
| 55' | currently measured voltage value at time t1 |
| 56' | currently measured voltage value at time t1+x |
| 57 | calculation algorithm |
| 58 | transition thermal characteristic |
| 59 | real energy losses |
| 60 | treatment parameters |
| 61 | actual temperature of the magnetic stimulation device |
| 62 | cooling parameters |
| 63 | result |
| 64 | start of magnetic stimulation device |
| 65 | input parameters |
| 66 | determine $T_{proc}$ |
| 67 | comparing $T_{Dmax}$ with $T_{max}$ |
| 68 | disable treatment |
| 69 | calculate and suggest at least one maximal treatment parameter |
| 70 | start of treatment |
| 71 | measure $T_M(t)$ |
| 72 | differing $T_M(t)$ with $T_D(t)$ |
| 73 | continue treatment |
| 74 | end |
| 75 | comparing $T_M(t)$ with $T_D(t)$ |
| 76 | comparing $T_M(t)$ with $T_{max}$ |
| 77 | disable treatment |
| 78 | curve of action potential |

79 threshold potential
79' threshold potential after depolarization
80 resting potential
80' resting potential after action potential
81 absolute refractory period
82 relative refractory period
83 sensory-threshold stimulus
84 below-sensory-threshold stimulus
85 over-sensory-threshold stimulus
86 motor-threshold stimulus
87 below-motor-threshold stimulus
88 over-motor-threshold stimulus
89 noxious-threshold stimulus
90 below-noxious-threshold stimulus
91 over-noxious-threshold stimulus
92 impulse
93 envelope
94 5 Hz train
95 50 Hz train
96 time with no stimulation
97 train
98 pulse
99 time with no stimulation
100 burst
101 constant envelope
102 biphasic envelope
103 first envelope
104 second envelope
105 rectangular envelope
106 magnetic flux density causing muscle contraction by rectangular envelope
107 increasing envelope
108 magnetic flux density causing muscle contraction by increasing envelope

## GLOSSARY

Patient refers to any living organism, such as human or animal.

Stimulation refers to a magnetic flux density inducing an electric current in the biological structure.

Biological structure/target biological structure includes a cell, a neuron, a neuromuscular plate, a nerve, a muscle fiber, a tissue, a filament, an organ, an adipocyte, a collagen, an elastin, a pigment or a skin.

Magnetic stimulation device refers to a complete magnetic stimulation device or any part of it, such as an applicator, a stimulating coil, resistors, wires etc.

Impulse refers to a single magnetic stimulus.

Pulse refers to a period of stimulation by a time-varying magnetic field of at least one magnetic stimulus and time duration of no stimulation, i.e. time duration between two impulses from rise/fall edge to next rise/fall edge.

Repetition rate refers to the frequency of firing the pulses; it is derived from the time duration of a pulse.

Calibration curve refers to the representative waveform of an operation parameter determined by mathematic and/or signal processing method, i.e. it refers to a plurality of values of an operation parameter determined in different values of characteristic quantity.

Calibration value refers to a correct value of an operation parameter which is established by factory settings, mathematical model, mathematic and/or signal processing methods.

Operation parameter impulse refers to an operation parameter waveform inducing one impulse.

Operation parameter refers to voltage, current or magnetic flux density.

Currently determined value of the operation parameter refers to the value of the operation parameter determined at a specified time during the currently examined magnetic pulse.

Characteristic quantity refers to time, frequency, amplitude or phase.

Treatment parameters refer to magnetic flux density, repetition rate, impulse duration or treatment duration.

Input parameters refer to treatment parameters, real and/or theoretical energy losses, transition thermal characteristic, actual temperature of the magnetic stimulation device, ambient temperature or cooling medium temperature and/or flow.

Mathematical model refers to an abstract model using mathematical language to describe the thermal behavior of a magnetic stimulation device.

Mathematic method refers to a calculation and/or statistic method.

Statistic method refers to any statistic quantity, e.g. mean, modus, median, running average, correlation and/or correlation coefficient.

Signal processing method refers to any method of signal processing, e.g. Fourier transformation, wavelet transformation, filtering etc.

Reference refers to the calibration curve and/or to the reference value measured in the same value of the characteristic quantity.

Normalized conditions refer to predetermined properties of the operation parameter, e.g. period, measurement time etc.

Energy storage device refers to a capacitor or other electrical energy storage device which is charged by a power supply and discharged to provide a current flow creating the magnetic field.

To relate refers to any relation of at least two values of operation parameter, i.e. relation may be correlation, correlation coefficient, ratio or any other method expressing the similarity of at least two values by mathematic and/or signal processing method.

Active response refers to any biological reaction based on the stimulation by time-varying magnetic field including a change in a permeability of cell membrane for ions or any other particles, generation of an action potential, at least partial muscle contraction, a change of rheological properties of synovial fluid.

Sensory-threshold stimulus is stimulation by magnetic flux density which induces sufficient current flow in the target biological structure when patient feels the very first perception of the induced current flow in the stimulated biological structure.

Motor-threshold stimulus is stimulation by magnetic flux density which induces sufficient current flow in the target biological structure at a level to begin to cause at least partial muscle contraction.

Noxious-threshold stimulus is stimulation by magnetic flux density which induces sufficient current flow in the target biological structure at a level which causes the patient to recognize first painful stimulus.

Below-sensory-threshold stimulus is stimulation by magnetic flux density that reaches sensory-threshold stimulus and is consequently slightly decreased after reaching the sensory-threshold stimulus.

Over-sensory-threshold stimulus is stimulation by magnetic flux density that reaches sensory-threshold stimulus and is consequently increased, however the motor-threshold stimulus is not reached.

Below-motor-threshold stimulus is stimulation by magnetic flux density that reaches motor-threshold stimulus and is consequently slightly decreased after reaching the motor-threshold stimulus.

Over-motor-threshold stimulus is stimulation by magnetic flux density that reaches sensory-threshold stimulus and is consequently increased, however the noxious-threshold stimulus is not reached.

Below-noxious-threshold stimulus is stimulation by magnetic flux density that reaches sensory-threshold stimulus and is consequently slightly decreased after reaching the sensory-threshold stimulus.

Over-noxious-threshold stimulus is stimulation by magnetic flux density that reaches sensory-threshold stimulus and is consequently increased.

Neural structure includes at least one neural cell, a neuron, a neuroglia, a Schwann cell, a nerve, a neural tissue, spine or brain.

Neural system includes central neural system and/or peripheral neural system. Central neural system (CNS) includes brain and/or spinal cord.

Deep neural structure refers to hippocampus, hypothalamus, hypophysis or thalamus, subthalamic nucleus, globus pallidus externa or substantia nigra reticulata or any other.

Action potential interference refers to creating an action potential within the cell or modifying the time distribution of the action potential.

Induced energy refers to energy stimulating the target neural structure, the amount of induced energy corresponds to repetition rate, magnetic flux density and impulse duration.

Envelope refers to shape of curve created by connection of induced energy amplitudes stimulating the target neural structure.

Repetition frequency refers to frequency of repeating the envelope shape; it is derived from a period of an induced energy envelope.

Well-being refers to physical, mental and/or psychosomatic status of a patient when the patient is free of pain or ailment and feels healthy and/or happy.

Conventional non-invasive and/or invasive treatments refer to treatments based on application of mechanical waves, e.g. ultrasound or shock wave therapy; or electromagnetic waves, e.g. radiofrequency or diathermy treatment or light treatment, such as intense pulsed light or laser treatment; or mechanical stimulation, e.g. positive or negative pressure, rollerball, massage etc.; or thermal treatment, e.g. cryotherapy; or electrotherapy method; or mesotherapy method and or any combination thereof.

Remodeling target biological structure refers to reducing the number and/or volume of the adipocytes by apoptosis and/or necrosis, cellulite treatment, body shaping and/or contouring, muscle toning, skin tightening, collagen treatment, skin rejuvenation, wrinkle removing, reducing stretchmarks, breast lifting, lip enhancement, treatment of vascular or pigmented lesions of the skin or hair removing.

Body area includes skin, muscle fiber, muscle or muscle group, collagen, elastin, adipose cell or tissue, limb and/or any other tissue.

Adipose tissue refers to at least one lipid rich cell, e.g. adipocyte.

Bolus refers to a layer of fluid material, e.g. water or fluid solution of ceramic particles, preferably enclosed in a flexible sac made of biocompatible material.

Muscle includes at least one of muscle fiber, muscle tissue or group, neuromuscular plate or nerve innervating the at least one muscle fiber.

Deep muscle refers to a muscle that is at least partly below superficial muscles and/or to the muscle that is covered by the thick layer of other tissue, e.g. mostly adipose tissue and/or the skin, with thickness 0.5, 1, 2, 3, 4, 5 or more centimeters.

## DESCRIPTION

Patients always have wanted to be in good health and look-well. However traditional invasive or contact treatment methods were very risky for the patients because such methods include an applicator made of biocompatible materials, a potential risk of infection or it may be painful for the patient. During the last few decades the contactless treatment methods were widely developed. One of these contactless methods is magnet treatment by high power magnetic field. The magnet treatment may be applied in urology, gynaecology, neurology, psychiatrics, stomatology, dermatology, geriatrics, orthopaedics, rehabilitation and in many different branches of medicine or aesthetic applications.

Apparatus for generating high power magnetic field is disclosed. In the preferred embodiment the magnetic field is time-varying, e.g. pulsed. Methods of use of the described apparatus are also described.

Therapeutic applications and treatment methods provided by the described apparatus Are described along with aesthetic applications and treatment methods provided by the described apparatus.

## SECTION I

A device for stimulation of a biological structure by high-power time-varying magnetic field is described.

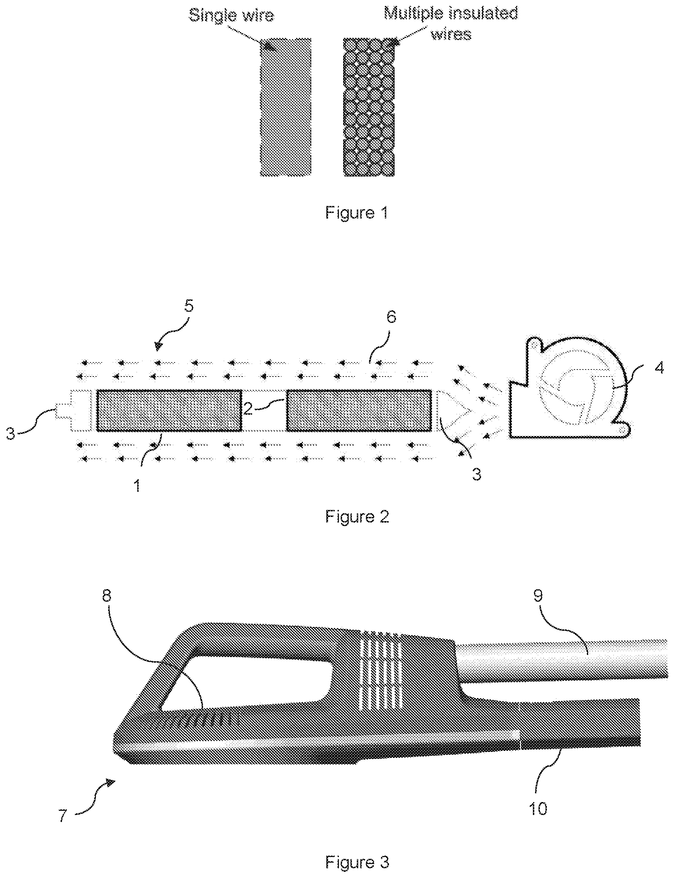

Fig. 1 illustrates a cross section of winding of magnetic field generating device (e.g. a coil) for a magnetic stimulation device. The magnetic field generating device may be constructed from litz-wire, wherein each wire is insulated separately. Each individual conductor is coated with non-conductive material so the magnetic field generating device constitutes multiple insulated wires. Unlike the conductors of existing magnetic field generating device, the present magnetic field generating device is not made of bare wire e.g. litz-wire without insulation, or conductive tapes, conductive strips, or copper pipe with hollow inductors. The insulation of wires separately is a substantial improvement, since this leads to a significant reduction of the induced eddy currents.

Energy loss due to eddy currents, per single wire, is described by Equation 1 below. The small diameter wires of the present magnetic field generating device significantly reduce self-heating of the magnetic field generating device and therefore increase efficiency of the present magnetic stimulation device:

$$E_{EDDY} = \frac{\pi^2 \cdot B_p^2 \cdot d^2 \cdot f^2}{6 \cdot k \cdot \rho \cdot D} \cdot m \cdot t_{imp} \qquad \text{Eq. 1}$$

where: $E_{EDDY}$ is energy loss (J); $B_p$ is the peak of magnetic field (T); f is frequency (Hz); d is the thickness of the sheet or diameter of the wire (m); k is constant equal to 1 for a thin sheet and 2 for a thin wire; p is the resistivity of material ($\Omega \cdot$m); D is the density of material (kg$\cdot$m$^3$); m is weight of wire material; $t_{imp}$ is the time of an impulse (s).

The individual insulation of each wire reduces eddy currents. The individually insulated wires may be wound either one by one or in a bundle of individually insulated wires so as to form a coil, which will serve as a magnetic field generator. The magnetic field generating device provides an improvement in the efficiency of energy transfer in the LC resonant circuit and also reduces or eliminates unwanted thermal effects.

The magnetic field generating device may have a planar shape where the individually insulated wires may have cross-section wires with conductor diameter less than 3 mm even more preferably less than 0.5 mm and most preferably less than 0.05 mm. The wires are preferably made of materials with higher density and higher resistivity e.g. gold, platinum or copper. The diameters of the single wires should be minimal. On the other hand the total diameter should be maximal because of inverse proportion between the cross-section of all wires forming the magnetic field generating device and the electrical resistance. Therefore the ohmic part of the heat is then lower. Eq. 2 describes energy loss of the magnetic field generating device:

$$E_R = \rho \cdot \frac{l}{S} \cdot I^2 \cdot t_{imp} \qquad \text{Eq. 2}$$

where: $E_R$ is the energy loss (J); $\rho$ is the resistance ($\Omega \cdot m$); I is the length of wire (m); S is the surface area ($m^2$); I is the current (A); $t_{imp}$ is the time of an impulse (s).

Total energy loss is given by Equation 3.

$$E_{TOT} = \sum E_i = E_{EDDY} + E_R , \qquad \text{Eq. 3}$$

Where: $E_{TOT}$ is the total energy loss (J); $E_{EDDY}$ is the energy loss of eddy currents (J); $E_R$ is the energy loss of ohmic resistance (J).

Dynamic forces produced by current pulses passing through the wires of the magnetic field generating device cause vibrations and unwanted noise. The individual insulated wires of the magnetic field generating device may be impregnated under pressure so as to eliminate air bubbles between the individual insulated wires. The space between wires can be filled with suitable material which causes unification, preservation and electric insulation of the system. Suitable rigid impregnation materials like resin, and elastic materials like PTE can be also used. With the magnetic field generating device provided as a solid mass, the vibrations and resonance caused by movements of the individual insulated wires are suppressed. Therefore noise is reduced.

The magnetic field generating device may be attached to the case of the applicator, such as a hand held applicator of the magnetic stimulation device; built-in applicator in e.g. chair, bed; or stand-alone applicator e.g. on mechanical fixture. The attachment may be provided by an elastic material e.g., silicone, gum; or other flexible manner. Connection with the magnetic field generating device of the applicator's case can be ensured by several points. The several fastening points ensure the connection of the magnetic field generating device to the casing by flexible material so that the main part of the magnetic field generating device and the main part of the casing of applicator are spaced apart. The spacing should be at least 0.1 mm so that air can easily flow. The gap between the magnetic field generating device and the casing can be used either for spontaneous or controlled cooling. The magnetic field generating device may optionally be connected to the case of the applicator by only one fastening point. The fastening points eliminate vibrations of wires which could be transferred to housing of the applicator and therefore reduce noise of the magnetic stimulation device.

Fig. 2 is a cross-section of the magnetic applicator which allows better flow on the lower and upper sides of the magnetic field generating device and thus more efficient heat dissipation. The magnetic stimulation device includes a magnetic field generating device **1,** the circuit wires **2** and the fastening points **3** for connection of the magnetic field generating device to the casing of the applicator (not shown). The fastening points **3** are preferably made of flexible material however the rigid material may be used as well. The fastening points **3** may be located on the outer circumferential side of the magnetic field generating device. However, alternatively it is possible to put these fastening points to a lower or upper side of the magnetic field generating device.

The fastening points **3** connect the magnetic field generating device to the case of the applicator in at least one point. The fastening points **3** maintain the magnetic field generating device and the main part of the case of the applicator spaced apart so that fluid (which may be air or any liquid) can flow between them. At least one blower **4** can be placed around the circumference of the magnetic field generating device, or perpendicular to the magnetic field generating device. The blower can be any known kind of device for directing the fluid e.g. outer air directed into the case of the applicator. This arrangement of the blower allows air to bypass the magnetic field generating device from upper and lower (patient's) sides. In still another embodiment the outer air can be cooled before directing into the case. The blower can have an inlet placed around the circumference of the magnetic field generating device for injecting air, to remove heat from the magnetic field generating device. A connecting tube (not shown) can ensure connection of the applicator **5** with the energy source and/or control unit of magnetic stimulation device. The connecting tube may also contain a conduit of the fluid.

The arrows **6** indicate the air flow through the applicator **5**. This arrangement of the blower allows the air to bypass the magnetic field generating device from upper and lower (patient's) side. Outlet may be preferably placed on upper side of the casing. By placing the blower around the circumference of the magnetic field generating device instead of on the top/below the magnetic field generating device, the blower **4** does not interfere with the magnetic flux peak and therefore its lifespan and reliability is increased.

Fig. 3 is an illustrative embodiment of a casing of the magnetic applicator. The overview drawing contains casing itself **7,** which might contain an outlet **8** preferably placed on upper side of the casing **7**. A connecting tube **9** may not only ensure connection of the applicator with the energy source and/or control unit of magnetic stimulation device, but also connection to a source of the fluid; however the conduit of the fluid **10** may also be connected separately.

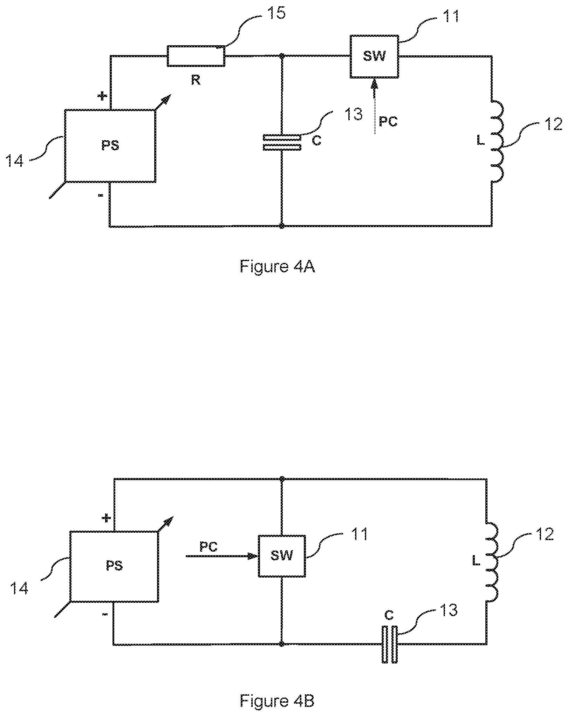

Fig. 4A and Fig. 4B illustrate circuits for providing high power pulses to the stimulating magnetic field generating device. Fig. 4A shows a circuit for providing high power magnetic impulses. Fig. 4B shows a circuit for providing high power impulses.

Existing magnetic stimulation devices achieve magnetic flux density of a few tenths to several Teslas. To achieve this level of magnetic flux density, the energy source used generates sufficient voltage. This voltage can reach thousands of volts. In Fig. 4A the circuits for providing high power pulses to the stimulating magnetic field generating device contain a series connection to the switching device **11** and the magnetic field generating device 12. The switching device **11** and the magnetic field generating device **12** together are connected in parallel with an energy storage device **13.** The energy storage device **13** is charged by the energy source **14** and the energy storage device **13** then discharges through the switching device **11** to the magnetic field generating device **12.**

During second half-period of LC resonance, the polarity on the energy storage device **13** is reversed in comparison with the energy source **14.** In this second half-period, there is a conflict between energy source **14,** where voltage on positive and negative terminals is typically thousands of Volts. The energy storage device **13** is also charged to the positive and negative voltage generally to thousands of Volts. As a result, there is in the circuit, consequently, twice the voltage of the energy source **14.** Hence the energy source **14** and all parts connected in the circuit are designed for a high voltage load. Therefore, the protective resistors and/or protection circuitry **15** must be placed between energy source **14** and energy storage device **13.** As a result a large amount of energy is transformed to undesired heat in the protective resistors and/or protection circuitry **15.**

Fig. 4B shows a circuit for providing high power impulses for improved function of the magnet stimulation device. The magnetic field generating device **12** and an energy storage device **13** are connected in series and disposed in parallel to the switching device **11.** The energy storage device **13** is charged through the magnetic field generating device **12.** To provide an energy pulse, controlled shorting of energy source **14** takes place through the switching device **11.** In this way the high voltage load at the terminals of the energy source **14** during the second half-period of LC resonance associated with known devices is avoided. The voltage on the terminals of energy source **14** during second half-period of LC resonance is a voltage equal to the voltage drop on the switching device **11.**

The switching device **11** can be any kind of switch such as diode, MOSFET, JFET, IGBT, BJT, thyristor or their combination. Depending on the type of component the load of energy source **14** is reduced to a few Volts, e.g., 1-10 volts. Consequently, it is not necessary to protect the energy source **14** from a high voltage load, e.g., thousands of Volts. The use of protective resistors and/or protection circuits is reduced or eliminated. The present designs simplify the circuits used, increase efficiency of energy usage and provide higher safety.

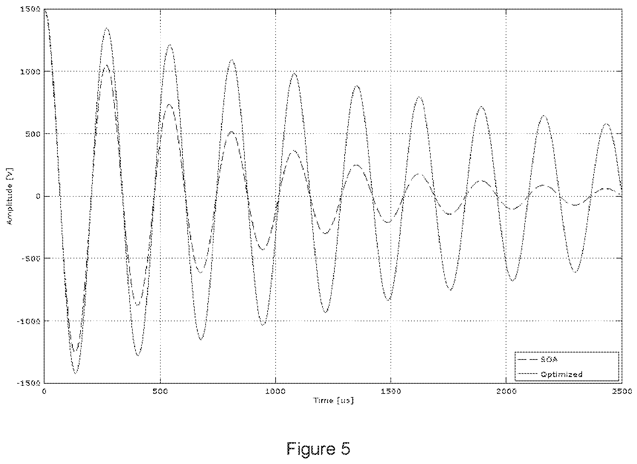

Fig. 5 shows an exponential voltage drop in the energy storage device. Energy savings during time-varying magnetic therapy may be characterized by reduced voltage drop in the energy storage device between the first, second and subsequent maximums of the resonant oscillation. The magnitude of the individual voltage oscillations is exponentially dampened up to establishing the energy balance. This allows increasing the maximum possible repetition rate of magnetic pulses, since the repetition rate is dependent on the speed with which it is possible to recharge the energy storage device. Since the energy storage device is recharged by the amount of energy loss during the previous impulse, it is possible to increase the repetition rate of the device up to hundreds of magnetic pulses per second without the need to increase the input power. The voltage drop between any of the successive amplitudes is not higher than 21 %, even more preferably not higher than 14 % and most preferably not higher than 7 %.

The device can be used for treatment/successive treatments in continual, interrupted or various duty cycle regime. The duty cycle may be higher than 10 %, which means interrupted regime with the ratio up to 1 active to 9 passive time units. The ratio may possibly change during the therapy. The device enables operation defined by the peak to peak magnetic flux density on the magnetic field generating device surface at least 3 T, more preferably at least 2.25 T, most preferably at least 1.5 T at repetition rates above 50 Hz, more preferably at repetition rates above 60 Hz, even more preferably at repetition rates above 70, most preferably at repetition rates above 80 Hz with treatment/successive treatments lasting several seconds or longer, for example, for at least 5, 10, 30, 60, 120 or 240 seconds, or longer. The total power consumption of the magnetic stimulation device is below 1.3 kW and the width of impulses is in the range of hundreds of μs.

The device enables achieving repetition rates above 100 Hz, more preferably repetition rates above 150 Hz, most preferably repetition rates above 200 Hz with the magnetic flux density providing a therapeutic effect on neurons and/or muscle fibers and/or endocrine cells (e.g. at least partial muscle contraction, action potential in cell). Based on achievement of repetition rates in order of few hundreds the device also enables assembling the magnetic impulses into the various shapes (e.g. triangular, rectangular, exponential), with the shape widths from 6 ms to several seconds or longer.

## Combined Magnetic and Radiofrequency Treatments

A combination of magnet treatment and radiofrequency treatment may be provided. International Patent Application PCT/US2012/64942 describes a device for radiofrequency treatment which may be used. In an alternative embodiment the radiofrequency treatment device may exclude the balun transformer, or the balun transformer may be included in the transmatch. The possible methods of treatment by combined methods are described below.

### Separate devices

Magnet treatment in combination with radiofrequency treatment may be represented by two independent treatment devices, e.g. one treating the target structure by radiofrequency waves and the second treating the target structure by a magnetic field. Both devices may have a separate applicator for treating the target structure, or one applicator may be used by at least two devices, i.e. the applicator may be modular for a plurality of devices.

### One device

The treatment device may include at least one HF frequency generator for providing energy for radiofrequency treatment and for providing energy for magnet treatment. In an alternative embodiment, the device may include at least one HF frequency generator for providing energy for radiofrequency treatment and at least one other independent frequency generator for providing energy for magnet treatment. The device may include plurality of applicators for providing separate radiofrequency or magnet treatments to the patient.

In alternative embodiment the applicator may provide a combination of radiofrequency and magnet treatment. In one embodiment, the applicator may include at least one radiofrequency electrode for providing radiofrequency treatment and at least one magnetic field generating device for providing magnet treatment. In another embodiment, the applicator may include at least one electrode for providing radiofrequency treatment and at least one magnetic field generating device providing magnet treatment, wherein the at least one RF source provides energy for both at least one electrode and at least one magnetic field generating device.

In still another embodiment the at least one RF source may provide the energy for the at least one magnetic field generating device providing magnet treatment wherein the at least one magnetic field generating device may be used as the at least one electrode. The essence is the far different stimulation frequencies which are used for RF treatment and magnet treatment. The magnetic field generating device in the high frequency field is similar to the electrode. This enables the magnetic field generating device to be the electrode for radiofrequency treatment. In the preferred embodiment a flat magnetic field generating device may be used as the electrode.

The frequencies for the radiofrequency treatment may be in the range of ones of MHz to hundreds of GHz, more preferably in the range of 13 MHz to 3GHz, most preferably around 13.56 or 40.68 or 27.12 MHz or 2.45 GHz. The term "around" should be interpreted as in the range of 5 % of the recited value. The impulse frequencies for the magnet treatment may be in the range of hundreds of Hz to hundreds of kHz, more preferably in the range of ones of kHz to tens of kHz, most preferably up to 10 kHz. However the repetition rate of the magnetic impulses may reach up to 700 Hz, more preferably up to 500 Hz, most preferably in the range of 1 to 300 Hz. The magnetic flux density of the magnet treatment is at least 0.1 T, more preferably at least 0.5 T, even more preferably at least 1 T, even more preferably at least 1.5 T, most preferably at least 2 T, or up to 7 Tesla on the magnetic field generating device surface.

Plurality of magnetic field generating devices

An alternative device may include a plurality of magnetic field generating devices.

Most of the currently used magnet treatment devices include the only one magnetic field generating device for treatment of a patient. An applicator including one magnetic field generating device is focused for treatment of the particular target area of the patient.

However, there are often applications where the target area is larger than the area one magnetic field generating device is able to stimulate, e.g. at least one muscle group, such as quads, glutes or pelvic floor muscles, abs, biceps and/or triceps or any region prone to cellulite, such as a belly region, love handles, buttocks, thigh region etc. In the currently used treatment devices these large target areas are treated by moving and repositioning the magnetic field generating device which leads to slow treatment.

In one aspect of the invention a magnetic stimulation device includes at least one applicator and a plurality of magnetic field generating devices. The magnetic field generating device may be air-cooled or cooled by any other fluid media.

The magnetic stimulation device may include at least one energy source, at least one energy storage device (e.g. a capacitor), at least one magnetic field generating device (e.g. a coil) and at least one switching device. The magnetic field generating device may include a core, however in a preferred embodiment the magnetic field generating device includes no core. The switching device may be any kind of switching device such as diode, MOSFET, JFET, IGBT, BJT, thyristor or a combination of them.

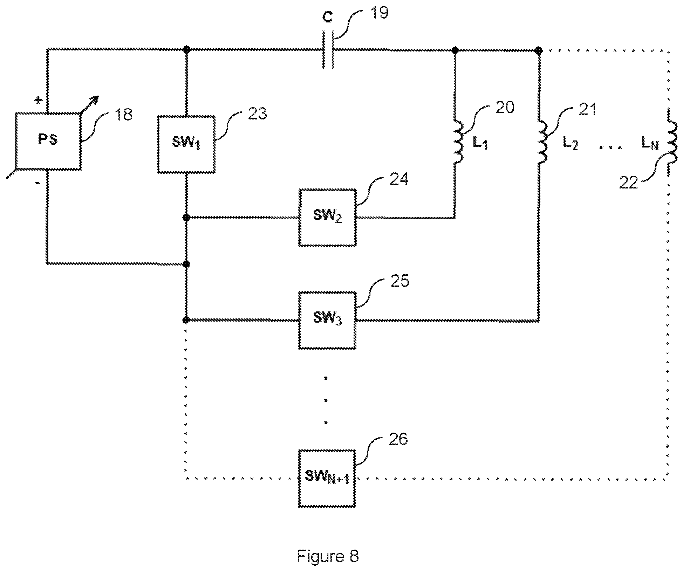

The at least one magnetic generating device may be in various shapes to enhance a variability of magnetic field profile. The shape of the magnetic field generating device may be circular, semicircular, rectangular, "figure 8", V-shape, Y-shape or a butterfly shape. The magnetic field generating device may be flat (2-D shape). In an alternative embodiment the magnetic field generating device may correspond to various 3-D bodies, e.g. a hemisphere. In another alternative embodiment the magnetic field generating device may be flexible to be better fitted to the patient. The magnetic field generating device may or may not include a core for the field shaping.

The positioning of the plurality of magnetic generating device may be very useful.

Large areas may be stimulated by the plurality of the magnetic field generating devices. The plurality of magnetic field generating devices may generate a plurality of independent magnetic fields, e.g. two magnetic field generating devices may generate two magnetic fields with two peaks of magnitude of magnetic flux density.

The plurality of magnetic field generating devices may be operated at various treatment parameters and/or operation modes to provide various treatment effects for the patient during the treatment, e.g. myostimulation, myorelaxation, analgesic effect or aesthetic effects such as adipose tissue reduction, muscle toning, muscle shaping, body contouring, body shaping, skin tightening, cellulite treatment, circumferential reduction, breast enhancement and/or lip enhancement.

The magnetic field generating devices may be positioned in isolated locations of the applicator. Alternatively, the magnetic field generating devices may be positioned next to each other, in an array or matrix, in a pattern or in randomized locations of the applicator.

The magnetic field generating devices may be positioned and/or movable in the at least one applicator in one plane; in at least two mutually tilted planes defined by a convex or concave angle, or perpendicular to each other; or in at least two parallel planes with the at least one magnetic field generating device in each parallel plane. The movement of the at least one magnetic field generating device may be translational and/or rotational, constant or accelerated. The movement may follow a predetermined, random or predefined trajectory, such as a pattern, array or matrix. The angles of the planes and/or the movement of the at least one magnetic field generating device may be adjusted by an operator following the patient's needs. In an alternative embodiment the patient may be positioned in the intersection of the magnetic fields generated by the plurality of magnetic field generating devices.

The positions of the at least two magnetic field generating devices may focus the magnetic fields to the target area; or the magnetic field generated by one magnetic field generating device may interfere with the magnetic field generated by another magnetic field generating device and the resulting magnetic field may be shaped. The magnetic flux density may be summed from the plurality of magnetic field generating devices.

The plurality of the magnetic field generating devices may extend the active time duration of the stimulation in the case that the switching devices are switched in sequence. Therefore the treatment is more effective and the treatment time may be shortened.

The magnetic stimulation device may include the at least one applicator, the at least one energy source and at least two magnetic field generating devices. However, in an alternative embodiment the magnetic stimulation device may include a plurality of applicators and/or a plurality of energy sources. The plurality of applicators may be used for treatment of at least two cooperating muscle groups with different treatment effects. In an exemplary application e.g. the triceps brachii muscle may be treated to achieve myostimulation effects and the biceps brachii muscle may be treated to achieve myorelaxation effects.

The at least one applicator may be movable in a predefined pattern, e.g. a scanning movement, or the movement may follow a random trajectory. The movement may be constant or accelerated to provide the most comfortable treatment for the patient. The movement of the applicator may be adjusted by the operator.

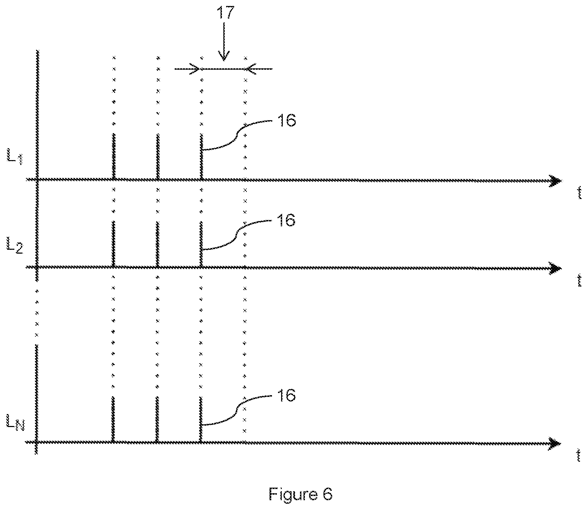

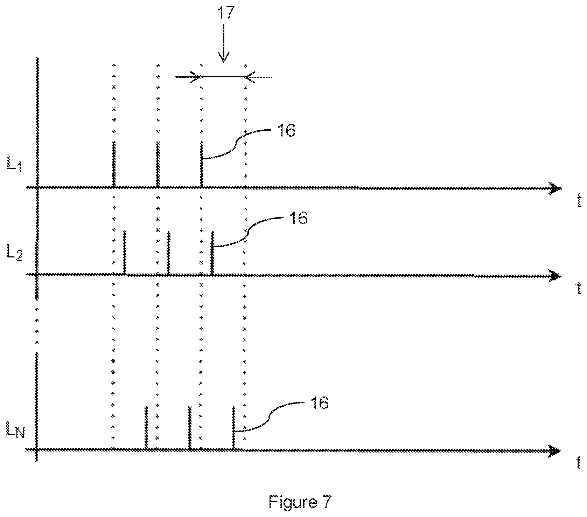

The treatment by the magnetic stimulation device may be in different operation modes. One operation mode may generate a plurality of impulses **16** at one time within the pulse **17** as illustrated in Fig. 6. Another operation mode may generate a plurality of the impulses **16** at different times within the pulse **17** as illustrated in Fig. 7. Both operation modes may be combined.

**Impulses Generated at the Same time**

The magnetic stimulation device may generate a plurality of the impulses **16** by the magnetic field generation devices $L_1, L_2, ... L_N$ at one time within the pulse **17**. This operation mode is illustrated in Fig. 6. As shown in Fig. 8, a magnetic stimulation device may include at least one energy source **18,** one energy storage device **19,** N magnetic field generating devices **20-22** and N+1 switching devices **23-26,** wherein N is positive integer greater than 1. This exemplary embodiment includes a minimum of hardware components. The value of inductance of each magnetic field generating device may be constant, however in an alternative embodiment different values of inductance may be used.

The switching devices **24-26** may be switched separately, with the magnetic field generated by separate magnetic field generating devices.

In an alternative embodiment any switching device may be switched in combination with at least one other switching device.

The magnetic flux density of the stimulation is proportional to the number and/or the inductance of active magnetic field generating devices **24-26**. The active magnetic field generating devices are the magnetic field generating device in the closed loop of the electric circuit. For example if the number of active magnetic field generating devices is 2 and the inductances of the magnetic field generating devices are the same, then the value of magnetic flux density for each magnetic field generating device may be one-half of the magnetic flux density which would be reached by one active magnetic field generating device with the same parameters and conditions, e.g. inductance, resistance, frequency, voltage. The total equivalent inductance of the magnetic stimulation device may be changed by switching a plurality of switching devices into a closed electric circuit. Therefore the impulse duration may be adjusted by adjusting the inductance. The value of total equivalent inductance ($L_{total}$) may be determined by Equation 1.

$$\frac{1}{L_{total}} = \frac{1}{L_1} + \frac{1}{L_2} + \cdots + \frac{1}{L_N} \qquad \text{Equation 1}$$

**Impulses Generated at Different times**

The magnetic stimulation device may generate a plurality of impulses **16** generated by the magnetic field generation devices $L_1$, $L_2$, ... $L_N$ at different times within the pulse **17**. This operation mode is illustrated in Fig. 7. This operation mode may multiply the repetition rate perceived by the patient, e.g. when the number of magnetic field generation device is 3 and the repetition rate of each magnetic field generating device is 100 Hz, then the patient may perceive the repetition rate 300 Hz. In an alternative example, this operation mode may be used for treatments of high repetition rate when the magnetic stimulation devices are switched to reach such repetition rates which may be sufficiently cooled.

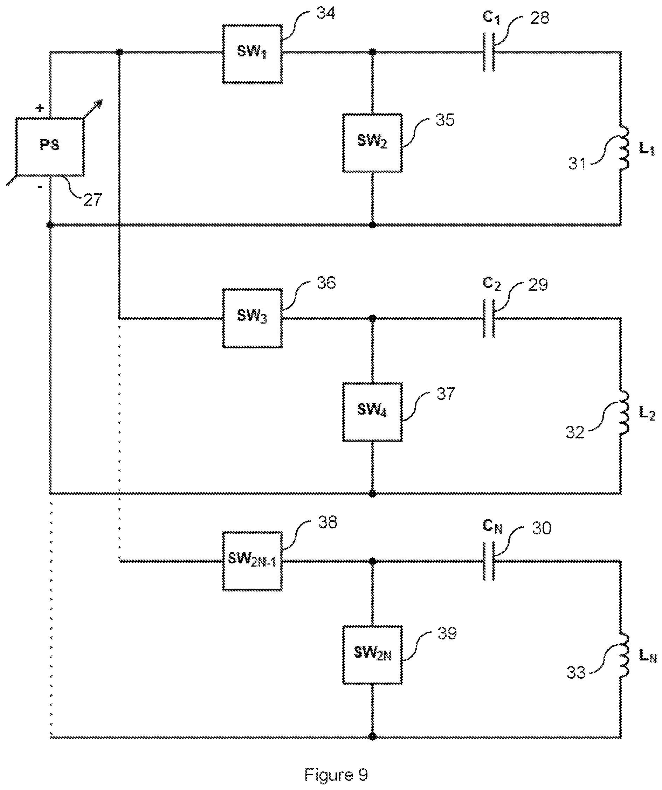

In the example of Fig. 9 a magnetic stimulation device includes at least one energy source **27,** N energy storage devices **28-30**, N magnetic field generating devices **31-33** and 2xN switching devices **34-39,** wherein N is positive integer greater than 1. The at least one energy storage device **28-30** may be selectively charged by the energy source **27** by selectively switching the switching devices **34, 36, 38** and the impulses may be selectively generated by selectively switching the switching devices **35, 37, 39.**

The benefit of this exemplary embodiment is the time independency of the impulses generated by the separate magnetic field generating devices. However, the switching devices may be synchronized to generate the impulses at one fixed time within the pulse or both operation modes may be combined using this embodiment. Another benefit of this embodiment is the possibility of providing various treatments by a plurality of magnetic field generating devices. Various treatments may provide various effects for the patient, e.g. stimulation, such as myostimulation, pain alleviation or myorelaxation.

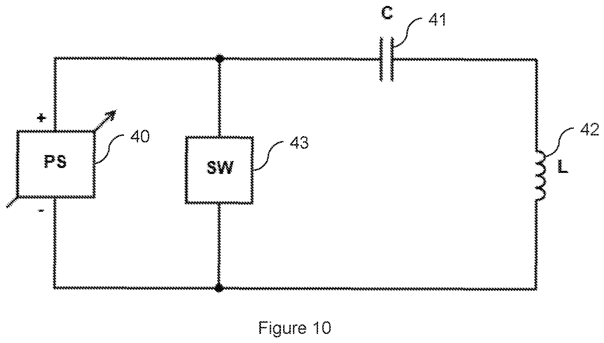

Fig. 10 illustrates an example where the magnetic stimulation device includes N energy sources **40,** N energy storage device **41,** N magnetic field generating devices **42** and N switching devices **43,** wherein N is positive integer greater than 1. The at least one energy storage device **41** may be selectively charged by the energy source **40** and the impulses may be selectively generated by selectively switching the switching devices **43.**

The impulses generated by the separate magnetic field generating devices are time independent. However, the switching devices may be synchronized to generate the impulses at one time within the pulse or both operation modes may be combined.

**Multiple applicators**

The magnetic stimulation device may include a plurality of applicators. The applicator includes at least one magnetic field generating device which may be movable. The benefit of this embodiment is that the movement and/or positioning of the plurality of the applicators may be independent. Hence different parts of the patient's body may be treated simultaneously. Therefore the total treatment time is reduced and patient's downtimes are reduced as well. The movement of the at least one applicator may be automatic so that manual manipulation may not be needed. The movement of the at least one applicator may follow a predetermined trajectory or it may be random. In an alternative embodiment the movement of the plurality of applicators may be synchronized.

The plurality of applicators may be positioned with respect to each other in one plane; in at least two mutually tilted planes defined by convex or concave angles, or perpendicular to each other; or in at least two parallel planes. The angles of the planes may be adjusted by an operator following the patient's needs. In an alternative embodiment the patient may be positioned in the intersection of the magnetic fields generated by the plurality of magnetic field generating devices.

The benefit of this application may be treatment of a plurality of cooperating muscles, such as agonists and antagonists, e.g. one muscle may be stimulated to achieve strengthening effect and on the other side the other muscle may be stimulated to achieve myorelaxation effect.

All the above described exemplary embodiments may be used in one or a plurality of applicators.

The inductance of magnetic field generating devices in each embodiment may vary. The capacitance of the energy storage devices in each embodiment may vary as well. The impulse duration may be variable and/or the magnetic flux density generated by different magnetic field generating devices may vary as well. **Safety concept**Method of operating the magnetic stimulation device.

An important parameter for the safe concept is the temperature of the magnetic stimulation device. Heat is generated by total energy losses which are caused by static and dynamic components (Equations 1 - 3). The heat is distributed within the magnetic stimulation device and the heat is dissipated by the cooling medium flow.

The generated heat needs to be monitored since overheating may cause damage to the magnetic stimulation device and heat damage of the medical device may also be a potential risk for the patient. Therefore heat generation has to be monitored to prevent thermal damage to the magnetic stimulation device and/or the patient.

Power losses and/or heat generation may be monitored and/or determined by the magnetic stimulation device based on determining the waveform of any operation parameter, e.g. voltage, electric current or magnetic flux density. The determined waveform is related with a reference and/or with the operation parameter measured in a different value of a characteristic quantity, e.g. time, frequency, amplitude or phase.

According to the invention a current value of an operation parameter, e.g. voltage, electric current or magnetic flux density, may be determined by measuring via a suitable sensor or by deriving from a value of voltage source, e.g. an energy storage device or power source. The currently determined operation parameter is processed by a mathematic and/or signal processing method.

**Operation parameter monitoring** According to one application of the invention the at least one currently determined operation parameter may be used for determining a correctness of the stimulation. The correctness of the stimulation may be determined by the relation between a current value of an operation parameter and a reference or the operation parameter measured in a different value of characteristic quantity. The relation is result of a mathematic and/or signal processing method.

## Calibration curve

According to one aspect of the application a calibration curve may be established. The calibration curve is calibration waveform of the operation parameter. The calibration curve may be implemented by the manufacturer as a factory setting. Alternatively, the calibration curve may be established by a mathematic and/or signal processing method.

The calibration curve may be determined from at least one waveform, more preferably at least 2 waveforms, even more preferably at least 5 waveforms, even more preferably 10 waveforms, most preferably at least 50 waveforms. The reference may be established by the complete calibration curve, a representative segment of the calibration curve or by predefined reference points of the calibration curve, e.g. a look-up-table.

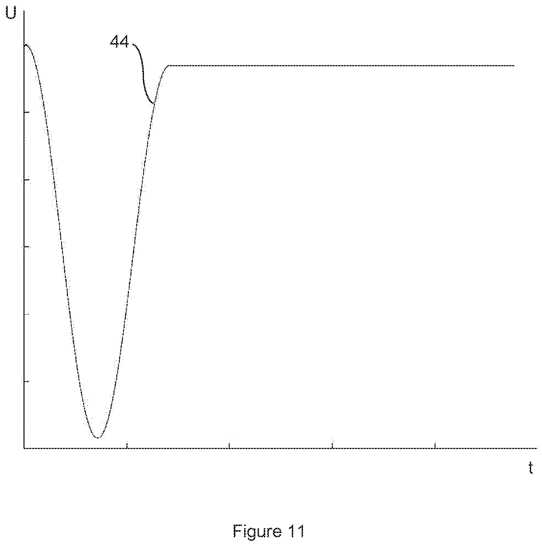

Fig. 11 illustrates a voltage calibration curve **44** of one impulse measured in the time domain. The voltage waveform may be determined e.g. on an energy storage device. However any operation parameter may be used for establishing the calibration curve.

The currently measured voltage waveform and the calibration curve are related using a mathematic and/or signal processing method. Based on the relation at least one threshold may be established. The at least one threshold may correspond to the correctness of the stimulation and/or notify operator of the magnetic stimulation device about an unintended event. The unintended event may refer to detection of a metal object e.g. metal jewelry such as ring or bracelet, or a prosthetic device such as an endoprosthesis or surgical nail within the proximity of the magnetic stimulation device; or to detection of a hardware error of the magnetic stimulation device, e.g. error of the switching device such as a thyristor. Based on the evaluation of any unintended event the treatment may be disabled and/or the notification for the operator may be generated by the magnetic stimulation device in a human perceptible form, e.g. by mechanical and/or electromagnetic means, such as audibly perceptible notification (e.g. beep) or visually perceptible notification (flashing light, color change etc.).

In an exemplary application of the aspect of the application, the relation between currently measured voltage waveform and the voltage calibration curve may be determined by a statistic method resulting in a correlation coefficient. The time duration of the correlated calibration curve and the voltage waveform may be longer than the time duration sufficient to reach the value of a second maximum. The correct stimulation may be determined if the correlation coefficient value is in absolute value at least 0.9, more preferably at least 0.95, most preferably at least 0.99. The unintended event may be detected if the correlation coefficient value is in absolute value at least 0.4, more preferably at least 0.6, even more preferably at least 0.7, most preferably at least 0.9. The value of correlation coefficient may be used for detection of a metal object within the proximity of the magnetic stimulation device e.g. metal jewelry such as a ring or bracelet, or a prosthetic device such as an endoprosthesis or a surgical nail; or for detection of hardware error of the magnetic stimulation device, e.g. error of a switching device such as thyristor.

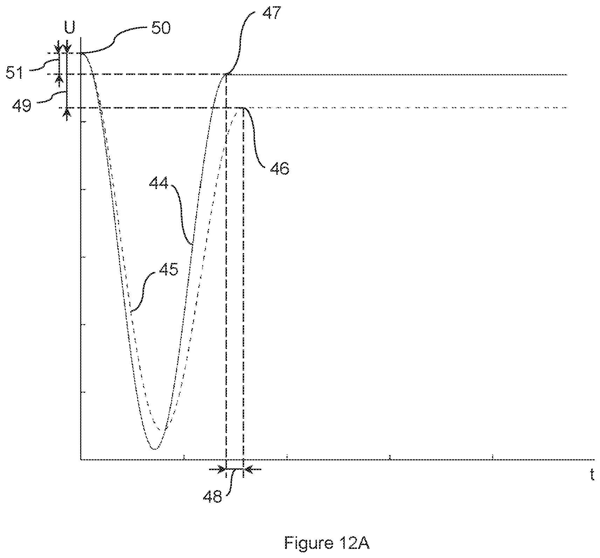

Fig. 12A illustrates the case when the metal object is within proximity of the magnetic stimulation device. There are two curves which refer to a voltage calibration curve **44** and the currently measured voltage waveform **45**. The currently measured voltage waveform **45** differs in the value of second maximum **46** compared to the value of second maximum **47** of the voltage calibration curve **44**. Further additional difference occurs in time shift **48** referring to the time when the currently measured voltage reaches the value of second maximum **46** compared to the time when the calibration curve reaches the value of second maximum **47**. Therefore the correlation coefficient may reach lower values in absolute values than in the case of correct stimulation when the value of the correlation coefficient in absolute value is at least 0.9, more preferably at least 0.95, most preferably at least 0.99. The detection of a metal object is very important for the patient's safety due to risk of injury caused to the patient by heat induction in the metal object and/or by the unintended movement of the metal object.

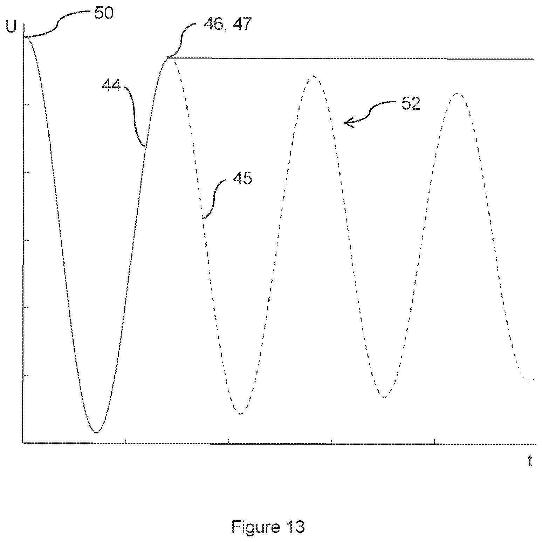

Fig. 13 illustrates the case when a hardware error occurs, e.g. a failure of the switching device. There are two curves which refer to a voltage calibration curve **44** and the currently measured voltage waveform **45**. The voltage calibration curve **44** value remains constant after reaching the value of second maximum **47**. However, the currently measured voltage waveform **45** continues in resonance **52** although the value of second maximum **46** equals to the value of second maximum **47** of the calibration curve **44**.

In the preferred application the relation between the voltage calibration curve **44** and the currently measured voltage waveform **45** may be determined by a time period longer than time duration sufficient to reach the value of second maximum of the operation parameter.

The calibration curve may be set by the manufacturer or by a mathematic and/or signal processing method. The magnetic stimulation device may verify and/or adjust the calibration values periodically after a predetermined time period and/or after changing any part of the magnetic stimulation device.

The benefit of using the correlation coefficient is that the method is independent of repetition rate and/or amplitude of the stimulation. The method also provides very precise and/or relevant results.

**Detecting Correct Values**

In an alternative aspect of the application, the correctness of the treatment may be determined simply by a relation of the at least one specific value of the currently measured voltage waveform **45** influenced by the metal object. The metal object may absorb a part of the stimulation energy. Therefore the currently measured voltage is lower than the calibration value and the currently determined voltage drop **49** is increased as is illustrated in Fig. 12A. The value of first maximum **50** corresponds with the maximum stimulation voltage generated by a voltage source, it may be simply determined from the voltage source. During the correct treatment based on energy losses a recharge of the energy storage device is not up to the value of first maximum **50** but only up to the value of second maximum **47** which is less than the value of first maximum **50**. Therefore a correct voltage drop **51** occurs which is determined by the difference of the value of first maximum **50** and the value of second maximum **47**. The correct voltage drop **51** corresponds with the value of first maximum **50**. The voltage drop occurs within each impulse. Therefore a threshold of correct voltage drop may be set up. In the case of no unintended event and the correct treatment, the value of currently measured voltage corresponds with the calibration value and the correct voltage drop **51** remains constant during the constant operation parameters and/or ambient conditions. With respect to correct voltage drop **51** a predetermined voltage drop threshold may be set up which corresponds with the correct magnetic stimulation and which may be considered as being correct. The correct values may be calibrated by the manufacturer or may be determined by mathematic and/or signal processing methods. The magnetic stimulation device may verify and/or adjust the calibration values periodically after a predetermined time period and/or after changing any part of the magnetic stimulation device.

The correct voltage drop threshold may be established at 30 %, more preferably 21 %, even more preferably 14 %, most preferably 7 % of the value of first voltage maximum. If the voltage drop reaches the threshold then the proximity of the metal object may be determined. If the voltage drop varies in time after reaching the value of second maximum then a hardware error may be detected. The notification relating to an unintended event may be generated in human perceptible form.

In an alternative approach, the correctness of the treatment may be also determined only by the relation values of second voltage maximums **46, 47** and/or by the relation of any other reference points in voltage calibration curve **44** and the currently measured voltage waveform **45**. The reference points and/or threshold may be established by the manufacturer as factory settings, e.g. a look-up-table, or the reference points and/or threshold may be established by the operator.

During the treatment several cases may occur. In the exemplary application the operation parameter may be voltage. These cases are:

1) The correct stimulation case where the currently measured voltage (a specific value or a waveform) is identical or within an acceptable range of a reference or the voltage value measured in different time of the same pulse (correlation coefficient equals almost 1).

2) The incorrect stimulation case, which may be determined by the relation of:

a) The currently measured voltage waveform and the calibration curve; or

b) The currently measured value of voltage measured at a predetermined time $t$ and the predetermined correct value of voltage at time $t$. (e.g. the time $t$ may be the moment of reaching the second maximum). If the relation exceeds a predefined threshold then an incorrect stimulation case is present and the magnetic stimulation device generates a notification to the personnel.

c) The currently generated voltage is measured in two different times: time $t$ and time $t+x$ and the currently measured values of voltage are related together. If the relation exceeds a predefined threshold then an unintended event is present and the magnetic stimulation device generates a notification to the personnel.

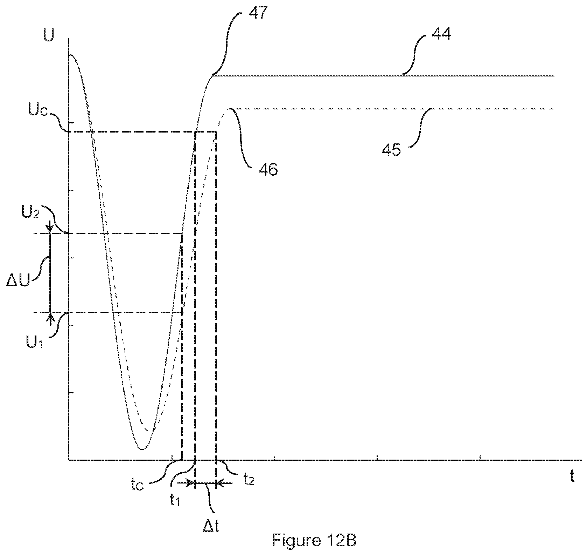

As shown in Fig. 12B, the correctness of the stimulation may be determined by at least one reference point in the currently measured voltage waveform. In the preferred application the value of second maximum is used because it is well defined and it may be easily determined. On the other side, the correctness of the stimulation may be determined by the relation of at least two reference points. One exemplary application may determine a voltage difference $\Delta U = U_2 - U_1$ at time $t_c$. Based on the value of the voltage difference proximity of metal object may be determined. In this exemplary application $U_2$ is constant because it is derived from a calibration value. Another exemplary application may determine a time difference $\Delta t = t_2 - t_1$ from when a calibration value and the measured voltage reach a selected voltage $U_c$. Then based on the value of the time difference proximity of metal object may be determined. In this exemplary application $t_1$ is constant because it is derived from a calibration value.

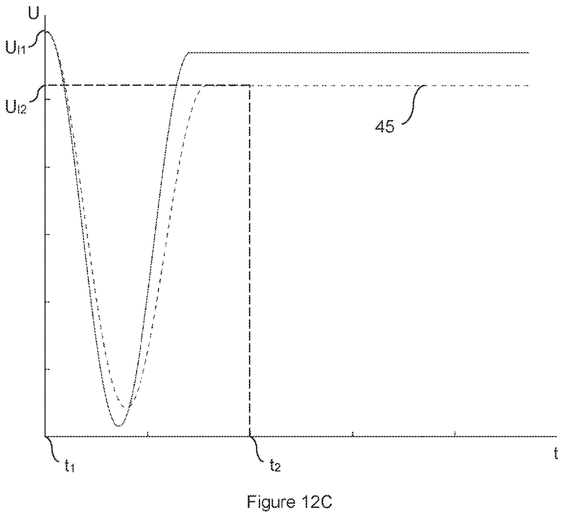

Fig. 12C shows determining an incorrect stimulation by currently measured values of voltage ($U_{I1}$, $U_{I2}$) measured in predetermined values of time ($t_1$, $t_2$). The correctness of the stimulation may be determined by the relation of $U_{I1}(t_1)$ and $U_{I2}(t_2)$. If the relation exceeds a predefined threshold then an unintended event is present and the magnetic stimulation device generates a notification to the personnel. In the preferred application the values of first and second maximum may be used.

In one aspect, a method of controlling a magnetic stimulation device for treating a biological structure by time-varying magnetic field includes determining at least one value, for example (Volts), of an operation parameter (Voltage) in at least one value (microseconds) of characteristic quantity (time), wherein the value of operation parameter is related to at least one of: a calibration curve; a calibration value; or at least one value (two voltage measurements at specific times) of the same operation parameter in a different value (microseconds) of the same characteristic quantity (time), wherein the calibration curve and/or the calibration value may be determined in the same value (microseconds) or in a different value (microseconds) of the same characteristic quantity (time). The calibration curve is plurality of calibration values (Volts in this example) of an operation parameter (Voltage) in a plurality of values (microseconds) of a characteristic quantity (time). A calibration value, in this example, is a specified voltage at a specified time.

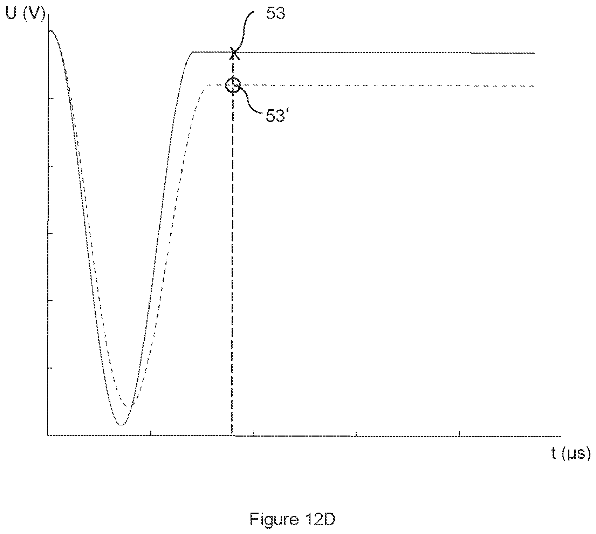

In Figs. 12D-12G, all currently measured values are shown as a circle with reference numbers marked with an apostrophe; all calibration values are marked as cross and reference numbers are without an apostrophe; all relations determined at the same time are reference numbers **53;** and all relations determined at different times are reference numbers **54.**

A complete waveform of one impulse is measured. The impulse (when the voltage value changes in time) lasts e.g. 280 μs during correct stimulation. The complete voltage waveform is related (using the definition in the glossary above) to the calibration curve (stored in memory of the magnetic stimulation device). The relation is expressed by the value of a correlation coefficient which indicates the similarity of the currently measured waveform and calibration curve.

Referring to Fig. 12D, the calibration voltage waveform **53** may be related to the currently measured voltage waveform **53'** with the same time duration, e.g. 350 μs, i.e. the time duration of calibration voltage waveform **53** equals the time duration of currently measured voltage waveform **53'**. The complete voltage waveform need not be determined. It is sufficient to set at least one calibration value. The currently measured voltage value **53'** is related to the predetermined calibration voltage value **53**. The ratio of the voltage values **53, 53'** (or value of voltage drop, or correlation) determines an incorrect stimulation. The currently measured voltage value **53'** and the calibration voltage value **53** may be determined at the same time.

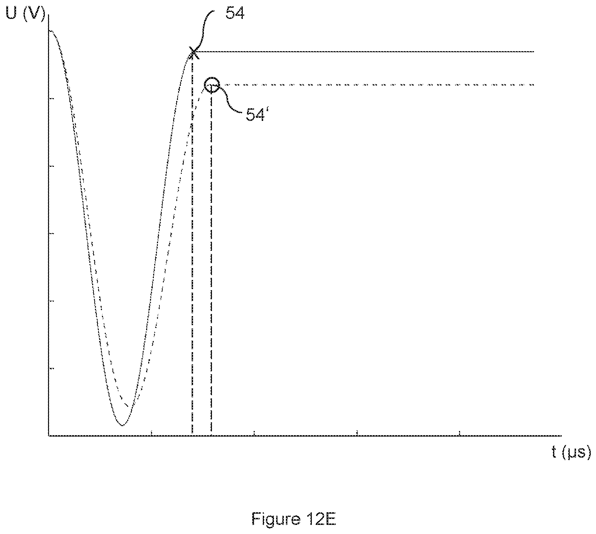

Referring to Fig. 12E, the calibration voltage waveform **54** may be related to the currently measured voltage waveform **54'** with a different time duration. The currently measured voltage value **54'** is measured at the time when the second maximum is reached. The measured voltage second maximum occurs at a time different than the time of second maximum of calibration curve.

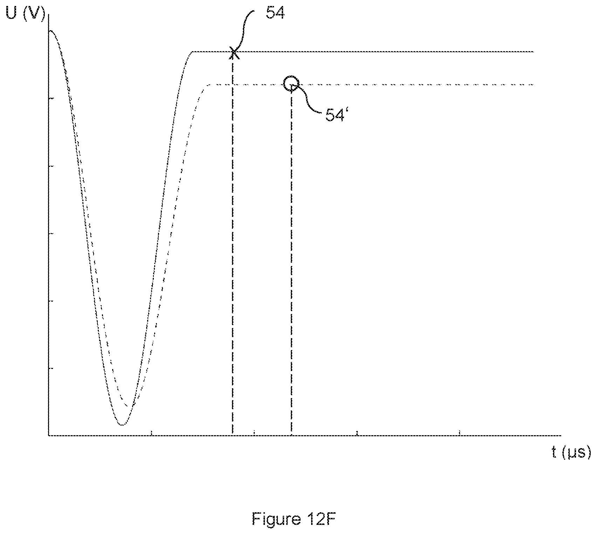

Turning to Fig. 12F, the currently measured voltage value **54'** is related to the predetermined calibration voltage value **54**. The ratio of the voltage values **54, 54'** (or value of voltage drop, or correlation) determines an incorrect stimulation. The currently measured voltage value **54'** and the calibration voltage value **54** may be determined at different times. The result of the relation is the same, although the currently measured voltage **54'** is measured at a different time (e.g. at time 600 $\mu$s) than the calibration voltage value **54** (e.g. at time 400 $\mu$s).

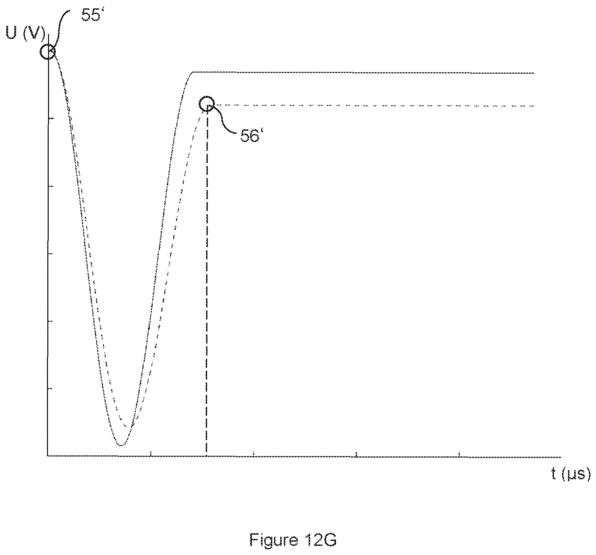

Moving to Fig. 12G, the complete voltage waveform need not be determined, and any calibration voltage value does not have to be set. A relation between the currently measured voltage values **55'** and **56'** is determined. The at least two currently measured voltage values **55', 56'** in the current pulse are measured at different times of the same pulse. The system may determine an incorrect stimulation e.g. based on knowledge of the correct voltage drop. The correct voltage drop may be determined by the system manufacturer/operator as an absolute voltage value in Volts (dependent on a first maximum value); or by a ratio of currently measured voltage values with respect to a first maximum value; or a percentage of a first maximum value; or it may be derived from a mathematical model.

In an alternative application the magnetic stimulation device may send a notification concerning the hardware error to the service department and/or manufacturer to repair the device. The magnetic stimulation device may also include a black box for storing data concerning unintended events to provide a statistics for the operator and/or the manufacturer.

The benefit of the application is determining an unintended event within each impulse. Hence patient's safety is significantly improved and the patient and/or the magnetic stimulation device is prevented from heat damage. Additionally, the magnetic stimulation device may be able to provide a notification concerning the unintended event to the operating personnel in human perceptible form. Further benefit is recognizing the type of unintended event.
magnetic field generating device.

In one embodiment, a method of controlling a magnetic stimulation device for treating a biological structure by a time-varying magnetic field includes measuring a voltage of the device over a time interval; relating the measured voltage to a calibration curve; and turning the device off and/or providing a notification to the operating personnel, based on the relating of the measured voltage to the calibration curve. The method may further include determining a correlation coefficient between the measured voltage and the calibration curve; and turning the device off and/or providing a notification to the operating personnel, if the correlation coefficient is below a predetermined value.

In another method of controlling a magnetic stimulation device for treating a biological structure by a time-varying magnetic field, steps include:

measuring a voltage of the device at a time *T1*; relating (i.e., comparing or otherwise determining) a function of the measured voltage at time *T1* to a predetermined calibration voltage at time *T1;* or relating the measured voltage at time *T1* to a predetermined calibration voltage at time *T1* + x; and then turning the device off and/or providing a notification to the operating personnel, based on the relating of the measured output voltage to the predetermined calibration voltage.

Alternatively, a method for detecting incorrect operation of a magnetic stimulation device for treating a biological structure by a time-varying magnetic field includes:

AA.] Determining that a relation between a measured voltage of the device and a calibration curve exceeds a predetermined threshold; or

BB.] Determining that a voltage measured at a predetermined time *T1* and a correct voltage value of a calibration curve at time *T1* exceeds a predetermined threshold; or

CC.] A relation of a first voltage measured at time *T1* and a second voltage measured at time *T1* + *x* exceeds a predefined threshold.

The device is turned off, and/or a notification is provided to the operating personnel, based on the relating of the measured output voltage to the predetermined calibration voltage.

**Heat monitoring**

According to another application of the invention at least one currently determined operation parameter may be used for determining a value of the generated heat. The generated heat may be used for prediction of a temperature of the magnetic stimulation device. Typically the method may be used for treatment planning and/or to predict the temperature of the applicator and/or the part of the magnetic stimulation device which is the most susceptible to overheating such as wires and/or resistors etc.

The magnetic stimulation device may be described by a transition thermal characteristic (TTC). The TTC may be determined by experimental measurement during standard ambient conditions such as temperature and/or pressure, or it may be a mathematical model based on technical and/or electric specifications of all components of the magnetic stimulation device. TTC characterizes the temperature dependence of the magnetic stimulation device on heat. TTC is established by the manufacturer as a factory setting.

The value of generated heat determined by the recited application of the invention corresponds with the treatment parameters. The temperature evolution of the magnetic stimulation device is dependent during the treatment on at least one of treatment parameters, actual temperature of the magnetic stimulation device, ambient temperature, cooling medium temperature, cooling medium flow or heat dissipation.

A calculation algorithm is set up to operate at least TTC and treatment parameters to determine the temperature of the magnetic stimulation device during the treatment. The maximal temperature of the magnetic stimulation device is limited and predetermined. However, in alternative applications the maximal temperature of the magnetic stimulation device may be adjusted by the operator. The maximal temperature may be considered to be safe for the patient.

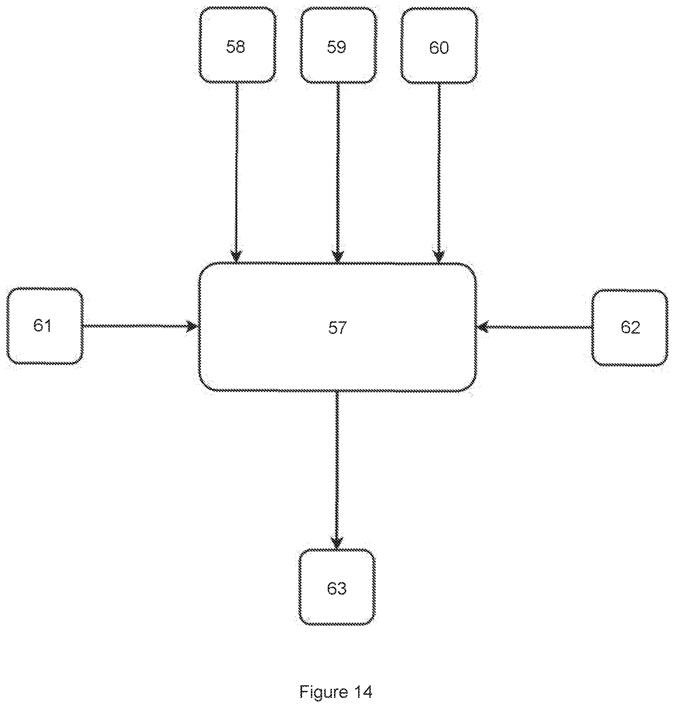

Fig. 14 illustrates a diagram of the calculation algorithm **57** operating with a plurality of inputs. Inputs may include TTC **58;** real and/or theoretical energy loss **59** (e.g. from TTC); at least one treatment parameter **60** such as repetition rate, magnetic flux density, impulse duration, amplitude modulation and/or treatment duration; actual temperature 61 of the magnetic stimulation device; cooling parameters 62 such as ambient temperature, cooling medium temperature, flow and/or pressure gradient, relative humidity, heat capacity and/or heat dissipation. Based on the input parameters the other parameters concerning the treatment may be determined as a result 63. In the preferred application the real energy loss for the at least one pulse may be used.

According to one aspect of the application, the magnetic stimulation device may stop the treatment in the case that the temperature determined by the calculation algorithm exceeds the maximal temperature. If the calculated temperature equals the maximal predetermined temperature then the treatment is started since the maximal predetermined temperature is considered to be safe for the patient. The treatment is stopped only if the calculated temperature exceeds the maximal predetermined temperature.

According to another aspect of the application, the magnetic stimulation device may disable the treatment in the case that the temperature determined by the calculation algorithm exceeds the maximal temperature. In this case the magnetic stimulation device may suggest at least one maximal value of treatment parameter. Based on the predicted temperature of the magnetic stimulation device the calculation algorithm may determine at least one value of treatment parameter to not exceed the maximal temperature of the magnetic stimulation device during the treatment. Based on the operator's preferences the value of the treatment parameter may be automatically adjusted by the magnetic stimulation device or it may be suggested to the operator in human perceptible form such as audibly perceptible notification (e.g. beep) and/or visually perceptible notification (e.g. flashing light, color change etc.). In an exemplary application the suggested treatment parameter may be a maximal achievable value of magnetic flux density which can be sufficiently cooled by the cooling system. However, any other treatment parameter may be suggested to the operator.

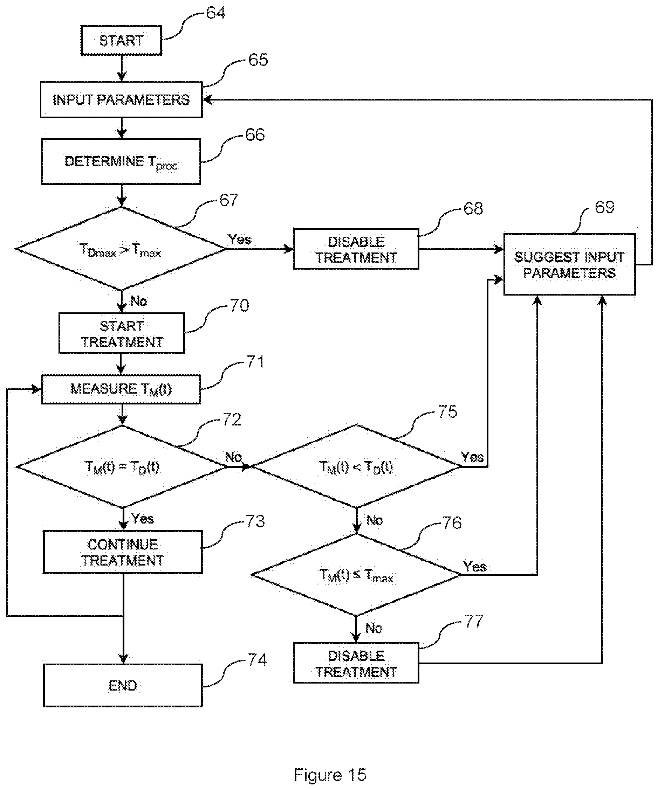

Fig. 15 illustrates a calculation algorithm to determine a maximal magnetic flux density which may be sufficiently cooled by the cooling system. As soon as the magnetic stimulation device is turned on **64** the operator may set the input parameters **65** which are considered by the operator as suitable for the patient. Next step **66** may follow. In the step 66, based on the input parameters the calculation algorithm may determine temperature distribution $T_{proc}$ including at least one of a temperature of the magnetic stimulation device determined in time t of the treatment ($T_D(t)$) and the maximal temperature of the magnetic stimulation device ($T_{Dmax}$) which may be reached during the treatment. In the next step 67, the magnetic stimulation device may determine whether the determined maximal temperature of the magnetic stimulation device exceeds maximal predetermined temperature ($T_{max}$).

In the case that $T_{Dmax}$ exceeds $T_{max}$, in step 68 the treatment may be disabled and/or a notification concerning the reason may be generated by the magnetic stimulation device in a human perceptible form. In the next step 69, the calculation algorithm may determine at least one maximal treatment parameter which may be reached to sufficiently cool the magnetic stimulation device and the magnetic stimulation device may suggest at least one maximal treatment parameter to the operator. Consequently, the operator may input 65 corrected treatment parameters within the acceptable cooling range.

If the magnetic stimulation device determines in the step 67 that $T_{Dmax}$ doesn't exceed $T_{max}$, then the treatment may be started 70. Afterwards, the actual temperature of the magnetic stimulation device ($T_M(t)$) may be measured in step 71. The temperature measurement may be achieved in real time continuously or in discrete time sequences, more preferably in predetermined discrete time values.

In step 72 the magnetic stimulation device may determine whether $T_M(t)$ differs from the determined temperature of the magnetic stimulation device ($T_D(t)$). If $T_D(t)$ equals to $T_M(t)$ then the treatment continues 73 by generating further magnetic impulse and by measuring the further $T_M(t)$ until the end 74 of the treatment and/or until the block 72 examines the difference in $T_D(t)$ and $T_M(t)$.

In the case that $T_D(t)$ and $T_M(t)$ differs in step 72 in consequence step 75 may follow. In step 75 the magnetic stimulation device may examine whether the $T_M(t)$ is lower than $T_D(t)$. If $T_M(t)$ is lower than $T_D(t)$ then the calculation algorithm may determine at least one maximal treatment parameter which may be reached to sufficiently cool the magnetic stimulation device and suggest in step **69** the at least one new maximal treatment parameter to the operator who may adjust the at least one treatment parameter in step **65.** The at least one new treatment parameter may be higher than the at least one originally suggested treatment parameter.

In the case that $T_M(t)$ is not lower than $T_D(t)$ then the magnetic stimulation device may examine in step **76** whether

$T_M(t)$ is lower than or equal to $T_{max}$. If $T_M(t)$ is lower than $T_{max}$ then the calculation algorithm may determine at least one maximal treatment parameter which may be reached in to sufficiently cool the magnetic stimulation device and the magnetic stimulation device may suggest in step **69** the at least one maximal treatment parameter to the operator who may adjust the at least one treatment parameter in step **65.** The at least one new treatment parameter may be lower than the at least one originally suggested treatment parameter. If $T_M(t)$ equals to $T_{max}$ then the magnetic stimulation device may generate the notification that the maximal predetermined temperature was reached.

If the magnetic stimulation device examines in step **76** that $T_M(t)$ is not lower than or equal to $T_{max}$ then the treatment is disabled **77** since the temperature has exceed $T_{max}$ and/or a notification may be generated by the magnetic stimulation device in a human perceptible form. The calculation algorithm may determine at least one maximal treatment parameter which may be reached to sufficiently cool the magnetic stimulation device and suggest in step **69** the at least one maximal treatment parameter to the operator who may adjust the at least one treatment parameter in step **65.**

According to still another aspect of the application, the calculation algorithm may monitor the actual temperature of any part of the magnetic stimulation device, e.g. temperature of the applicator. The temperature of the applicator may be determined by real heat losses during the treatment without needing a temperature sensor in the applicator.

According to still another aspect of the application, the calculation algorithm may determine the cooling parameters, e.g. cooling medium flow, sufficient to cool the heat generated during the treatment. The consumption of the cooling may be optimized.

The calculation algorithm may run in real time. Accordingly, the application may monitor the actual temperature of the magnetic stimulation device and examine whether the treatment corresponds with the result based on determination by the calculation algorithm. The notification in human perceptible form may be generated and/or the treatment may be limited and the at least one treatment parameter may be suggested in human perceptible form, and/or the treatment may be disabled if the difference reaches a predetermined and/or adjustable threshold.

The methods described above may be utilized in any characteristic quantity domain.

The application method is not limited by the recited characteristic quantities. It should be interpreted in the broadest sense.

The application is not limited to the recited operation parameters and/or the characteristic quantity. Any combination of suitable operation parameters and/or characteristic quantities may be used as well. The method should be interpreted in the broadest sense.

The method is not limited to application of a magnetic field hence it may be also used for other equivalent treatment, e.g. radiofrequency treatment, light treatment and/or mechanical treatment such as ultrasound treatment or shock-wave treatment.


**SECTION II**

The apparatus and methods of operating said apparatus may be used for stimulation a target biological structure. The stimulation may achieve various effects.

Following the definition of World Health Organization, health is defined as a state of complete physical, mental and social well-being and not merely the absence of disease or infirmity. However, it seems to be that a state of complete physical, mental and social well-being corresponds rather to happiness than to health. The more appropriate definition of health is by Webster that is the state of being well, sound, or whole, in body, mind, or soul; especially, the state of being free from physical disease or pain.

Electric current is induced in the stimulated biological structure during pulsed magnet treatment. Due to the high value of magnetic flux density the biological structure may be targeted and stimulated more specifically. A distribution of magnetic field is uniform in the biological structure because water and biological molecules are diamagnetic substances. The magnetic field is not affected by diamagnetic substances. Therefore no loss of intensity or magnetic flux density occurs when passing through the biological structure or tissue. Even the scalp and skull are transparent to magnetic fields. Hence magnetic impulses may pass through these structures unimpeded. Particles (e.g. atoms, ions, molecules etc.) in the biological structures are influenced by the magnetic field and permeability of a cell membrane also increases.

An essential principle of magnet therapy used for biological structure stimulation is the influence of the magnetic field on the cell. The cell membrane is polarized due to the induced electric current. One of fundamental phenomenon of electric current in biological tissue may be an action potential occurrence, a transfer of neural excitation and/or a partial or full muscle contraction may be induced. Additionally, the effect of the generated action potential may modulate a painful stimulus transmission, providing a pain management effect. Furthermore, the action potential generation may be used for diagnostics or neural diseases treatment. Convenient repetition rates may cause pain alleviation and/or myorelaxation. Various repetition rates may cause muscle stimulation, stimulation of denervated muscleor may improve movability of a joint. Different repetition rates may improve the patient's mental state.

Advantages of the present magnet therapy include: affecting the deep structures which are problematically stimulated by superficial stimulation; non-invasive or non-contact application of magnetic flux, it may be applied even with clothes; absolute non-invasiveness of the stimulation and elimination of skin irritation in the place of magnetic field application; high rate of acceptability of the stimulation by patients; elimination of stimulation side effects; elimination necessity of applicator made of biocompatible materials; providing a clean and sterile applicator on the highest level; possibility of local or area treatment.

It is to be understood that the method is not limited to the particular applications and that the method may be practiced or carried out in various ways.

Present invention relates to methods using magnetic stimulation of magnetic flux density at least sufficient to cause active response of a biological structure at the repetition rates at least 50 Hz.

The present methods may be used for muscle stimulation and exercising of any muscle or muscle group or any other biological structure, especially for the deep muscles, e.g. psoas major muscle, and the diaphragm. The method may stimulate other biological structures, e.g. selective stimulation of the particular muscle groups for improving their functionality or for generating a contraction pattern from the muscle group for improving the efficiency of the movement or generating the muscle memory. Pelvic floor muscles may be exercised as well to treat e.g. incontinence. Incontinence is a disability of mainly older patients to restrain evacuations of urine or feces. Incontinence is currently treated by exercising pelvic floor muscles or by utilizing vaginal or rectal probes using direct current therapy, or using urologic chairs using stimulation by pulsed magnetic field. However, urologic chairs achieve low magnetic flux density at high repetition rate. Efficacy of urological chairs is low because the values of stimulation parameters, repetition rate and magnetic flux density, are insufficient. Therefore the therapy is relatively time consuming and uncomfortable for the patient.

Another field of application may be treatment of erectile dysfunction. A synergic effect may be also myorelaxation.

The present methods may be used for treatment of major depressive disorder, epilepsy, schizophrenia, Parkinson's disease, Alzheimer's disease, Tourette's syndrome, amyotrophic lateral sclerosis, multiple sclerosis, attention deficit or hyperactivity disorder, obesity, bipolar disorder or mania, anxiety disorders, posttraumatic stress disorder, obsessive compulsive disorder, pain, migraine, chronic pain, neuropathic pain, rehabilitation following stroke (neuro plasticity induction), tinnitus, stimulation of implanted neurons to facilitate integration, substance-related disorders (dependence and abuse and withdrawal diagnoses for alcohol, cocaine, amphetamine, caffeine, nicotine, cannabis), spine injury & regeneration/rehabilitation, head injury, sleep deprivation reversal, primary sleep disorders (primary insomnia, primary hypersomnia, circadian rhythm sleep disorder), cognitive enhancements, dementias, premenstrual dysphoric disorder (PMS), drug delivery systems (changing the cell membrane permeability to a drug), induction of protein synthesis (induction of transcription and translation), stuttering, aphasia, dysphagia, essential tremor, or eating disorders (bulimia, anorexia, binge eating). The present invention relates to methods using stimulation of neural structure by time-varying magnetic field of magnetic flux density sufficient to induce at least action potential and/or active response of the neural structure. The broad spectrum of applications of biological structure stimulation by time-varying magnetic field is achieved due to high repetition rates and/or high value of magnetic flux density. Based on the repetition rate resolution the biological structure may be stimulated by envelope of suitable repetition frequency. Methods may be used for inducing at least action potential generation, action potential modulation and/or causing active response.

The applicator for magnet therapy includes at least one magnetic field generating device for generating time-varying magnetic field. The applicator may or may not include a magnetic core. The applicator is placed proximate to the patient's body and as the electric current flows in the magnetic field generating device the time-varying magnetic field is generated. The magnetic flux density is applied into the biological structure with or without the applicator contacting the skin. The electric current is induced and stimulates the target neural structure. Due to the stimulation at least action potential and/or a partial muscle contraction may occur.

The applicator for magnet therapy is placed proximate to the patient's body. The magnetic flux is applied into the biological structure. The electric current is induced and stimulates the neuromuscular plate. Due to the stimulation at least a partial muscle contraction is caused.

The present method stimulates the neural structure by time-varying magnetic field defined by peak to peak magnetic flux density of at least 0.1 T, more preferably at least 0.5 T, even more preferably at least 1 T, even more preferably at least 1.5 T, most preferably at least 2 T, or up to 7 Tesla on the magnetic field generating device surface and/or repetition rate at least 50, 80, 90, 100, 120, 140, 180, 200, 250 or up to 700 Hertz with treatment/successive treatments lasting several seconds or longer, e.g. at least 5, 10, 30, 60, 120 or 240 seconds, or longer. The impulse duration is in the range of tens to hundreds of $\mu$s.

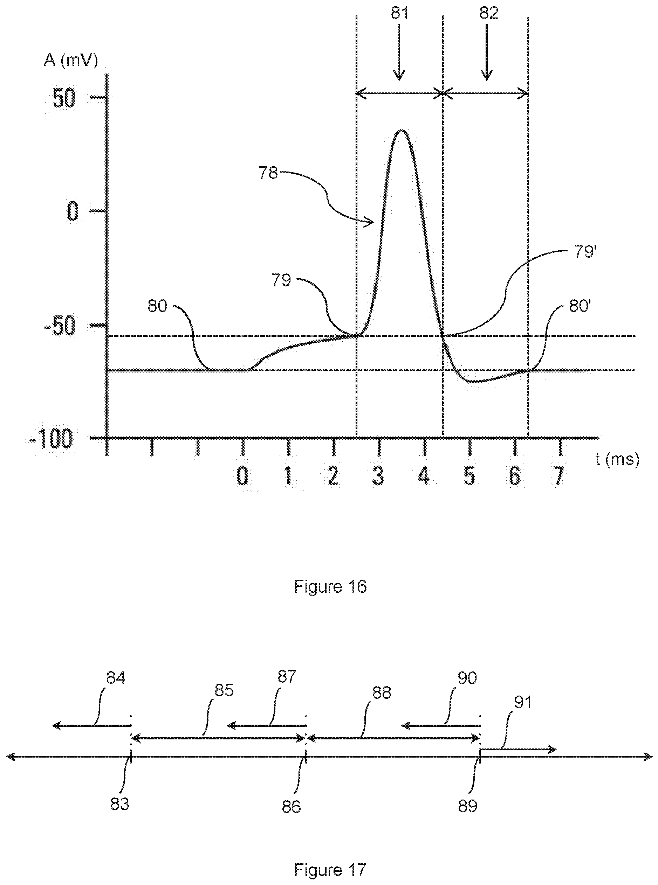

As shown in Fig. 16, the stimulation of the biological structure, a cell, is explained by a curve **78** of an action potential. The action potential of the cell rapidly increases after the stimulus (induced by time-varying magnetic field) and reaches the threshold **79** - so called depolarization. After reaching the maximal amplitude value, the membrane permeability changes and repolarization occurs. The negative value is reached in relation to resting potential **80**. Afterwards the potential recharges back to the value of resting potential **80'**. The time period from the threshold **79** to the return of potential to the threshold **79'** (which equals threshold value **79**) is called absolute refractory period **81**. The cell is not able to be stimulated any more during the absolute refractory period **81**, even by very strong stimulus. The time period from the end of absolute refractory period **81** to resting potential **80'** is called relative refractory period **82**. The cell is able to be stimulated only by the intensive over-threshold stimulus during the relative refractory period **82.** Over-threshold stimulus is a stimulus of higher magnetic flux density than the value of threshold stimulus. The absolute refractory period **81** is the same time duration for all the cells, however, the relative refractory period **82** varies following the cell type.

Referring to Fig. 17, following the perceived intensity of the stimulus the magnetic flux density may reach several values of stimulation. The magnetic flux density may induce sensory-threshold stimulus **83,** below-sensory-threshold stimulus **84,** over-sensory-threshold stimulus **85,** motor-threshold stimulus **86,** below-motor-threshold stimulus **87,** over-motor-threshold stimulus **88,** noxious-threshold stimulus **89,** below-noxious-threshold stimulus **90;** inducing the over-noxious-threshold stimulus **91** is not recommended. In general, the below-threshold stimulus is reached by inducing the threshold stimulus and consequently slightly decreasing the magnetic flux density. The over-threshold stimulus is reached by reaching the threshold stimulus and consequently slightly increasing the magnetic flux density. Therefore the situation may occur, that the value of magnetic flux density inducing the over-sensory-threshold equals to the value of magnetic flux density inducing the below-motor-threshold stimulus. Likewise the situation may occurs, that the value of magnetic flux density inducing the over-motor-threshold stimulus equals to the value of magnetic flux density inducing the below-noxious-threshold stimulus. Following the stimulation process the difference is that the magnetic flux density inducing the over-sensory-threshold stimulus/over-motor-threshold stimulus never reaches the motor-threshold stimulus/noxious-threshold stimulus.

## Modulation

According to one application of the invention, the envelope may be generated. The envelope may include all repetition rates. In the preferred application the envelope may include higher repetition rates, e.g. exceeding 100 Hz, more preferably at least 150 Hz, even more preferably at least 200 Hz, most preferably at least 250 Hz, or up to 700 Hz. The envelope is perceived by the target neural structure as continuous stimulation due to the fact that the neural structure can't distinct single pulses during the stimulation. The lower value of repetition rate is limited by the time duration of the absolute refractory period. Generally, at least two pulses are necessary to create a simple shape of the envelope, e.g. rectangular or trapezoid. However, the more complex envelope shape is the more pulses are needed. The induced energy (IE) stimulating the target neural structure is a function of repetition rate, magnetic flux density and/or impulse duration. The envelope may consist of several impulses called train. The number of pulses in one train varies in range of at least 2 pulses to thousands of pulses. The repetition frequency of envelope is given by the envelope period, i.e. the envelope may include time with no stimulation as well. The envelope may consist of stimulation signals with various burst frequencies, e.g. 5, 10, 20, 50, or more Hz. The envelope may be generated by several modulation approaches.

## MFD modulation

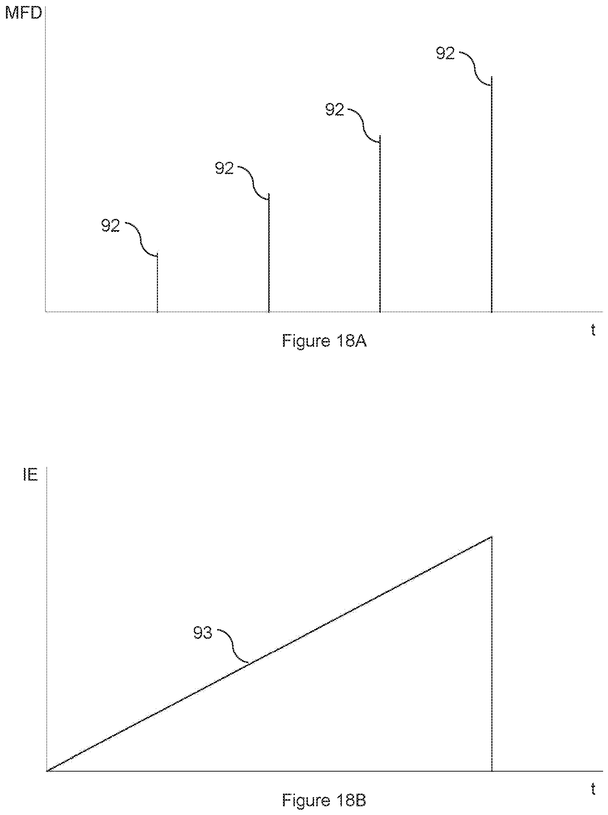

According to one aspect of application, envelope may be generated by time-varying magnetic field of varying peak magnetic flux density hence the process is called magnetic flux density modulation (MFDM). The principle of MFDM is described in Figures 18A and 18B. The repetition rate of the time-varying magnetic field is constant hence the period of the pulse is constant. The impulse duration remains constant as well. However, the magnetic flux density of each impulse 92 varies with respect to the preceding impulse 92, as in Fig. 18A. Therefore each impulse magnetic flux density is different from magnetic flux density of the preceding impulse. The principle is explained by triangular shaped envelope **93** as shown in Fig. 18B.

## RR modulation

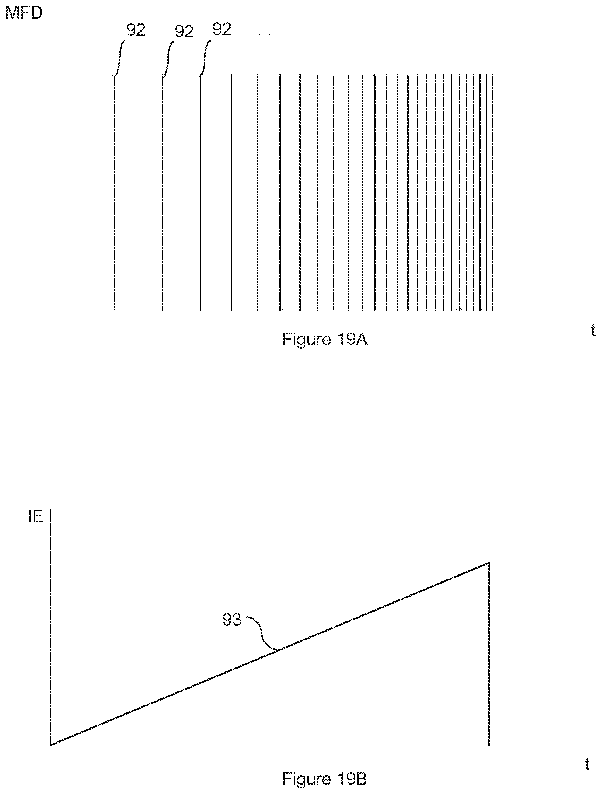

According to another aspect the application, envelope may be generated in repetition rate domain hence the process is called repetition rate modulation (RRM). The principle of RRM is described in Figures 19A and 19B. The magnetic flux density of each impulse **92** remains constants. The impulse duration remains constant as well. Therefore the induced energy for one pulse is constant. However, the repetition rate varies hence the time duration of each pulse varies with respect to the preceding pulse, see Fig. 19A. The actual value of induced energy corresponds to the actual repetition rate of the time-varying magnetic field. When the repetition rate increases the value of induced energy increases or vice versa. The principle is explained by triangular shaped envelope **93,** see Fig. 19B.

## IWM

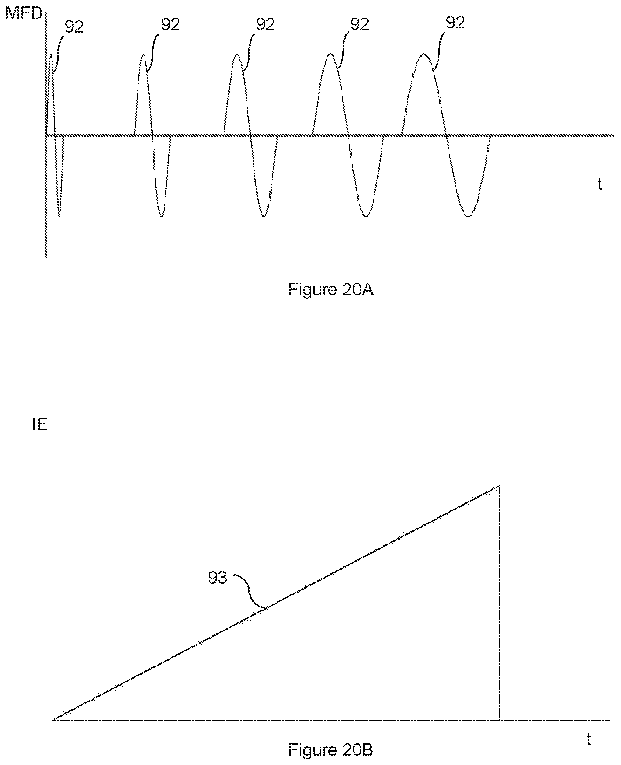

According to still another aspect of the application, envelope may be generated in impulse duration domain. The principle of impulse duration modulation is shown in Figures 20A and 20B where the magnetic flux density and the repetition rate of time-varying magnetic field remains constant. However, the impulse **92** duration of each pulse varies as shown Fig. 20A. The principle is explained by triangular shaped envelope **93** in Fig. 20B.

The modulation approaches are not limited by exemplary waveform. Therefore the envelope may be rectangular, square, saw-tooth, trapezoidal, sinusoidal, exponential etc. Person skilled in the art of neurology and/or physiotherapy may modulate various envelopes and/or envelopes combination.

The application is not limited to use the only single modulation approach. In the preferred application any combination of the upper mentioned approaches may be used.

**Diagnostic sequence**

According to another application, the target biological structure may be stimulated by a pretreatment sequence including a plurality of pulses of different repetition rate. Based on the pretreatment sequence the *optimal/most comfortable* magnetic flux density may be determined for the treatment of the patient. The pretreatment sequence includes at least one repetition rate, more preferably at least two different repetition rates. The complete pretreatment sequence may last up to 120 seconds, more preferably in the range of 1 to 60 seconds, most preferably around 30 seconds.

According to one aspect of the application the pretreatment sequence may include one repetition rate including at least one pulse, more preferably a plurality of pulses, e.g. at least two pulses, more preferably 5 pulses, even more preferably at least 10 or more pulses fired by the magnetic stimulation device. The magnetic flux density may be adjusted by an operator during the pretreatment sequence to provide the patient the *optimal/most comfortable* treatment. This aspect may be used for treatments including at least one repetition rate. This aspect may be also used for treatments including a plurality of repetition rates where the highest repetition rate may be preferably involved in the pretreatment section.

According to another aspect of the application the pretreatment sequence may include a plurality of different repetition rates. The first repetition rate may be the lowest repetition rate of the treatment. The second repetition rate may be the highest repetition rate of the treatment. The at least one pulse, more preferably a plurality of pulses, e.g. at least five pulses, more preferably 10 pulses or more pulses of each repetition rate may be fired by the magnetic stimulation device.

In an exemplary aspect of the application, e.g. a treatment where the lowest repetition rate is 5 Hz and maximal repetition rate is 50 Hz, the pretreatment sequences may include two trains of repetition rate 5 Hz **94** and 50 Hz **95.** Each train may last 1 second and the trains may be separated by time with no stimulation **96.** The trains may alternate all the time of the pretreatment sequence. The complete exemplary pretreatment sequence may last 30 seconds. The magnetic flux density may be adjusted by an operator during the pretreatment sequence to provide the patient the most comfortable treatment. The magnetic flux density of the trains may be the same for at least two trains.

In an alternative aspect of the application, the pretreatment sequence may be in repetition rate domain.

**Psychiatric, psychologic and hormonal application**

Around 20% of the world's children and adolescents suffer for mental diseases or problems. About half of mental diseases begin before the age of 14. Similar types of diseases are being reported across cultures all over the world.

Over 800 000 people die due to suicide every year and suicide is the second leading cause of death in the patients in the age of 15-29. 75 % of suicides occur in low- and middle-income countries. Neural diseases and use of alcohol contribute to many suicides around the world. Early identification and effective treatment are crucial to ensure that people receive the successful care they need.

According to another application of the invention, the neural structure stimulation by time-varying magnetic field may be used for treatment of psychiatric diseases. The successive repetition rate of the present method reaches at least 100 Hz, more preferably at least 110 Hz, most preferably at least 120 Hz. The magnetic flux density of the present treatment method exceeds at least 0.1 T, more preferably at least 0.5 T, even more preferably at least 1 T, most preferably at least 2 T or up to 7 T.

The method may be used for treatment of psychiatric diseases, such as e.g. depression, schizophrenia, migraine, depression, dyskinesias, Alzheimer's disease, Asperger's syndrome, Tourette's syndrome, attention-deficit hyperactivity disorder (ADHD), cognitive impairment, dementia, dystonia, restless legs, tremor, aphasia, ataxia or sphincter of Oddi disorders.

The applicator is placed in the proximity of the stimulated neural structure, e.g. brain, to focus the magnetic flux density to the targeted neural structure. The targeted neural structure is most commonly e.g. prefrontal cortex for alleviating the symptoms of depression or schizophrenia. However, person skilled in the art of neurology and/or physiotherapy may apply the stimuli to other locations which stimulation cause similar effect. Furthermore, the magnetic flux density and/or the repetition rate may be sufficient to induce at least action potential or even a partial seizure in the CNS.

The most commonly stimulated neural structure for migraine or headache treatment is trigeminal or occipital nerve. The trigeminal or occipital nerve stimulation is able to prevent and treat migraines.

The stimulation by time-varying magnetic field may also stimulate and/or temper the production of hormones, e.g. hypophysis hormones. However, person skilled in the art of neurology and/or physiology may apply the stimuli to other locations to influence the hormone production of other glands or visceral organs as well.

Additionally, the treatment of substance addiction may be promoted by the present method.

According to an alternative approach of the application for psychiatric diseases treatment, the neural structure may be stimulated by low repetition frequency envelope created by time-varying magnetic field of repetition rate exceeding 100 Hz, more preferably at least 150 Hz, even more preferably at least 200 Hz, most preferably at least 250 Hz, or up to 700. The repetition frequency may be up to 350 Hz, more preferably, up to 200 Hz, even more preferably up to 100 Hz.

## Neurodegenerative disease application

According to another application of the invention, the neural structure stimulation by time-varying magnetic field may be used for treatment of neurodegenerative diseases and deep neural structure using repetition rates over 100 Hz, and a repetition rate of e.g., 130 - 160 Hz. However, the repetition rate may also exceed 200 Hz or 260 Hz, the repetition rate may reach up to 700 Hz. The impulse duration is in the range of 10 to 700 $\mu$s, more preferably in the range of 30 to 500 $\mu$s, even more preferably 50 to 250 $\mu$s, most preferably in the range of 60 - 90 $\mu$s. Magnetic flux density necessary for the effect of application is at least 0.1 T, more preferably at least 0.5 T, even more preferably 1 T, even more preferably at least 2.5 T, or up to 7 T.

The application of the invention may be used for treatment of neurodegenerative diseases, such as e.g. Alzheimer's disease, Parkinson's disease, Huntington's disease, Asperger's syndrome, Tourette's syndrome amyotrophic lateral sclerosis, depression, quadriplegia, paraplegia, dyskinesias, multiple sclerosis, attention-deficit hyperactivity disorder (ADHD), cognitive impairment, dementia, dystonia, restless legs, tremor, aphasia, ataxia, insufficient blood flow in the brain and many other diseases, disorders or syndromes. The stimulation by time-varying magnetic field is targeted to the damaged nerve or CNS to treat essential symptoms of the disease. The method may stimulate deep neural structures, e.g. brain structures. The effect of the stimulation may be similar to the effect of conventional invasive deep brain stimulation using implanted electrodes into the proximity of nucleus basalis of Meynert.

According to alternative approach of the application for neurodegenerative diseases treatment, the neural structure may be stimulated by low repetition frequency envelope created by time-varying magnetic field of repetition rate exceeding 100 Hz, more preferably at least 150 Hz, even more preferably at least 200 Hz, most preferably at least 250 Hz, or up to 700 Hz. The repetition frequency may be up to 350 Hz, more preferably, up to 200 Hz, even more preferably up to 100 Hz.

According to another aspect of the application for treatment of neurodegenerative diseases, the neural structure stimulation by time-varying magnetic field may be used for alleviating symptoms of e.g. Parkinson's disease. The neural structure may be stimulated by envelope of repetition frequency in the range of 40 to 60 Hz, more preferably in the range of 45 to 55 Hz, most preferably around 50 Hz. The magnetic flux density is sufficient to induce at least motor-threshold stimulus, more preferably at least over-motor-threshold stimulus. The treatment time may last for at least 20 minutes, more preferably at least 30 minutes, most preferably up to 60 minutes.

As the present methods for stimulation by time-varying magnetic field into deep neural structures are non-invasive, the disadvantages of surgery along with operative and post-operative risks for the patient are avoided.

## Diagnostics

According to another application of the invention, the neural structure stimulation by time-varying magnetic field may be used for neural system diagnostics. The neural structure may be stimulated by time-varying magnetic field generating various envelopes. The magnetic flux density of the present treatment method exceeds at least 0.1 T, more preferably at least 0.5 T, even more preferably at least 1 T, most preferably at least 2 T or up to 7 T.

According to one aspect of the application in neural system diagnostics, the neural structure may be stimulated by single pulses. The operator stimulates a patient by predefined different repetition rates to determine minimal magnetic flux density inducing at least partial muscle contraction for each repetition rate value. Feedback registering the at least partial muscle contraction may be e.g. visual, or by using technical device. Based on the measurement the graph of dependency of magnetic flux density and repetition rate may be compiled. The graph is very useful in the therapy planning.

In an alternative application the neural structure may be stimulated by rectangular or increasing shaped envelope as well.

According to another aspect of the application in neural system diagnostics, the neural structure may be stimulated by single pulses or rectangular shaped envelope, and by increasing shaped envelope. Both envelopes are used for determination of minimal magnetic flux density value sufficient to induce at least partial muscle contraction. The envelope duration may last 1 second. After the determination of both magnetic flux densities values, the level of muscle denervation may be calculated.

According to still another aspect of the application in neural system diagnostics, the neural structure may be stimulated in the at least one predefined location and in another predefined location the response of the stimulus may be measured. Hence the reaction time may be determined. In the preferred application, the time-varying magnetic field stimulates at the at least one predefined location on the arm and the recording device is placed on the hand. The recording device may be contact electrode or non-contact magnetic measuring device, e.g. superconducting quantum interference device. The feedback may be determined visually as well. The preferred repetition rate value for stimulation is in the range of 1 to 50 Hz and the impulse width is in the range of tens to hundreds of μs. In an alternative application the stimulation may be in predefined location, e.g. brain, and the feedback may be registered visually. It may be used planning neural surgery or during the surgery.

According to still another aspect of the application in neural system diagnostics, the neural structure may be stimulated during the maximal voluntary contraction. The additional contraction originating from stimulation by time-varying magnetic field is superposed. Several neural disorders may be determined, e.g. conduction deficiency, based on the determination of the additional muscle contraction strength. The contraction may be isometric, concentric or eccentric.

According to alternative aspect of the application in neural structure diagnosis, the neural structure may be stimulated by low repetition frequency envelope created by time-varying magnetic field of repetition rate exceeding 100 Hz, more preferably at least 150 Hz, even more preferably at least 200 Hz, most preferably at least 250 Hz, or up to 700 Hz. The repetition frequency may be up to 350 Hz, more preferably, up to 200 Hz, even more preferably up to 100 Hz.

**Analgesics**

Following the definition by World Health Organization, pain is defined as an unpleasant sensory or emotional experience associated with actual or potential tissue damage, or described in terms of such damage.

Pain may be caused by spastic and/or overtoned muscle, hence the myorelaxation effect may be cause analgesic effect as well.

According to still another application of the invention, the neural structure stimulation by time-varying magnetic field may be used for pain management. In general, the method is focused on neural structure stimulation by time-varying magnetic field of repetition rate at least 100 Hz and/or magnetic flux density sufficient to induce and/or modulate an action potential in a stimulated neural structure. The neural structure may be stimulated by at least repetition rate 100 Hz, more preferably at least 120 Hz, most preferably at least 140 Hz. The stimulation causes the pain alleviating effect. The principle is stimulation of neural structures which are able to transmit pain excitement. The purpose of the present stimulation by time-varying magnetic field is pain relief. The alternative effect may be a suppression of pain excitement transmission at different levels of receptors or CNS. Further, the higher repetition rate may include myorelaxative effect induced by short magnetic flux density impulses.

The application of the invention may be used for alleviation of e.g. back pain, Guillain Barre syndrome, diabetic polyneuropathy, dental procedure pain, knee osteoarthritis, anesthesia (pain relief during surgery), angina (chest pain from heart disease), ankylosing spondylitis, back pain, bum pain, cancer pain, chronic pain, dysmenorrhea (painful menstruation), headache, hemiplegia, hemiparesis (paralysis on one side of the body), labor pain, local anesthesia during gallstone lithotripsy, facial pain, trigeminal neuralgia, bruxism (tooth grinding) pain, myofascial pain, neck and shoulder pain, pain from broken bones, rib fracture or acute trauma, diabetic peripheral neuropathy, phantom limb pain, post-herpetic neuralgia (pain after shingles), irritable bowel syndrome, postoperative pain, post-stroke rehabilitation, rheumatoid arthritis, skin ulcers, temporomandibular joint pain, spinal muscular atrophy (in children), pain during hysteroscopy, carpal tunnel syndrome, soft tissue injury, intermittent claudication, knee replacement pain, achalasia, bursitis, esophageal spasm, fibromyalgia, fracture pain, herpes, hip pain, interstitial cystitis, joint pain, local anesthesia, menstrual cramps, muscle cramps, muscle spasticity, muscle strain or pain, musculoskeletal trauma, myofascial pain dysfunction syndrome, nerve damage, osteoarthritis, pain medication adjunct, pancreatitis, sacral pain, shingles, shoulder subluxation, sports injuries, thrombophlebitis, whiplash and neuralgias, central and peripheral neuropathic pain, spinal stenosis, sphincter of Oddi disorders or repetitive strain injuries.

Pain is transmitted by several types of pain conducting fibers differing by myelin sheath thickness and by the properties of the fiber. The magnetic flux density threshold of major pain fibers is written in Table 1.

Table 1

| Fiber | Diameter | Myelin sheath thickness | Magnetic flux density threshold |
|-------|----------|-------------------------|----------------------------------|
| Aα | Large | Large | Low |
| Aδ | Medium | Small | Medium |
| C | Small | No myelin | High |

**EP 4 574 201 A2**

There are several theories of pain management. Pattern theory premises that the pain excitement is transmitted from the peripheral receptor to the CNS in a pattern coded signal and the pain is interpreted by decoding the signal in CNS. Another pain management approach reveals gate-control theory based on a premise that the pain is transmitted through a "gate" in substantia gelatinosa in spinal dorsal horn wherein the large-diameter fibers block the "gate", whereas small-diameter fibers open the gate. Last pain management theory is called release of endogenous opioids based on the effect of endorphins, enkephalins and dynorphins. The secretion of these three endogenous opioids is activated by stimulation of A$\delta$-and C-fibers by low repetition rate time-varying magnetic field or by the low repetition rate envelope.

According to one aspect of the application in pain management, the neural structure may be stimulated by time-varying magnetic field at low repetition rate to cause pain management via stimulating central neural system or any part of it, e.g. anterior cingulate cortex or gyrus. The repetition rate may be at least several Hz, more preferably at least 10 Hz, even more preferably at least 20 Hz, even more preferably at least 50 Hz.

According to one approach of the aspect of the application in pain management, the neural structure may be stimulated by an envelope created by higher repetition rate values, e.g. exceeding 100 Hz, more preferably at least 150Hz, even more preferably at least 200Hz, most preferably at least 250Hz, or up to 700 Hz. The envelope may be generated with predefined repetition frequency and/or shape. Furthermore, the neural structure, e.g. central neural system, may be stimulated by envelope at low repetition frequency at least 1 Hz, more preferably at least 2 Hz, even more preferably at least 5 Hz, even more preferably at least 10 Hz, even more preferably at least 200 Hz, or up to 350 Hz. The neural structure may be stimulated by various envelope shapes. The peak to peak magnetic flux density of the time-varying magnetic field may reach at least 0.1 T, more preferably at least 0.5 T, even more preferably at least 1 T, even more preferably at least 1.5 T, most preferably at least 2 T or up to 7 Tesla on the magnetic field generating device surface.

According to another aspect of the application in pain management, the neural structure may be stimulated by time-varying magnetic field at repetition rate in the range of 80 to 120 Hz, more preferably 90 to 110 Hz, even more preferably 95 to 105 Hz, most preferably around 100 Hz, with magnetic flux density inducing at least below-sensory-threshold stimulus or more preferably at least sensory-threshold stimulus following the application and indication. The method may stimulate the large diameter neural fibers. In general, time-varying magnetic field of repetition rate around 100 Hz with magnetic flux density inducing at least sensory-threshold stimulus is used preferably for pain relief effect. In a preferred application the magnetic flux density may induce the at least sensory-threshold stimulus and the time-varying magnetic field is modulated in the repetition rate range of 80 and 120 Hz. The method may be used for e.g. pain management in rheumatology or for treating posttraumatic states. In another preferred application the time-varying magnetic field repetition rate is at least 90 Hz, more preferably at least 100 Hz, even more preferably at least 110 Hz. The magnetic flux density may induce at least sensory-threshold stimulus, more preferably at least over-sensory-threshold stimulus to cause analgesic effect. The method may be used e.g. for chronic pain relief or Sudeck syndrome treatment amongst other.

In still another preferred application the method is used for treatment of phantom pain. The magnetic flux density may induce below-sensory-threshold stimulus. The treatment lasts at least 20 minutes, more preferably in the range of 25 to 30 minutes, or longer.

According to still another aspect of the application in pain management, the neural structure may be stimulated by time-varying magnetic field at sufficient magnetic flux density and at repetition rate up to 700 Hz, more preferably up to 500 Hz, most preferably up to 250 Hz to locally relief the pain and to enhance the patient comfort without taking pain killers or analgesics due to selected effect to nerve endings. The impulse duration is less than 1 ms, more preferably less than 800 $\mu$s, most preferably in the range of tens to hundreds of $\mu$s. The longer the impulse is the stronger subjective contraction intensity is perceived.

According to one approach of the aspect of the application in pain management, the neural structure may be stimulated in constant magnetic flux density manner by repetition rate in the range of 50 to 250 Hz, more preferably at least 100 Hz, most preferably at least 110 Hz and the pulse duration preferably at least 70 $\mu$s, more preferably at least 100 $\mu$s, most preferably at least 250 $\mu$s or up to 1000 $\mu$s. The magnetic flux density is sufficient to induce at least sensory-threshold stimulus, more preferably at least over-sensory-threshold stimulus. Quick adaptation to stimulus occurs during the stimulation in time therefore the magnetic flux density may be adjusted during the procedure following the patient's needs and perception. The method may be indicated for e.g. pain relief or for burns treatment.

According to another approach of the aspect of the application in pain management, the neural structure may be stimulated by a time-varying magnetic field to induce electric current of random repetition rate fluctuation around a predefined repetition rate. In the preferred application the repetition rate fluctuates at least 5 %, more preferably at least 15 %, most preferably at least 30 % or up to 50 %, the magnetic flux density is sufficient to induce at least sensory-threshold stimulus, more preferably at least over-sensory-threshold stimulus of the neural structure. The neural structure is not able to adapt for the stimulation by time-varying magnetic field since the repetition rate varies. Additionally, the magnetic flux density may be adjusted during the time. The method may provide analgesic effect.

According to still another approach of the aspect of the application in pain management, the neural structure may be stimulated by trains **97** of several pulses **98** and time with no stimulation after the train **97.** The group of several pulses **98**

29

and the time **99** with no stimulation is called burst **100**. Therefore one burst **100** consists of the only one train **97** and time with no stimulation **99.** The train **97** preferably consists of at least 2 pulses, more preferably 5 pulses, even more preferably tens pulses or up to hundreds pulses; repetition rate of the pulses **98** is at least 100 Hz. The burst repetition rate may vary following the patient's needs. In the preferred application the burst repetition rate vary from 1 to 10 Hz. The number of pulses **98** in train **97** and/or the time with no stimulation **99** may vary following the patient needs. The magnetic flux density is sufficient to induce at least motor-threshold stimulus, more preferably over-motor-threshold stimulus, even more preferably below-noxious-threshold stimulus.

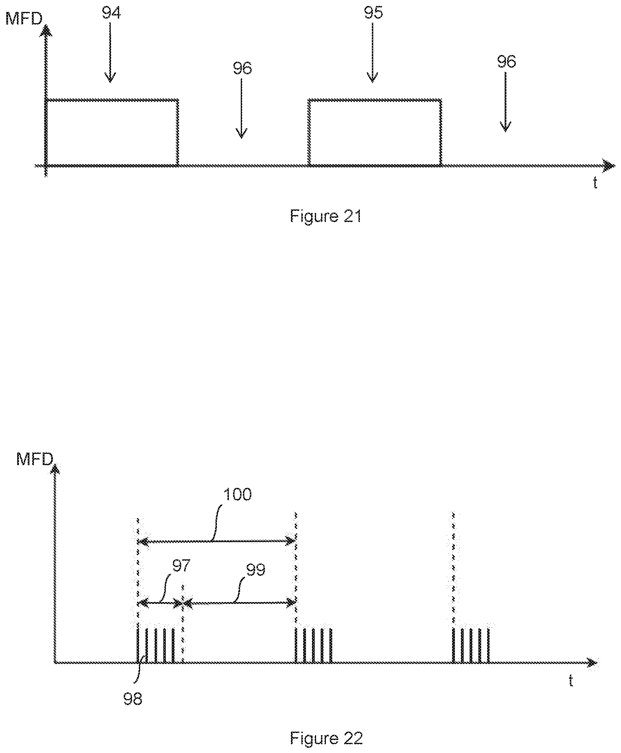

Fig. 22 shows an example of application of the stimulation by time-varying magnetic field with a repetition rate of 125 Hz in train **97** consisting of 5 pulses **98,** with a burst repetition rate 10 Hz. Total time duration of one burst **100** is 100 ms. Total stimulation time of one train **97** is 40 ms, hence the time with no stimulation **99** is 60 ms. The most important advantage of this approach is analgesic effect and almost no adaptation of the neural structure to the stimulation. The approach may be used e.g. for alleviating the acute pain.

The treatment program, envelope shape and magnetic flux density are indicated individually following the patient's needs and his/her subjective perception. The present method may be applicable for treatment of e.g. post-operative pain, labor pains, acute and chronic pain, neuralgia and neuropathia, musculoskeletal pains, sciatica, arthritis, knee or ankle pain, tendonitis, lower back pain, chronic headache, trigeminal pain, thalamic pain, causalgia, cancer pain, angina pectoris (reduces angina attacks and nitroglycerine consumption), metastatic bone pain, pain caused by burn injury, postoperative nausea, suprapubic or perineal pain, dysmenorrhea, postpartum pain, phantom pain, stump pain, anxiety, or for improving breath parameters via improving tidal inspiration volume and vital capacity etc.

According to still another aspect of the application in pain management, the neural structure may be stimulated by time-varying magnetic field at repetition rate in the range of 80 to 240 Hz, more preferably in the range of 100 to 200 Hz and by magnetic flux density inducing at least over-sensory-threshold stimulus, more preferably at least motor-threshold or over-motor-threshold stimulus inducing the at least partial muscle contraction, the noxious-threshold stimulus mustn't be induced. The stimulation may cause analgesic effect and the method may be used for treatment of e.g. causalgia, backbone pains, headache, migraine or thalamic, phantom or posttraumatic pains.

According to still another aspect of the application in pain management, the neural structure may be stimulated by time-varying magnetic field for alleviating the pain via spinal cord and/or spinal nerve stimulation. The aspect of the application may be based on the pattern theory of pain perception. The time-varying magnetic field may be delivered to the target area of backbone and/or spine. Specifically, the method exploits benefits and pain alleviating effect of stimulation with repetition rate at least 125 Hz, more preferably at least 135 Hz, most preferably around 145 Hz or up to 170 Hz. The magnetic flux density is sufficient to induce at least motor-threshold stimulus, more preferably below-noxious-threshold stimulus. Additionally, during the application at least partial muscle contraction may occur hence the local perfusion may increase. Still another benefit of the presented aspect may be myorelaxative effect, long lasting analgesic effect which may be used e.g. for alleviating pain after injuries, in orthopaedics, rheumatology, migraine, headache, neck pain, lumbago, pain of upper or lower extremities and improving the perfusion of itself. Furthermore, various pain syndromes may be treated by the present method.

According to still another aspect of the application in pain management, the neural structure may be stimulated by time-varying magnetic field at repetition rate of at least 160 Hz, more preferably at least 170 Hz, most preferably around 180 Hz, or up to 250 Hz. The present method may be based on gate-control theory of pain. In the preferred application the impulse duration lasts 500 $\mu$s at repetition rate around 180 Hz. Optimal magnetic flux density is sufficient to induce at least motor-threshold stimulus, more preferably below-noxious-threshold stimulus. The magnetic flux density may be adjusted following the patient's needs based on the sensitivity of a patient and the depth of the stimulated neural structure. However, the minimal magnetic flux density exceeds 0.1 T, more preferably 0.5 T, even more preferably 1 T, most preferably up to 7 T to induce the at least one action potential. Benefit of the present method is myorelaxative effect. The stimulated location may be situated over the backbone, e.g. over cervical, thoracic, lumbar and sacral regions. Several stimulation positions are established to alleviate a pain in different locations or extremities. The most significant usage may be for alleviating chronic pain, pain originating from coxarthrosis or gonarthrosis, posttraumatic states or rheumatic pain.

According to still another aspect of the application in pain management, the neural structure may be stimulated by time-varying magnetic field at repetition rate at least 180 Hz, more preferably at least 200 Hz, even more preferably up to 240 Hz for alleviating the pain caused by e.g. causalgia, backbone pains, headache, migraine or thalamic, phantom or posttraumatic pains. The magnetic flux density is sufficient to induce at least motor-threshold stimulus and then the stimulation may continue up to below-noxious-threshold stimulus.

**Analgesic effect caused by modulated stimulation**

According to still another aspect of the application in pain management, the neural structure may be stimulated by envelope at lower repetition frequencies, e.g. below 100 Hz. Therefore the time-varying magnetic field repetition rate reaches the higher values, e.g. exceeding 100 Hz, more preferably at least 150Hz, even more preferably at least 200Hz, most preferably at least 250Hz, or up to 700 Hz in order to generate the envelope with repetition frequency up to 350 Hz, more preferably, up to 200 Hz, most preferably below 100 Hz.

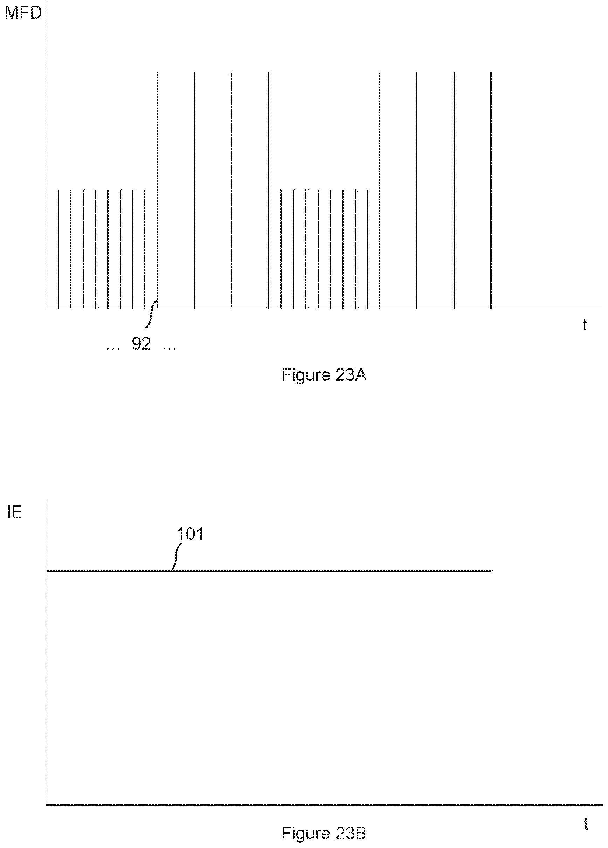

According to one approach of the aspect of the application in pain management, the neural structure may be stimulated by the high repetition rate time-varying magnetic field. The envelope **101** remains constant, see Fig. 23B, and may cause analgesic effect. The constant envelope **101** may be generated by e.g. constant repetition rate and magnetic flux density and impulse duration, or any combination of modulation approaches, e.g. varying magnetic flux density and repetition rate of impulses **102** (see Fig. 23A). The magnetic flux density is sufficient to induce at least sensory-threshold stimulus, more preferably at least over-sensory-threshold stimulus, most preferably at least motor-threshold stimulus. The method may be used for alleviating a pain caused by neuropathy, neuralgia, or for innervation deficiency treatment, treatment of paresis or plegia. Additional effects may be improved metabolism or perfusion. The perfusion diseases may be treated as well, e.g. Raynaud's syndrome.

According to another approach of the aspect of the application in pain management, the neural structure may be stimulated by high repetition rate time-varying magnetic field generating low repetition frequency envelope up to 20 Hz, more preferably in the range of 1 to 15 Hz, most preferably in the range of 2 to 10 Hz. The magnetic flux density is sufficient to induce at least below-sensory-threshold stimulus, more preferably at least sensory-threshold stimulus, even more preferably motor-threshold stimulus or even noxious-threshold stimulus. The target neural structures are large diameter neural fibers in the case of lower magnetic flux densities. In the case of higher magnetic flux densities the target neural fibers are small diameter fibers. The impulse duration is at least 100 $\mu$s, more preferably at least 300 $\mu$s, even more preferably at least 500 $\mu$s or up to 1000 $\mu$s. The stimulation of small diameter fibers induces the increase of secretion of endogenous endorphins.

According to still another approach of the aspect of the application in pain management, the neural structure may be stimulated by high repetition rate time-varying magnetic field generating low repetition frequency envelope in the range of 20 to 40 Hz, more preferably around 30 Hz. The magnetic flux density is sufficient to induce at least below-sensory-threshold stimulus, more preferably at least sensory-threshold stimulus, the repetition frequency remains preferably constant. The approach may be applied for pain alleviation in rheumatology or posttraumatic states.

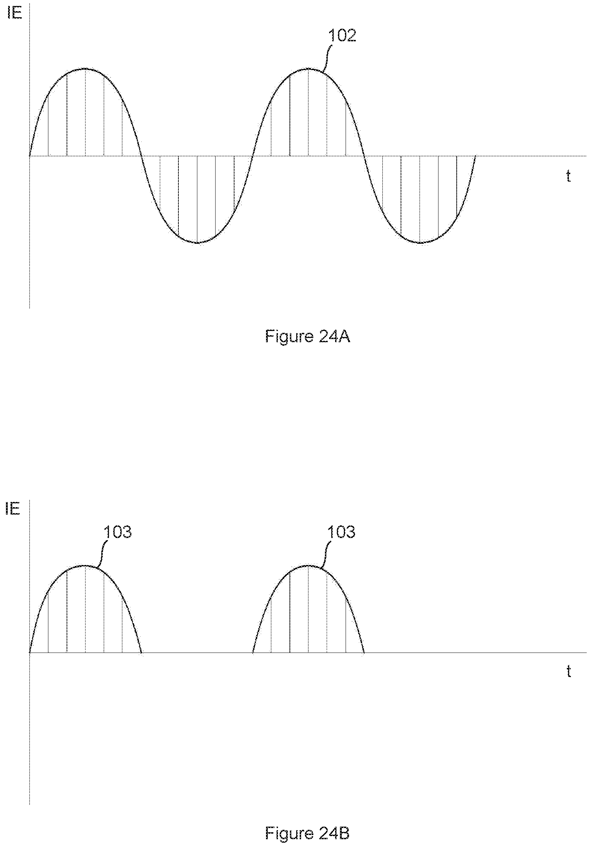

According to still another approach of the aspect of the application in pain management, the neural structure may be stimulated by monophasic envelope or by different wave rectification of biphasic envelope **102** (BE) as shown in Fig. 24A.

The first envelope **103** (FE) is half-wave rectified BE **102.** Therefore the repetition frequency of FE **103** is the same as the repetition frequency of the original BE **102**. Further, FE **103** includes magnetic stimulation for the same time duration as the original BE **102,** the second time duration of the period is no stimulation, e.g. the FE **103** includes half-period of sinusoidal and second half-period with no stimulation in the case of symmetric BE **102** (see Fig. 24B). The effect of FE **103** may be rather stimulating, toning or excitatory. However, it may cause analgesic effect by magnetic flux density sufficient to induce at least below-sensory-threshold stimulus, more preferably at least motor-threshold stimulus.

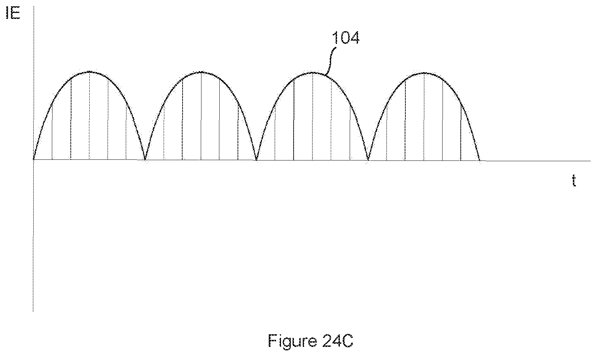

The second envelope **104** (SE) is full-wave rectified BE **102.** SE **104** includes magnetic stimulation for entire period. Therefore, in the case of symmetric BE **102** the repetition frequency of SE **104** is double repetition frequency of the original BE **102.** (see Fig. 24C). The SE **104** effect may be rather inhibitory, e.g. analgesic or relaxation. Following the pain alleviating effect the muscle contraction is not painful during the increased magnetic flux density of the time-varying magnetic field. The both envelopes, FE and/or SE, may be superposed on the constant envelope (CE) inducing at least below-sensory-threshold stimulus. The superposition of CE and FE and/or SE may induce at least sensory-threshold stimulus, more preferably at least motor-threshold stimulus.

FE and/or SE may be combined in one treatment. The ratio of FE and SE may vary as well. The amplitude and/or repetition frequency of both envelopes may vary as well.

The present approach is contactless and may be used over the painful joints, paravertebrally, over Head areas, over the painful point, according to the nerve, over the organ or muscle.

The present approach may be applied e.g. for treatment of pain, neuralgia, arthrosis pain, spondylosis, periarthritis, epicondylitis, myalgia, lumbago, torticollis, posttraumatic states, Raynaud's disease, Sudeck's syndrome, varicosities or pain in abdominal region.

According to still another approach of the aspect of the application in pain management, the neural structure may be stimulated by various envelopes. The envelope repetition frequency is in the range of 40 to 120 Hz, more preferably in the range of 50 to 100 Hz. The magnetic flux density reaches sufficient value to induce at least below-sensory-threshold stimulus, more preferably at least sensory-threshold stimulus, even more preferably over-sensory-threshold stimulus. The impulse duration is at least 20 $\mu$s, more preferably at least 50 $\mu$s, even more preferably at least 100 $\mu$s, most preferably up to several hundreds of $\mu$s. The treatment duration may be at least 20 minutes or up to 60 minutes.

According to still another approach of the aspect of the application in pain management, the neural structure may be stimulated by envelope generated by two repetition rates interference. The first repetition rate may differ from the second repetition rate. In the exemplary embodiment the first repetition rate may be 500 Hz and the second repetition rate may be 490 Hz. The interference of the two repetition rates generates envelope of repetition frequency given by the repetition rate difference hence the interference stimulation repetition frequency may be 10 Hz in the exemplary application.

The effect of the modulated repetition frequency in the range up to 10 Hz may cause pain relief effect by selective application the time-varying magnetic field to the neural structures, e.g. C-fibers. The effect of the stimulation may increase the opioid generation to relief the pain. In alternative application the modulated time-varying magnetic field repetition frequency may be around 100 Hz, e.g. in the range of 80 to 120 Hz, more preferably in the range of 90 to 110 Hz. The higher value of repetition frequency induces spasmolytic effect. The repetition frequency in the range of 85 to 105 Hz, more preferably in the range of 90 to 100 Hz may be used to spasmolytic stimulation of spastic muscles, in the preferred application the repetition frequency may vary. In another preferred application the repetition frequency is constant around 100 Hz, the application may be used for treatment of e.g. spastic obstipation.

The advantages of the present methods compared to conventional electrotherapy methods are: non-invasive and contactless application; high rate of treatment acceptability by a patient; elimination of electrotherapeutic side-effect such as heat production in proximity of the stimulation electrode; and low neural structure stimulation adaptability for these aspects of the application.

**Myorelaxation**

According to still another application of the invention, the neural structure stimulation by time-varying magnetic field may be used for myorelaxation. The neural structure may be stimulated by at least repetition rate at least 100 Hz, more preferably at least 110 Hz, even more preferably at least 120 Hz, most preferably 130 Hz. The neural structure may be stimulated by various time-varying magnetic field shapes. The magnetic flux density may be sufficient to cause at least motor-threshold stimulus and at least partial muscle contraction. In general, the myorelaxation effect may be reached by higher repetition rates and/or relatively short impulse duration in the range of tens to several hundreds of $\mu$s.

The application of the invention may be used for treatment of e.g. postoperative ileus (bowel obstruction), over-active bladder, Guillain Barre syndrome, quadriplegia, paraplegia, dyskinesias, paresthesias, knee osteoarthritis, anesthesia (pain relief during surgery), bruxism (tooth grinding) pain, myofascial pain, neck and shoulder pain, irritable bowel syndrome, temporomandibular joint pain, spinal muscular atrophy (in children), chronic obstructive pulmonary disease rehabilitation, achalasia, bursitis, esophageal spasm, fibromyalgia, menstrual cramps, muscle cramps, muscle spasticity, muscle strain or pain, musculoskeletal trauma, myofascial pain dysfunction syndrome, sacral pain, thrombophlebitis, central and peripheral neuropathic pain, spinal stenosis, repetitive strain injuries or detrusor instability.

Further application of magnet therapeutic method using sufficient magnetic flux density at high repetition rates over 100 Hz is focused especially on spastic muscle and its trigger point. The method proposes stimulation of muscle hypertonia of the overloaded muscle fibers to relieve a local spasm (the hypertonia in stimulated muscle fiber is relieved). The method affects the muscle insertion as well. The method may even affect the whole muscle group for specific movement.

The stimulation by pulsed magnetic field is divided into two separate periods. The neuromuscular plate is stimulated by pulsed magnetic field during the first period. The magnetic flux density is sufficient to induce at least motor-threshold stimulus to cause at least partial muscle contraction in the stimulated biological structure. The muscle is activated by isometric contraction and sedation follows. The most reactive fibers are selectively inhibited. During the second period the repetition rate is increased to at least 100 Hz, 150 Hz, or 200 Hz. The muscle is relaxed due to high repletion rate. The method is used for high-quality relaxation of at least one muscle fiber.

The method may be used also in sport medicine for stretching of athletes before a performance and muscle relaxation after the performance, thereafter it significantly contributes to muscle regeneration. Further applications are treating for muscle imbalance or muscle relaxation caused by overload, pain relief or elimination and preparation the muscle for physical activity.

According to one aspect of the application in myorelaxation, the neural structure may be stimulated by higher repetition rates over 100 Hz. The neural structure stimulation may be focused on e.g. spastic muscle and its trigger point. Proposed stimulation may enable muscle hypertonia alleviation of the overloaded muscle fibers to relieve a local spasm. Similarly it may stimulate the muscle insertion or muscle group as well.

According to the one approach of the aspect of the application in myorelaxation, the stimulation by time-varying magnetic field may be divided into two separate periods, where the neuromuscular plate is stimulated by time-varying magnetic field during the first period. The magnetic flux density is sufficient to induce at least motor-threshold stimulus to cause at least partial muscle contraction in the stimulated biological structure. The muscle is activated by isometric contraction by repetition rate below 100 Hz and sedation follows. The most reactive fibers are selectively inhibited. During the second period the repetition rate is increased to at least 100 Hz, 150 Hz, or 200 Hz. The muscle is relaxed due to high repletion rate. The method is used for high-quality relaxation of at least one muscle fiber.

According to another aspect of the application in myorelaxation, the neural structure may be stimulated by higher repetition rates in the range of 150 to 210 Hz, more preferably in the range of 165 to 195 Hz, even more preferably in the range of 175 to 185 Hz, most preferably around 180 Hz. The magnetic flux density is sufficient to induce at least over-sensory-threshold stimulus, more preferably motor-threshold, even more preferably over-motor-threshold stimulus. The impulse duration is at least tens of $\mu$s, more preferably at least 250 $\mu$s, most preferably at least 500 $\mu$s. The time duration of a pulse may be at least 1 ms, more preferably at least 2.5 ms, most preferably around 5.5 ms. A temporary reflex adjustment of at least one hypertonic muscle fiber or muscle or muscle groups may be achieved by the application of time-varying magnetic field, thereafter myorelaxation effect may be provided.

According to still another aspect of the application in myorelaxation, the neural structure may be stimulated by time-varying magnetic field at repetition rate in the range of 150 to 250 Hz. The magnetic flux density is sufficient to induce at least over-sensory-threshold stimulus, more preferably motor-threshold, even more preferably over-motor-threshold stimulus.

According to one approach of the aspect of the application in myorelaxation, the neural structure may be stimulated by time-varying magnetic field at repetition rate in the range of 160 Hz to 200 Hz, even more preferably in the range of 170 Hz to 190 Hz, even more preferably in the range of 175 to 185 Hz, most preferably around 180 Hz.

According to another approach of the aspect of the application in myorelaxation, the neural structure may be stimulated by time-varying magnetic field at repetition rate in the range of 175 to 225 Hz, even more preferably in the range of 180 to 220 Hz, most preferably around 200 Hz. In a preferred application the time-varying magnetic field at repetition rate 200 Hz is constant containing no modulation.

According to still another approach of the aspect of the application in myorelaxation, the neural structure may be stimulated by time-varying magnetic field at repetition rate up to 250 Hz, more preferably up to 235 Hz, even more preferably up to 225 Hz, most preferably up to 220 Hz.

**Myorelaxation effect caused by modulation**

According to still another aspect of the application in myorelaxation, the neural structure may be stimulated by envelope at lower repetition frequencies, e.g. below 100 Hz. Therefore the time-varying magnetic field repetition rate reaches the higher values, e.g. exceeding 100 Hz, more preferably at least 150Hz, even more preferably at least 200Hz, most preferably at least 250Hz, or up to 700 Hz in order to generate the envelope with repetition frequency up to 350 Hz, more preferably, up to 200 Hz, most preferably below 100 Hz.

According to one approach of the aspect of the application in myorelaxation, the neural structure may be stimulated by envelope of repetition frequency range up to 100 Hz, more preferably up to 50 Hz, most preferably up to 30 Hz. The magnetic flux density is sufficient to induce at least over-sensory-threshold stimulus, more preferably motor-threshold, even more preferably over-motor-threshold stimulus.

According to one approach of the aspect of the application in myorelaxation, the neural structure may be stimulated by time-varying magnetic field at repetition rate in the range of 30 to 150 Hz, more preferably in the range of 50 to 100 Hz. The time-varying magnetic field is preferably modulated to prevent the neural structure to adapt the magnetic stimulation. The magnetic flux density is sufficient to induce at least over-sensory-threshold stimulus, more preferably motor-threshold, even more preferably over-motor-threshold stimulus

All the mentioned aspects of the application of the invention causing myorelaxation effect may also include analgesic effect. However, the patient may perceive different subjective intensity of analgesic effect for the specific aspects of the application of the invention.

The application of the invention may be also used in sport medicine for stretching of athletes before a performance and muscle relaxation after the performance, thereafter it significantly contributes to muscle regeneration. Further applications are treating for muscle imbalance or muscle relaxation caused by overload, pain relief or elimination and preparation the muscle for physical activity.

**Stimulation**

According to still another application of the invention, the neural structure stimulation by time-varying magnetic field may be used for causing stimulation effect. The neural structure may be stimulated by the envelope hence the repetition rate exceeds 100 Hz, more preferably at least 150 Hz, even more preferably at least 200 Hz, most preferably at least 250 Hz or up to 700 Hz. The envelope may be generated with predefined repetition frequency. Further, neural structure may be stimulated by low repetition frequency envelope to induce at least motor-threshold stimulus and at least partial muscle contraction occurs. The magnetic flux density of the present treatment method exceeds at least 0.1 T, more preferably at least 0.5 T, even more preferably at least 1 T, most preferably at least 2 T or up to 7 T. In an alternative application of the invention the stimulation effect may be caused by stimulating CNS.

The application of the invention may be used for muscle stimulation in general, however, the application may be used for treatment of e.g. urinary incontinence, fecal incontinence, Guillain Barre syndrome, quadriplegia, paraplegia, hemiplegia, hemiparesis (paralysis on one side of the body), post-stroke rehabilitation, spinal cord injury, spinal muscular atrophy (in children), gastroparesis, chronic obstructive pulmonary disease rehabilitation or spinal stenosis as well. The application of the invention may also improve the breath parameter such as tidal inspiration volume.

According to one aspect of the application in muscle stimulation, the at least partial muscle contraction may be caused by neural structure stimulation at low repetition rate via stimulation of central neural system or any part of it, e.g. brain or motor cortex. The repetition rate may be at least several Hz, more preferably at least 10 Hz, even more preferably at least 20 Hz, even more preferably at least 30 Hz, most preferably at least 50 Hz. The magnetic flux density may reach at least 0.002 T, more preferably at least 0.1 T, even more preferably at least 1 T, most preferably at least 2 T, or up to 7 T.

According to another approach of the aspect of the application in pain management via CNS stimulation, the neural structure may be stimulated by envelope at lower repetition frequencies, e.g. at least 1 Hz, more preferably at least 2 Hz, even more preferably at least 5 Hz, even more preferably at least 10 Hz, or up to 350 Hz. Therefore the time-varying magnetic field repetition rate reaches the higher values, e.g. exceeding 100 Hz, more preferably at least 150 Hz, even more preferably at least 200 Hz, most preferably at least 250 Hz, or up to 700 Hz in order to generate the envelope.

The aspect of the application in muscle stimulation may have great benefit in treatment of motoric and/or mobility dysfunctions, e.g. the treatment may accelerate recovery after stroke.

According to another aspect of the application in muscle stimulation, the neural structure may be stimulated by envelope of repetition frequency up to 10 Hz, more preferably in the range of 0.5 to 5 Hz. The low repetition frequency may cause the stimulation effect to smooth muscle. The application is beneficial for treatment of digestive diseases, e.g. obstipation.

According to still another aspect of the application in muscle stimulation, the neural structure may be stimulated by envelope of repetition frequency in the range of 20 to 40 Hz, more preferably in the range of 25 to 35 Hz, most preferably around 30 Hz. The pulses are formed in trains of duration up to hundreds of ms, more preferably up to 500 ms, even more preferably in the range of 40 to 400 ms. The application may be useful for stimulation of patients suffering for hemiplegia.

According to still another aspect the application in muscle stimulation, the neural structure may be stimulated by increasing envelope. Repetition rates over 50 Hz, 100 Hz, 150 Hz, or 200 Hz are favorable mainly for treatment of denervated muscle. Denervated muscle lacks the ability of voluntary contraction due to a lesion or nerve degradation caused by e.g. polio or trauma, so that the signals from the central neural system are not received. The muscle loses the ability of contraction and/or flexibility and it atrophies. Effects of muscle atrophy are significant just after three weeks of inactivity. Due to ineffective muscle contraction the function of the denervated muscle is performed by the synergist muscles. Therefore the synergists are overloaded and many dysbalances may occur. Additionally, the overloaded muscle become tough and hard and pain may occur as well. The present method provides contactless method for prevention and treatment of muscle dysbalances caused by denervated muscle.

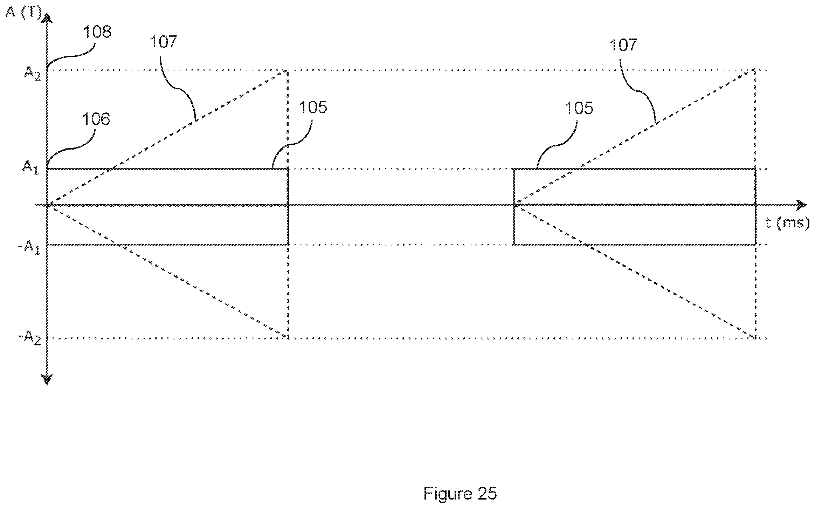

The stimulation of denervated muscle is based on the adaptability of health motor unit for the raising magnetic flux density. Denervated muscle lacks an ability of adaptation to raise induced electric stimulus as normal healthy muscle. Hence the denervated muscle may be stimulated by low magnetic flux density. Fig. 25 illustrates the different shapes of the envelope of the stimulation signal and corresponding different threshold values of a healthy muscle. The time duration of the envelope may last at least 6 ms, 10 ms or 20 ms. When the healthy muscle is stimulated by a rectangular envelope **105** the muscle contraction may occur at magnetic flux density $A_1$ **106**. When the healthy muscle is stimulated by increasing envelope **107** the muscle contraction may occur at magnetic flux density value $A_2$ **108**. However, when the denervated muscle is stimulated by increasing envelope **107** the denervated muscle contraction may occur at magnetic flux densities below $A_2$ **108**. Magnetic flux density value $A_2$ **108** is multiplication of magnetic flux density value $A_1$ **106,** wherein the multiplication coefficient is positive number greater than 1.

The stimulation results in an at least partial contraction of denervated muscle and the contraction of healthy muscle is eliminated or minimized.

According to one approach of the aspect of the application in muscle stimulation, the neural structure may be stimulated by the increasing or triangular envelope of repetition frequency in the range of 25 to 65 Hz, more preferably in the range of 35 to 55 Hz, even more preferably in the range of 40 to 50 Hz, most preferably around 45 Hz. In an alternative application the period may include a time with no stimulation, however, the repetition frequency of complete envelope remains as mentioned before. The method may be used for stimulation of denervated muscle structure in advance.

According to still another aspect of the application in muscle stimulation, the neural structure may be stimulated by envelope of repetition frequency in the range of 20 to 80 Hz, more preferably in the range of 30 to 70 Hz, most preferably in the range of 40 to 60 Hz. An application may e.g. use repetition rate at least 35 Hz, more preferably at least 40 Hz, most preferably at least 45 Hz, the envelope may be rectangular. The stimulation may be used for e.g. muscle exercising and/or strengthening or muscle reeducation after injuries. Another application may be used for muscle fatigue testing. In another application the envelope repetition frequency is at in the range of 40 to 60 Hz, more preferably in the range of 45 to 55 Hz, most preferably around 50 Hz. In the most preferred application the envelope is rectangular shaped and lasts for 10 ms with 10 ms time of no stimulation.

According to still another aspect of the application in muscle stimulation, the neural structure may be stimulated by monophasic envelope or by different wave rectification of biphasic envelope **102** (BE) as shown in Fig. 24A.

The first envelope **103** (FE) is half-wave rectified BE **102.** Therefore the repetition frequency of FE **103** is the same as the repetition frequency of the original BE **102.** Further, FE **103** includes magnetic stimulation for the same time duration as the original BE **102,** the second time duration of the period is no stimulation, e.g. the FE **103** includes half-period of sinusoidal and second half-period with no stimulation in the case of symmetric BE **102** as shown in Fig. 24B. The effect of FE **103** may be rather stimulating, toning or excitatory. However, it may cause analgesic effect by magnetic flux density sufficient to induce at least below-sensory-threshold stimulus, more preferably at least motor-threshold stimulus.

The second envelope **104** (SE) is full-wave rectified BE **102.** SE **104** includes magnetic stimulation for entire period. Therefore, in the case of symmetric BE **102** the repetition frequency of SE **104** is double repetition frequency of the original BE 22. (see Fig. 24C). The SE **104** effect may be rather inhibitory, e.g. analgesic or relaxation. Following the pain alleviating effect the muscle contraction is not painful during the increased magnetic flux density of the time-varying magnetic field. The both envelopes, FE and/or SE, may be superposed on the constant envelope (CE) inducing at least below-sensory-threshold stimulus. The superposition of CE and FE and/or SE may induce at least sensory-threshold stimulus, more preferably at least motor-threshold stimulus.

FE and/or SE may be combined in one treatment. The ratio of FE and SE may vary as well. The amplitude and/or repetition frequency of both envelopes may vary as well. The optimal value of magnetic flux density is sufficient to induce at least sensory-threshold stimulus, more preferably at least motor-threshold stimulus.

In an alternative approach of the aspect of the application in muscle stimulation the FE may be interrupted by the time period with no stimulation lasting for at least 0.1 second, more preferably at least 0.5 second, most preferably at least 1 second, the continual repeating of FE may last for at least 0.1 second, more preferably at least 0.5 second, most preferably at least 1 second as well. In another alternative approach the periodic FE and SE alternation is also applicable.

According to still another approach of the aspect of the application in muscle stimulation, the neural structure may be stimulated by alternation of FE and SE in ratio 1:1. In alternative approach FE and SE may alternate in at least 0.1 second, more preferably at least 0.5 second, most preferably at least 1 second lasting periods of each envelope. The resulting stimulation is called modulated with short periods (MSP). Therefore the MSP period is as the burst of FE and/or SE. In the preferred application the repetition of MSP is up to 20 Hz, more preferably up to 15 Hz, most preferably up to 10 Hz.

The present method may also be used for functional joint blockade treatment. A joint may include muscular structure providing the movement of the joint, joint itself including synovial fluid. Functional joint blockade may be caused by spastic muscles in the vicinity of the joint whose secondary effect is pain. The most common functional joint blockades are in the backbone. These can cause vertebrogenic problems, headache and backbone pain, migraine, perfusion ailment resulting to dizziness, backbone sharp pain directing to limbs, visual insufficiency, tinnitus, toothache or earache etc. The scoliosis, sacral pain (even after fractures), weakness of pelvic floor muscles and incontinence, urine retention or constipation or functional sterility may also be treated by treatment of functional joint blockade.

For the purpose of functional blockade treatment and joint movability improvement the preferred repetition rates may be at least 50 Hz, 60 Hz, 70 Hz or 100 Hz, magnetic flux density at least 0.2 T, 0.4 T, 0.5 T, or at least 1 T or up to 7 T.

Formerly the functional joint blockades were treated by manual positioning, traction, mobilization of the soft tissues, reflex therapy or even pharmacologically. The present method achieves at least partial muscle contraction in the vicinity of the joint, e.g. backbone, by stimulation of neuromuscular plate by pulsed magnetic field at low repetition rate and high magnetic flux density providing at least partial muscle contraction. The joint contact surfaces are moved and the joint is moved due to at least partial muscle contractions of muscles in the vicinity of the joint, e.g. the muscles in the vicinity of the backbone are represented by rotators which causes local microrotations of the backbone. The functional joint blockade is unblocked via muscle mobilization by mechanical approach.

Still another approach of the present method is affecting the rheological properties of synovial fluid by pulsed magnetic field. The total effect of this method may be synergic. Although the method is explained on example of backbone functional joint blockade, the application of the method is not limited to the backbone. A person skilled in anatomy or physical therapy is able to apply the method for any other joint provided by sufficient amount of neuromuscular structures in vicinity. The present method may be preferably used in combination with analgesic methods. It is very convenient to use the present method in combination with method providing myorelaxation since the functional joint blockade is caused by spastic biological structures in vicinity.

According to still another aspect of the application in muscle stimulation, the neural structure may be stimulated by envelope generated by two repetition rates interference. The first repetition rate may differ from the second repetition rate. In the exemplary embodiment the first repetition rate may be 500 Hz and the second repetition rate may be 490 Hz. The interference of the two repetition rates generates envelope of repetition frequency given by the repetition rate difference hence the interference signal repetition frequency may be 10 Hz in the exemplary application. The effect of the modulated repetition frequency in range up to 10 Hz may cause stimulation effect by the magnetic flux density induce at least sensory-threshold stimulus, more preferably motor-threshold stimulus.

**Special applications**

According to still another application of the invention, the neural structure stimulation by time-varying magnetic field may be used for causing healing effect of a biological structure. The neural structure may be stimulated by time-varying magnetic field at repetition rate advantageously up to 175 Hz, more preferably up to 150 Hz, most preferably in the range of 50 to 130 Hz and magnetic flux density sufficient to induce at least below-sensory-threshold stimulus, more preferably at least sensory-threshold stimulus. The suitable treatment duration may be up to 120 minutes, more preferably up to 90 minutes, most preferably in the range of 30 to 60 minutes. Following the applied stimulus polarity the effect may treat inflammatory biological structures or tissue, or may improve the treatment of non-inflammatory tissue.

According to still another application of the invention, the neural structure stimulation by time-varying magnetic field may be used for improving the sleep patterns or habits. The neural structure may be stimulated by time-varying magnetic field at repetition rate in the range of 150 to 300 Hz, more preferably in the range of 175 to 250 Hz, most preferably around 200 Hz. In the preferred application the magnetic flux density is sufficient to induce at least below-sensory-threshold stimulus, more preferably at least sensory-threshold stimulus to cause sedation effect which may be used for improving the sleep patterns or habits.

According to still another application of the invention, the neural structure stimulation by time-varying magnetic field may be used for edema treatment.

According to one aspect of the application in edema treatment, the neural structure may be stimulated by repetition rate at least 80 Hz, more preferably at least 100 Hz, most preferably at least 120 Hz, by pulse duration in the range of tens to several hundreds of $\mu$s, and magnetic flux density sufficient to induce at least below-sensory-threshold stimulus, more preferably at least sensory-threshold stimulus. The suitable treatment duration may be up to 60 minutes, more preferably up to 45 minutes, most preferably in the range of 15 to 30 minutes. The application may additionally treat inflammation as well.

According to another aspect of the application in edema treatment, the neural structure may be stimulated by envelope of repetition frequency up to 75 Hz, more preferably in the range of 3 to 60 Hz, even more preferably in the range of 10 to 50 Hz, most preferably in the range of 20 to 40 Hz to cause edema reduction. The stimulation may activate the muscle pump hence the target area perfusion may be improved and the lymphatic circulation may be improved as well.

According to still another aspect of the application in edema treatment, the neural structure may be stimulated by envelope of repetition frequency up to 70 Hz, more preferably up to 60 Hz, most preferably in the range of 30 to 50 Hz. The magnetic flux density is sufficient to induce at least sensory-threshold stimulus, more preferably at least motor-threshold stimulus to cause edema reduction. The stimulation may be indicated in cases of e.g. limited movement of the biological structure. The suitable treatment duration may last up to 60 minutes, more preferably up to 45 minutes, most preferably in the range of 15 to 30 minutes.

The described applications of the invention may be used for treatment of e.g. obesity, hypertension, pain, pregnancy-related nausea or vomiting, postoperative nausea or vomiting, atopic eczema, dry mouth, enhanced blood perfusion of the uterus and placenta, hemophilia, pruritus, labor induction, Raynaud's phenomenon, sickle cell anemia pain, skin flap ischemia (during plastic surgery) or tinnitus (ringing in the ear).

All the described applications of the invention may also provide trophotropic, anti-oedematous or placebo effect which contributes to improving the patient's well-being and comfort. Local metabolism may be increased as well.

All the mentioned applications may be applied in constant repetition rate and/or repetition frequency manner. The envelope modulation or combination thereof is applicable as well.

The values of magnetic flux density and repetition rate are cited in several preferred applications since the perception of the stimulation is subjective. Nevertheless, the magnetic flux density and repetition rates and/or repetition frequencies are not limited by the recited values. A person skilled in physical therapy is able to repeat and apply the therapy methods adjusting the magnetic flux density, repetition rate and/or frequency following the patient's needs. Further the applications may be used in combinations, e.g. diagnostic application and/or stimulation application.

A person skilled in the physical therapy is able to use various envelopes and waveform, e.g. pulse, sinusoidal, rectangular, square, triangular, saw-tooth, trapezoidal, exponential etc. The invention is not limited to recited shapes of envelopes.

Stimulation of biological structure by time-varying magnetic field following the recited methods may be but not limited to continuous, pulsed, randomized, or in bursts. The pulse may be but not limited to monophasic, symmetric, asymmetric, most preferably biphasic.

**SECTION III**

The apparatus and methods of operating the apparatus may be used for stimulation a target biological structure to achieve at least one esthetical effect.

One subsection of the third section discloses the methods used for aesthetic treatment of the patient.

During last few decades patient have not only wanted to be in good health, they have also wanted to look-well, i.e. to be well-shaped, without any unattractive fat and to have a young appearance, without wrinkles, stretchmarks or sagging breasts. This has resulted in a progressive evolution of invasive aesthetic methods such as aesthetic surgery removing and remodeling the human body by invasive and potentially dangerous methods, e.g. liposuction or implants. The side effect of invasive methods may be scars. The side effects resulted in the rapid progress in non-invasive method, e.g. lipolysis or removing skin imperfections.

Due to increased permeability of the cell membrane, the pulsed magnet treatment effects especially these processes and can induce following effects: at least partial muscle contraction; reduction of adipose tissue - volume and/or number of the adipose cells; neogenesis and/or remodeling of collagen and/or elastin fibers; improving circulation of blood and/or lymph and improves local and/or adipose tissue metabolism.

With the present methods, factors for enhancing visual appearance of the body are: stimulation of major muscle, e.g. gluteus maximus; stimulation of deep muscle which is enabled by high value of magnetic flux density; non-contact application of magnetic flux density, it may be applied even through clothing; stronger muscle contraction due to higher value of magnetic flux density; higher-quality of muscle targeting; treatment is not influenced by small movements during treatment; treatment time duration is shortened due to high value of magnetic flux density and/or higher repetition rate; no delays occur.

It is to be understood that the method is not limited to the particular applications and that the method may be practiced or carried out in various ways.

Present method is especially applied for enhancing the visual appearance of body parts including or proximate to major muscle structures. Further the method is applicable for enhancing the visual appearance of patients with high value of BMI. The present method is not limited to the application of the stimulation signal to major muscle. Muscles other than major muscles may be stimulated as well.

The applicator of magnetic stimulation signal is placed proximate to the patient's body. As used here, proximate to includes both contactless and in actual contact with the skin of the patient. The muscles are selectively stimulated by the stimulation signal and the magnetic flux density of the stimulation may be adjusted following the patient's feeling or needs. The treatment time is shortened due to selective stimulation of the correct muscles. Additionally, due to the high value of magnetic flux density, the muscle is stimulated more effectively. Further, the treatment may be non-invasive or even preferably contactless due to the high value of magnetic flux density. The patient may be treated without removing clothing, reducing patient discomfort. Additionally, following the high efficiency of the muscle contraction the collagen and/or elastin fibers above the muscle structure are remodeled, hence the visual appearance is enhanced.

The magnetic stimulation of the biological structure has various applications for enhancing visual appearance of the contour body area. High density magnetic field reaches such values which may be used for: adipose tissue reduction, wherein the adipose tissue reduction is achieved by reduction of number and/or volume of adipose cells; muscle toning, wherein the muscle appearance enhancement is achieved by adipose tissue reduction with no muscle bulking; muscle shaping, wherein the muscle appearance enhancement is achieved by adipose tissue reduction and/or muscle bulking; body contouring, wherein the silhouette appearance enhancement is achieved by adipose tissue reduction with no muscle bulking; body shaping, wherein the silhouette appearance enhancement is achieved by adipose tissue reduction and/or muscle bulking; skin tightening, wherein the skin appearance enhancement is achieved by obtaining smoother and younger appearance, including wrinkles reduction; cellulite treatment, wherein the appearance enhancement is achieved by adipose tissue reduction, muscle contraction and/or elastic fibers neogenesis; circumferential reduction, wherein the reduction is achieved by adipose tissue reduction and/or the muscle bulking; breast enhancement, wherein the appearance enhancement effect is achieved by elevation or shape modification; lip enhancement, wherein the lip appearance enhancement is achieved by obtaining fuller and firmer appearance.

The applicator for magnet treatment is placed proximate to the patient's body. The magnetic flux density is applied into the target biological structure. Electric current is induced and stimulates the neuromuscular plate and/or the nerve innervating the at least one muscle fiber. The stimulation causes at least a partial muscle contraction.

The present method stimulates the biological structure by pulsed magnetic field. The peak to peak magnetic flux density on the magnetic field generating device surface is at least 0.15, 0.2, 0.8, 1.5, 2, 2.4 or up to 7 Tesla at repetition rate at least 1, 10, 30, 50, 55, 60, or up to 700 Hertz with treatment/successive treatments lasting several seconds or longer, for example, for at least 5, 10, 30, 60, 120 or 240 seconds, or longer. The pulse width is in the range of tens to hundreds of $\mu$s.

Cellulite is an effect of skin change resulting in orange peel appearance. The cause of the cellulite is orientation of collagen fibers in so called "fibrous" septae. The fibrous septae contract and harden over time creating a dimple effect. Additionally, blood and lymphatic vessels lack circulation due to the contraction and hardening of the septae. The lymph flow may be blocked resulting in swelling. Another cause of cellulite is adipose cells protruding to dermis.

One application of time-varying magnetic field for enhancing the visual appearance of body area may be stimulation of a muscle by magnetic flux density for reducing the cellulite. The magnetic flux density is delivered through the skin to the neuromuscular plate and/or nerve innervating at least one muscle fiber. The electric current is induced in the target biological structure causing at least partial muscle contraction. The at least partial muscle contraction causes the movement of the skin and all the biological structures subtending epidermis. Additionally, the at least partial muscle contraction improves blood circulation by itself, or via the movement of the muscle in the vicinity including fibrous septae. Additionally, blood and/or lymph circulation is improved in the layers subtending epidermis since the muscle contraction moves the fibrous septae. Also local and/or adipose tissue metabolism is improved. The at least partial muscle contraction is more effective for adipose tissue metabolism as the value of magnetic flux density increases since the muscle contraction is stronger. The higher magnetic flux density effects the higher number of muscle fibers contraction and the more adipose tissue is reduced. Therefore the visual appearance of regions prone to cellulite is enhanced.

The method causes the circumferential reduction i.e. a reduction of the size of the treated body area. The method is mostly indicated for the regions with cellulite, especially for buttocks, abdomen, hips, thighs or arms. However, the indication is not limited to the mentioned regions and the method may be used for stimulation of any other body area.

The present method may provide a massage effect via the stimulation which is caused by the at least partial muscle contraction. Therefore the massage effect may be achieved by contactless methods instead of manual massage techniques or soft tissue techniques. The massage effect improves lymph circulation.

In another aspect, improvement of functionality and/or the appearance of the muscle is achieved with results similar to body exercise. The results are achieved by application of high magnetic flux density to the body area and inducing at least partial muscle contraction. Higher values of magnetic flux density applied result in a stronger muscle contraction. The patient feels firmer and tighter.

With the present method muscle contractions induced by the applied magnetic flux density may help to tone the muscle providing a more attractive appearance. As the muscle structure is stimulated by time-varying magnetic field the entire limb may be moved due to the high power of the magnetic stimulation signal. Nevertheless, the method is not limited to the applications to the limbs and the method is able to be applied to stimulation of any muscle, e.g. gluteus maximus or any muscle/deep muscle to induce body contouring and/or body shaping effect and fat burn. Additionally, shortened and/or flabby muscles are stretched. The physical fitness of the patient is improved as well.

The present methods may also induce muscle contraction to reduce effect of skin laxity. Skin laxity may be caused by e.g. the aging process or increasing number and/or volume of adipose cells which pulls down the skin by gravity, rapid weight loss or skin stretching during the pregnancy. The muscles are stimulated by the induced electric current to contract. Repetitive contractions cause the muscles to obtain the tonus and flexibility. Therefore the skin appearance is enhanced by stimulating the flabby muscles. The effect of skin tightening is achieved. The method also stimulates the creation of the collagen and elastin fibers in the layers subtending the epidermis hence the skin obtains enhanced visual appearance. The method may be widely applied but not limited to application to the regions of neck, breasts, arms or abdomen. The method provides the smoother and younger appearance of the skin to the patient.

Similar methods of stimulation the muscle structure by time-varying magnetic field for inducing the at least partial muscle contraction may be used for treatment of wrinkles as well. Wrinkles are results of extrinsic and intrinsic factors. Nowadays, wrinkles are considered to be negative effect of natural aging process which decreases the production of collagen and elastin fibers and weakens the skin which becomes thinner. As the muscle stimulation by the magnetic flux density induces at least partial muscle contraction, the stimulation of collagen and elastin fibers neogenesis is improved. Additionally, the muscles subtending the stimulated region are toned and the skin gets a younger and enhanced visual appearance. Therefore, the effect of skin tightening is achieved.

Wrinkles may be prevented or reduced by practicing facial exercises which causes a massage effect to the facial tissues, improving blood and lymph circulation. Additionally, the facial muscles are relaxed and toned after the exercise.

A similar effect as facial exercise may be achieved by non-invasive and/or contactless method of stimulating the facial muscles by magnetic flux density. Further additional advantage of the present method is the improvement of restoration of the collagen and elastin fibers, more effective toning and strengthening of the facial muscles.

The present methods may improve the neogenesis and remodeling of collagen fibers in the lips to reach a full, plump and firmer appearance. The magnetic flux density is applied to the lips by an applicator. Therefore the lips become fuller and firmer without any need of invasive method such as injection of the synthetic fillers, permanent makeup or the facial implants. The present method stimulates the remodeling and/or neogenesis of collagen fibers in a natural way. Additionally, the collagen is natural substance of the human body which provides the elasticity to the structure.

The present methods may be used for enhancing the visual appearance of breasts. Cooper's ligament may be stimulated, improved and/or firmed by the at least partial muscle contraction. The muscle stimulation induces the elevation of the breast tissue. Additionally, the breast tissue is stimulated to be modified in a shape, wherein the shape includes the size and/or the contour of the breast tissue. Therefore the visual appearance is enhanced and breasts are more attractive for the patient. The present method is a non-invasive alternative for current aesthetic surgery method for the treatment of sagging breast tissue. The present method provides a patient a method of breast visual appearance enhancement without surgery. Therefore the method lacks post-surgery complications such as scars, postoperative pain or long recovery period. Various treatment protocols may be used.

Following the recited methods the stimulation signal may be but is not limited to continuous, pulsed, randomized or burst. The impulse may be but not limited to monophasic, polyphasic and/or biphasic.

In the preferred application of the present method the trains of pulses, called bursts are used.

Repetition rate and/or magnetic flux density may vary during the treatment protocol. Further the magnetic stimulation signal may include several periods of stimulation signal of different repetition rates, therefore the modulation of the signal is in repetition rate domain. The stimulation signal may include several periods of stimulation signal of different magnetic flux densities, therefore the modulation of the signal is in magnetic flux density domain. In yet another approach the envelope of the stimulation signal may be modulated by combinations of repetition rate domain and magnetic flux density domain.

Various envelopes of the stimulation signal and waveform, e.g. pulse, sinusoidal, rectangular, square, triangular, saw-tooth, trapezoidal, exponential etc. for the purpose of muscle stimulation may also be used, and is not limited to recited shapes of stimulation signals.

The values of magnetic flux density and repetition rate are cited in several preferred applications since the perception of the stimulation is subjective. Nevertheless, the magnetic flux density and repetition rates are not limited by the recited values. A person skilled in the physical therapy is able to repeat and apply the treatment methods adjusting the magnetic flux density and/or repetition rate following the patient's sensitivity or needs.

The present method is not limited to be used independently. For enhancing the result the method may be used in combination with other conventional non-invasive and/or invasive aesthetic medicine method.

All the recited methods may be applied to a patient in a non-invasive and/or contactless way. Therefore the present methods provide an effective alternative approach of enhancing the visual appearance with no need of invasive treatment or surgery. Further, the visual results are appreciable after several treatments. Additionally, the results include not only the visual appearance enhancement but even the improvement of the muscle structures, hence the patient feels firmer and tighter. The muscle structures become toned with no need of any diet or spending time by exercising in fitness.

Thus, novel systems and methods have been described. Various changes and substitutions may of course be made without departing from the spirit and scope of the invention. The invention, therefore, should not be limited, except by the following claims and their equivalents.

**Combined magnet+RF**

This sub-section of the third section discloses a methods which may be used for remodeling the adipose tissue, body shaping and/or contouring, muscle toning, skin tightening, skin rejuvenation, wrinkle removing, reducing stretchmarks, breast lifting, lip enhancement or treatment of cellulite in general by application of electromagnetic radiation to target structure to selectively heat the target tissue to remove and/or remodel adipose tissue from the target tissue. The second approach is to transmit a magnetic stimulation to the target structure, inducing at least partial muscle contraction within the target structure to remodel the adipose tissue by natural adipose tissue catabolism. Adipose tissue catabolism may be caused by apoptosis or necrosis of the adipocytes. The muscle contraction caused by induced eddy current is the same as a natural contraction. The adipose tissue may be reduced in natural way. Additionally, the muscle may be shredded in a natural way. Therefore the effect results in body shaping and/or contouring may be significantly improved.

The present invention discloses the advanced approaches in aesthetic applications, e.g. for cellulite treatment and/or body shaping. Combined methods of treatment by electromagnetic field and treatment by magnetic field are used. The electromagnetic field may include treatment by radiofrequency, infrared or optical waves. The magnet treatment may be provided by permanent magnets, electromagnetic devices generating a static magnetic field or time-varying magnetic field generating devices. In the preferred application the treatment by a pulsed magnetic field and radiofrequency treatment may be combined. However the application is not limited by the recited combination so the combined method may include magnet treatment and any treatment by electromagnetic field, e.g. light treatment, IR treatment or treatment by radiofrequency waves, e.g. microwaves, short waves or long waves.

The combination of the recited method may improve currently used applications in various aspects and the effect of the treatments may be significantly enhanced. The application of a radiofrequency electromagnetic field is combined with application of a magnetic field applied before, simultaneously or after the radiofrequency treatment. The application of a magnetic field induces many benefits for radiofrequency treatment, such as applications inducing at least partial muscle contraction, myorelaxation effect or analgesic effect. The perfusion or metabolism may be improved as well.

The at least partial muscle contraction may induce enhanced effects on adipose tissue reduction by catabolism of the adipose tissue and burning energy from adipose tissue. The total adipose tissue reduction effect is enhanced by radiofrequency treatment.

Additionally, the at least partial muscle contraction may improve a blood flow and/or perfusion in the treated area. The improved blood flow may be caused by activation of muscle pump and/or by the muscle necessity of more oxygen due to the at least partial contraction. Due to increased blood flow and/or local perfusion, the risk of overheated muscle is limited or even eliminated. Further the homogeneity of the thermal field induced by thermal effect of radiofrequency treatment may be significantly enhanced and/or the temperatures may be well-balanced/compensated in the target treatment area. Still another benefit is prevention of creation any hot spot caused by steep thermal gradient.

Due to improved blood flow, perfusion and/or lymph flow the metabolism may be improved. Additionally, the effect of radiofrequency treatment may be enhanced by improved metabolism, e.g. cellulite treatment, body shaping and/or contouring, skin tightening or skin rejuvenation. Further benefit may be reducing or eliminating the risk of panniculitis or local skin inflammation since any clustering of the treated adipocytes may be prevented by the improved metabolism. The improved blood and/or lymph flow may contribute the removing of the adipocytes. The removing of the adipocytes may be promoted by higher number of cells phagocytosing the adipocytes as well. Synergic effects of magnet and RF treatment significantly improves metabolism. Therefore the possibility of adverse event occurrence is limited and treatment results induced by the present invention are reached in shorter time period.

Further the at least partial muscle contraction may improve the movement of lymphatic vessel and the lymph flow may be improved.

In the preferred application the RF and/or magnetic field may be modulated. In the most preferred application both stimulation signals are modulated. The magnetic stimulation may be modulated in the magnetic flux density domain, repetition rate domain, or impulse duration domain, to provide different treatment effects and to prevent adaptation of the target biological structure. The radiofrequency treatment may be modulated in the frequency domain, intensity domain and/or time domain to reach the most complexity and/or efficiency of the target treated biological structure. The modulation in the time domain may be changing the active and passive periods of stimulation, e.g. the radiofrequency treatment may include period with no stimulation, i.e. the radiofrequency treatment is not continual but the treatment is provided in pulses. The periods of no stimulation may vary and may be adjusted by the operator. Due to modulation during the treatment, different target biological structures may be treated in the different depth.

The application may be contact or the preferred application of the invention the treatment may be applied contactless. Contactless application may avoid all biocompatibility factors which may occur during contact treatment. In the most preferred application the treatment may be provided by self-operated device. Hence the continual surveillance and/or control by the operator is not essential for correct and/or safe operation of the treatment device. Self-operated treatment may be provided by a handheld applicator or the applicator may be fixed to stand-alone device. The self-operated treatment may be also enabled using various types of sensors in communication with the device for monitoring the treatment and/or the patient. The at least one sensor may be e.g. reactive sensor, electrochemical sensor, biosensor, biochemical sensor, temperature sensor, sorption sensor, pH sensor, voltage sensor, sensor for measuring distance of applicator from the patient surface and/or from the treated area, position sensor, motion detector, photo sensor, camera, sound detector, current sensor, sensor for measuring of specific human/animal tissue and/or any suitable sensors measuring biological parameters and/or combination thereof such as sensor for measuring dermal tensile forces, sensor for measuring the activity of the muscle, muscle contraction forces, tissue impedance or skin elasticity.

Further the homogeneity of the treatment may be improved by several approaches. A first approach may be represented by a moveable applicator providing the dynamic treatment to a large target area. The dynamic treatment improves the homogeneity of applied treatment energy and additionally due to large area the effect is uniform and/or well balanced. Static positioning of the applicator may be used as well. Another approach of improving homogeneity may be represented by using a bolus. The bolus may provide improved transmittance of the electromagnetic energy to the treated biological structures. Additionally, the bolus may prevent occurrence of hot spots within the treated area; the bolus may provide constant temperature to the target treated surface area; or the bolus may increase the homogeneity of the radiofrequency waves application by providing a homogenous medium for electromagnetic waves propagation not being influenced by the interface of the target treated area and an air. The bolus may profile the electromagnetic field to enhance the effect of the treatment. In still another approach an air gap may be between the applicator and the patient.

The treatment by magnetic and/or electromagnetic field may be in continuous or discrete modes. In one application the magnetic treatment may be applied in continual mode with no pauses and the electromagnetic treatment may be applied in pulsed mode to provide improved adipose tissue reduction caused by natural process and by the increased temperature. In another application the electromagnetic treatment may be applied continuously with no pauses and the magnetic treatment may be applied in pulsed mode to provide improved thermal reduction of adipose tissue and by improved metabolism due to improved blood flow. Both modes may be combined in various treatment sequences.

In the preferred application the treatment is started at the moment when the target biological structure reaches the predetermined temperature. The temperature in the target tissue may be up to 80 °C, more preferably in the range of 37 to 60 °C, even more preferably in the range of 40 to 45 °C,. The temperature may be adjusted based on the intended use, e.g. adipose tissue reduction, collagen production or muscle contraction. In an alternative application the intended use may be coagulation and/or ablation. The temperature in the target biological structure may be measured by invasive method, e.g. using an invasive probe; or by contact method, e.g. using thermocouple sensor; or by contactless method, e.g. using infrared sensor or camera.

The temperature of the target biological structure may be determined by a mathematic method. The sensor for measuring the temperature in the target biological structure may be attached to the applicator.

A benefit of the application of magnet treatment and electromagnetic treatment may be causing an analgesic effect of the application and providing a possibility of treating a patient with higher sensitivity for thermal effects induced by electromagnetic treatment, i.e. patients with any predisposition inducing increased thermal sensitivity. The analgesic effect may be induced by magnet treatment by suitable repetition rates and it may be induced immediately during the magnet treatment. The analgesic effect may last up to several hours after magnet treatment. The magnetic flux density of the magnetic stimulation may preferably reach at least motor-threshold intensity inducing at least partial muscle contraction therefore the homogeneity of the thermal field is significantly enhanced.

Another benefit of application the magnet treatment may be causing a myorelaxation effect. The magnet treatment may be applied on spastic muscle structures to relieve the hypertonus of the muscle and improving the blood and/or lymph flow. Therefore relieving the hypertoned muscle may contribute to the analgesic effect and contribute to the acceptability of the treatment by the patient.

The blood and/or lymph flow may be limited in the spastic muscles and the metabolism may be limited as well, meaning that the risk of clustering the treated target structures may be higher and possible adverse events may occur. The recited risks may be eliminated by the used of magnet treatment.

**Magnet before RF**

In one aspect of the invention, the treatment by magnetic field may be applied to the target structure before the radiofrequency treatment to prepare the target structure for following treatment by radiofrequency field. The effect of magnet treatment is to induce at least partial muscle contraction or to stimulate a muscle structure to increase a muscular tonus of the target structure. Both effects may provide a massage effect for the structure within the proximity of the target structure hence the blood and/or lymph circulation may be improved to promote local metabolism. The temperature may be locally increased by the improved blood flow and the target structure may accept the following radiofrequency treatment at significantly higher quality. Additionally, the collagen and/or elastin fibers may be remodeled or restored and/or its neogenesis may be improved to provide a younger, smoother and enhanced skin appearance.

Additionally, previous application may improve acceptability of the electromagnetic field by increasing the temperature of the skin and the transmittance of the electromagnetic field may be improved due to less value of skin impedance. Further the radiofrequency may penetrate deeper target structures relative to treatment without a preceding magnet treatment of the target structure and/or area.

Another benefit may be releasing the adipose tissue in the muscle by muscle contraction and/or by temperature increase causing better liquidity of adipose tissue. Still another benefit of the at least partial muscle contraction may be mechanical breaking large adipose tissue bulks into smaller bulks which may be easier metabolism of the adipose tissue and/or the smaller adipose tissue bulks may be removed faster by the lymphatic and/or blood flow. Due to improved metabolism and/or circulation the cellulite may be treated in a short time and the visual effect on skin appearance may be significantly enhanced.

**Magnet during RF**

In another aspect of the invention, the treatment by magnetic field may be applied to the target structure simultaneously with the radiofrequency treatment to improve effects of the electromagnetic treatment inducing heat in the target structure.

The simultaneous application of magnet treatment and radiofrequency treatment may be in two modes: a first mode may generate the magnet impulses while radiofrequency treatment is active or another mode may generate radiofrequency treatment while the magnet treatment is not in an active stimulation period, i.e. the period of magnet treatment and radiofrequency treatment alternates. Both modes amplify the resulting effect of the treatment. Therefore the results are achieved in significantly shorter time than the same results achieved by separate applications of the radio frequency and magnet treatments.

The simultaneous method of magnet treatment and radiofrequency treatment of the target tissue may increase the peak magnetic component of the entire treatment resulting in improved heating of the target structure including containing higher water volume, e.g. skin. Due to increased temperature of skin, the production and/or remodeling of collagen and/or elastin fibers may be improved and the skin may be provided with a younger, smoother and enhanced appearance. The effect of overheating the muscle is reduced by the improved blood flow.

**Magnet after RF**

In still another aspect of the invention, the treatment by magnetic field may be applied to the target structure after the treatment by electromagnetic field to enhance and/or contribute to the effects of radiofrequency treatment by influencing the target structure by magnetic field.

The magnetic field may treat the target structure to cause at least partial muscle contraction proximate to the target structure to improve blood flow and provide homogenous temperature distribution at high quality after creating a temperature distribution at lower quality by radiofrequency treatment.

All of the methods may be provided by the above recited technical solutions. The above mentioned methods may be used separately or in any combination.

All the described applications of the invention may also provide trophotropic, antioedematous or placebo effect which contributes to improving the patient's well-being and comfort. Local metabolism may be increased as well.

All the mentioned applications may be applied in constant repetition rate and/or repetition frequency manner. The envelope modulation or combination thereof is applicable as well.

The values of magnetic flux density and repetition rate are cited in several preferred applications since the perception of the stimulation is subjective. Nevertheless, the magnetic flux density and repetition rates and/or repetition frequencies are not limited by the recited values. A person skilled in physical therapy is able to repeat and apply the therapy methods adjusting the magnetic flux density, repetition rate and/or frequency following the patient's needs. Further the applications may be used in combinations, e.g. diagnostic application and/or stimulation application.

A person skilled in the physical therapy is able to use various envelopes and waveform, e.g. pulse, sinusoidal, rectangular, square, triangular, saw-tooth, trapezoidal, exponential etc. The invention is not limited to recited shapes of envelopes.

Stimulation of biological structure by time-varying magnetic field following the recited methods may be but not limited to continuous, pulsed, randomized, or in bursts. The pulse may be but not limited to monophasic, symmetric, asymmetric, most preferably biphasic.

Moreover, the following aspects refer to preferred embodiments in the context of the invention:

1. A magnetic stimulation device which produces a time-varying magnetic field for treatment, wherein the device comprises:

a connection to an energy source, a switching device, a magnetic field generating device, an energy storage device; wherein the magnetic field generating device comprises insulated wires;
and a voltage drop in the energy storage device between successive amplitudes output from the energy storage device is not higher than 21 %.

2. The device of aspect 1 where a voltage drop in the energy storage device between the successive amplitudes is not higher than 14 %.

3. The device of aspect 1 where a voltage drop in the energy storage device between the successive amplitudes is not higher than 7 %.

4. The device of aspect 1 wherein the switching device is connected in parallel to the magnetic field generating device and the energy source.

5. The device of aspect 1 wherein the magnetic field generating device is flexibly attached to casing.

6. The device of aspect 4 having no protective resistors between the connection to the energy source and the energy storage device.

7. The device of aspect 4 including a blower on one side of the magnetic field generating device and positioned to blow air across the magnetic field generating device in a direction parallel to a plane of the magnetic field generating device.

8. The device of aspect 1 wherein the insulated wires have diameter lower than 0.5 mm.

9. The device of aspect 4 where the device enables treatment or successive treatments with peak to peak magnetic flux density of at least 1.5 T and with single magnetic pulses repeated at a repetition rate above 50 Hz.

10. The device of aspect 9 wherein the device enables repetition rates above 100 Hz with a magnetic flux density causing at least partial contraction of a muscle of a patient.

11. The device of aspect 1 where the device enables treatment or successive treatments with peak to peak magnetic flux density of at least 1.5 T at repetition rates above 70 Hz.

12. The device of aspect 1, wherein the device further comprises at least one blower and a casing; wherein the magnetic field generating device and the casing are arranged in a manner that fluid can flow in-between and wherein the magnetic field generating device is cooled by fluid flow over at least upper and lower sides of the magnetic field generating device.

13. The device of aspect 12 wherein the blower is arranged on a circumference of the magnetic field generating device.

14. The device of aspect 12 wherein the magnetic field generating device is a flat type magnetic field generating device.

15. The device of aspect 12 wherein the magnetic field generating device comprises insulated wires.

16. The device of aspect 12 wherein the magnetic field generating device is attached to the casing by at least one fastening point.

17. The device of aspect 16 wherein the at least one fastening point is located on an outer circumferential side of the magnetic field generating device.

18. The device of aspect 16 wherein the fastening point is flexible.

19. The device of aspect 16 wherein the fastening point is rigid.

20. The device of aspect 12 wherein spacing between the magnetic field generating device and the casing of applicator is at least 0.1 mm.

21. The device of aspect 12 further including an outlet on the upper side of the casing.

22. The device of aspect 12 wherein the fluid is air.

23. The device of aspect 22 wherein the fluid is cooled air.

24. A magnetic stimulation device producing a time-varying magnetic field for treatment, wherein the device comprises:
a connection to an energy source, a switching device, a magnetic field generating device producing time-varying magnetic field, and an energy storage device; wherein the switching device is connected in parallel to the energy source.

25. The device of aspect 24, wherein the switching device is able to short the energy source.

26. The device of aspect 24, wherein the energy storage device and magnetic field generating device are in serial connection.

27. The device of aspect 24 including a blower on one side of the magnetic field generating device and positioned to blow air across the magnetic field generating device in a direction parallel to a plane of the magnetic field generating device.

28. The device of aspect 27 including a blower on both sides of the magnetic field generating device.

29. The device of aspect 24 wherein the magnetic field generating device comprises insulated wires.

30. The device of aspect 29 having no protective resistors between the connection to the energy source and the energy storage device.

31. The device of aspect 29 wherein the insulated wires have a diameter lower than 3 mm.

32. The device of aspect 29 wherein the insulated wires have a diameter lower than 0.5 mm.

33. The device of aspect 24 wherein the magnetic field generating device is a flat type magnetic field generating device.

34. A magnetic stimulation device producing a time-varying magnetic field for treatment, wherein the device enables modulation of the time-varying magnetic field in magnetic flux density domain, repetition rate domain and/or impulse duration domain.

35. The device of aspect 34, wherein the device enables determining optimal value of at least one treatment parameter.

36. A magnetic stimulation device producing a time-varying magnetic field for treatment, wherein the device comprises:
a connection to an energy source, a switching device, a magnetic field generating device, an energy storage device; wherein the magnetic stimulation device enables treatment with peak to peak magnetic flux density of at least 1.5 T and with single magnetic pulses repeated at a repetition rate above 50 Hz.

37. The device of aspect 36, wherein the repetition rates are above 60 Hz.

38. The device of aspect 36, wherein the repetition rates are above 70 Hz.

39. The device of aspect 36, wherein the treatment last at least 10 seconds,

40. The device of aspect 36, wherein peak to peak magnetic flux density is at least 2,25 T.

41. The device of aspect 36, wherein the switching device is connected in parallel to the magnetic field generating device and the energy storage device, and with the device having a voltage drop between successive amplitudes output from the energy storage device not higher than 21%.

42. The device of aspect 36 further including a blower on one side of the magnetic field generating device and positioned to blow air across the magnetic field generating device in a direction parallel to a plane of the magnetic field generating device.

43. The device of aspect 41 having a repetition rate above 100 Hz and a magnetic flux density to cause at least partial muscle contraction in a muscle of a patient.

44. A magnetic stimulation device producing a time-varying magnetic field for treatment, wherein the device comprises:
a connection to an energy source, a switching device, a magnetic field generating device, an energy storage device; wherein the device enables repetition rates above 100 Hz with a magnetic flux density ensuring at least partial muscle contraction.

45. The device of aspect 44, wherein the repetition rates are above 150 Hz.

46. The device of aspect 44, wherein the repetition rates are above 200 Hz.

47. A magnetic stimulation device generating time-varying magnetic field for treatment of a patient, wherein device comprises:
an HF frequency generator, a switching device, a magnetic field generating device, an energy storage device; wherein the magnetic field generating device providing magnet treatment and radiofrequency treatment.

48. The magnetic stimulation device of aspect 47, wherein the magnetic field generating device is flat type magnetic field generating device.

49. The magnetic stimulation device of aspect 47, wherein the magnetic field generating device is an electrode for radiofrequency treatment.

50. The magnetic stimulation device of aspect 47, wherein the magnetic flux density generated by the magnetic stimulation device is at least 0,1 T.

51. The magnetic stimulation device of aspect 47, wherein the magnetic flux density generated by the magnetic stimulation device is at least 0.5 T.

52. The magnetic stimulation device of aspect 47, wherein the magnetic flux density generated by the magnetic stimulation device is at least 1 T.

53. The magnetic stimulation device of aspect 47, wherein the magnetic flux density generated by the magnetic stimulation device is at least 1.5 T.

54. The magnetic stimulation device of aspect 47, wherein a repetition rate is in the range of 1 to 300 Hz.

55. The magnetic stimulation device of aspect 47, wherein a frequency of radiofrequency treatment is in the range of 13 MHz to 3 GHz.

56. The magnetic stimulation device of aspect 47, wherein a frequency of radiofrequency treatment is around 13.56 or 40.68 or 27.12 MHz or 2.45 GHz.

57. The magnetic stimulation device of aspect 47, wherein the magnetic stimulation device provides magnet and radiofrequency treatment simultaneously.

58. The magnetic stimulation device of aspect 47, wherein the magnetic stimulation device provides alternating magnet and radiofrequency treatment.

59. A magnetic stimulation device including:
at least one applicator, at least one energy source, a plurality of switching devices, at least one energy storage device and a plurality of magnetic field generating devices, wherein the at least one switching device is electrically connected in parallel to the energy source.

60. The device of aspect 59, wherein the plurality of magnetic field generating devices are positioned with respect to each other.

61. The device of aspect 60, wherein the plurality of magnetic field generating devices are in one plane.

62. The device of aspect 60, wherein the plurality of magnetic field generating devices are in a plurality of planes.

63. The device of aspect 62, wherein at least one plane is tiltable and/or movable with respect to the at least one another plane.

64. The device of aspect 59, wherein the magnetic stimulation device includes a plurality of applicators.

65. The device of aspect 64, wherein the at least one magnetic field generating devices is in the at least one applicator.

66. The device of aspect 59, wherein the magnetic stimulation device includes at least one applicator, one energy source, one energy storage device, N magnetic field generating devices and N+1, wherein N is positive integer greater than 1.

67. The device of aspect 66, wherein the value of inductance of at least one magnetic field generating device differs from the value of inductance of at least one another magnetic field generating device.

68. The device of aspect 66, further including adjusting total equivalent inductance of the magnetic stimulation device based on the number of active magnetic field generating devices used.

69. A magnetic stimulation device including at least one applicator, at least one energy source, a plurality of switching devices, at least one energy storage device and a plurality of magnetic field generating devices, enable moving of the at least one magnetic field generating device.

70. The device of aspect 69, wherein the movement of the at least one magnetic field generating device is constant and/or accelerated.

71. The device of aspect 69, wherein the movement of the at least one magnetic field generating device is translational and/or rotational.

72. The device of aspect 69, wherein the movement of the at least one magnetic field generating device is random.

73. The device of aspect 69, wherein the movement of the at least one magnetic field generating device follows a predetermined trajectory.

74. The device of aspect 73, wherein the predetermined trajectory is a pattern, an array or a matrix.

75. A magnetic stimulation device including at least one applicator, at least one energy source, a plurality of switching devices, a plurality of energy storage devices and first and second magnetic field generating devices enabling:

generating impulses via the first magnetic field generating device; and
generating impulses via the second magnetic field generating device independently of the impulses generated by the first magnetic field generating device.

76. The device of aspect 75, wherein the energy storage devices are recharged by the at least one energy source simultaneously or at different times within one pulse.

77. The device of aspect 75, wherein the first magnetic field generating device generates a first magnetic field of different treatment parameters from the a second magnetic field generated by the second magnetic field generating device.

78. The device of aspect 75, wherein the first and second magnetic field generation devices generates impulses at one time within one pulse.

79. The device of aspect 78, wherein the first and second magnetic fields interfere.

80. The device of aspect 75, wherein the first and second magnetic field generation devices generate impulses in a plurality of different times within one pulse.

81. The device of aspect 75, wherein myostimulation, myorelaxation, analgesic effect or at least one aesthetic effect is achieved.

82. The device of aspect 75 further comprising positioning a treatment target area of a patient at an intersection of the magnetic fields generated by the plurality of magnetic field generating devices.

83. The device of aspect 75, wherein the magnetic stimulation device includes at least one applicator, one energy source, N energy storage devices, N magnetic field generating devices and 2N switching devices, wherein N is positive integer greater than 1.

84. The device of aspect 75, wherein the magnetic stimulation device includes at least one applicator, N energy sources, N energy storage devices, N magnetic field generating devices and N switching devices, wherein N is positive integer greater than 1.

85. A magnetic stimulation device including at least one applicator, at least one energy source, a plurality of switching devices, at least one energy storage device and a plurality of magnetic field generating devices, enabling generating a plurality of peaks of magnitudes of magnetic flux density using a plurality of magnetic field generating devices.

86. The device of aspect 85, wherein the plurality of magnetic field generating devices treats a large area.

87. A magnetic stimulation device for treating a biological structure by time-varying magnetic field enabling:

determining at least one value of operation parameter in at least one value of a characteristic quantity, wherein the value of operation parameter is related with at least one of:

a) a calibration curve,
b) a calibration value,
c) at least one value of the same operation parameter in the different value of the same characteristic quantity,

wherein the calibration curve and/or the calibration value may be determined in the same value or in the different value of the same characteristic quantity.

88. The method of aspect 87 wherein the relation is determined by mathematic and/or signal processing method.

89. The method of aspect 88, wherein the at least one treatment parameter is limited and/or the treatment is disabled if the relation exceeds a predetermined threshold.

90. The method of aspect 88, wherein the at least one value of relation is established by factory settings and/or by an operator.

91. The method of aspect 88, wherein the value of relation determines an unintended event.

92. The method of aspect 91, wherein the unintended event is a metal object within proximity of the magnetic stimulation device and/or a hardware error of the magnetic stimulation device.

93. The method of aspect 89, wherein the limited treatment parameter and/or disabled treatment protects the patient and/or the magnetic stimulation device from heat damage.

94. The method of aspect 89, wherein the limited treatment parameter and/or disabled treatment protects the patient from injury caused by movement of a metal object.

95. The method of aspect 91, wherein the unintended event is notified in a human perceptible form.

96. The method of aspect 95, wherein the notification in human perceptible form is generated by audible and/or visual means.

97. The method of aspect 87, wherein the calibration curve is established as factory settings.

98. The method of aspect 87, wherein the calibration curve is determined by mathematic and/or signal processing method of the at least one operation parameter waveform.

99. The method of aspect 98, wherein the calibration curve is determined from at least ten waveforms.

100. The method of aspect 87, wherein the calibration curve and/or calibration value is established as factory settings in value of operation parameter measured during standard ambient conditions.

101. The method of aspect 88, wherein the relation is determined by at least one predetermined point of a currently measured waveform.

102. The method of aspect 101, wherein the at least one predetermined point is set as the factory settings.

103. The method of aspect 101, wherein the at least one predetermined point is set by the operator.

104. The method of aspect 101, wherein the predetermined points are first and second maximum of the measured waveform.

105. The method of aspect 88, wherein the relation corresponds to heat generation.

106. The method of aspect 105, wherein the actual heat is determined by mathematic and/or signal processing method.

107. The method of aspect 105, wherein the actual heat is determined by relation of the value of operation parameter in first and second maximums values of the measured waveform.

108. The method of aspect 88, wherein a magnetic field generating device is operated to maximally reach a predetermined temperature of the magnetic stimulation device.

109. The method of aspect 108, wherein a transition thermal characteristic of the magnetic stimulation device is determined.

110. The method of aspect 109, wherein the transition thermal characteristic is a thermal function of the magnetic field generating device.

111. The method of aspect 108, wherein the at least one treatment parameter is adjustable by the operator and wherein the at least one treatment parameter may be suggested by the magnetic stimulation device to the operator.

112. The method of aspect 111, wherein the magnetic stimulation device determines the at least one maximal treatment parameter based on actual temperature of the applicator and/or the at least one of the other treatment parameters.

113. The method of aspect 108, wherein the maximal predetermined temperature of the magnetic stimulation device is adjustable by the operator.

114. The method of aspect 113, wherein the magnetic stimulation device operates the cooling medium flow to sufficiently cool the applicator and/or the magnetic field generating device to reach the adjusted and/or predetermined temperature.

115. The method of aspect 108, wherein an actual temperature of the applicator and/or the magnetic field generating device is determined by the at least one of actual ambient air temperature, cooling medium temperature, magnetic flux density, repetition rate, impulse duration or treatment duration.

116. The method of aspect 105, wherein the heat is determined by the relation of the calibration curve and currently measured operation parameter waveform.

## Claims

1. A magnetic stimulation device for treatment of a patient using a time-varying magnetic field, wherein the magnetic stimulation device comprises:

   a first energy source (14), a first energy storage device (13), a first switching device (11), and a first applicator, the first applicator comprises the first magnetic field generating device (12) within a casing (7),

   wherein the first magnetic field generating device (12) is flat and circular,
   wherein the first magnetic field generating device (12) is oriented within the casing (7) such that the first magnetic field generating device (12) is spaced apart from the casing (7) by a gap in a distance of at least 0.1 mm,
   wherein the casing (7) comprises:

   an inlet and an outlet,
   a first side configured to be positioned proximate to the patient,
   a second side configured to be positioned opposite to the first side such that the second side is configured to be positioned farther from the patient than the first side,
   wherein the second side comprises the outlet configured to direct air out of the casing (7);

   a blower configured to direct air into the casing (7) such that the air flows through the gap in-between the first magnetic field generating device (12) and the first side of the casing (7),

   wherein the blower is positioned within the casing (7) such that the blower is positioned around at least one point of a circumference of the first magnetic field generating device (12);
   wherein the first energy source (14) is configured to charge the first energy storage device (11),
   wherein the first magnetic field generating device (12) is connected to the first energy storage device (13) and to the first switching device (11);
   wherein the first switching device (11) is configured to be switched in order to enable a discharge of energy from the first energy storage device (13) to the first magnetic field generating device (12) such that the first magnetic field generating device (12) generates an impulse of the time-varying magnetic field,

> wherein the impulse has a peak-to peak magnetic flux density in a range of 0.2 Tesla to 7 Tesla on a surface of the first magnetic field generating device (12), and a repetition rate in a range of 1 Hz to 700 Hz;
> wherein the time-varying magnetic field comprises biphasic impulse;
> wherein the time-varying magnetic field is configured to be applied to a body of the patient.

2. The magnetic stimulation device according to claim 1,
   **characterized in that**
   the first magnetic field generating device (12) comprises at least one insulated wire.

3. The magnetic stimulation device according to any preceding claim,
   **characterized in that**
   the first magnetic field generating device (12) comprises a litz-wire.

4. The magnetic stimulation device according to any preceding claim,
   **characterized in that**
   the first magnetic field generating device (12) is configured to generate impulses of the time-varying magnetic field for a plurality of periods of different repetition rates.

5. The magnetic stimulation device according to any preceding claim,
   **characterized in that**
   the first magnetic field generating device (12) is configured to establish at least one pulse of the time-varying magnetic field, wherein the pulse comprises the impulse and wherein the pulse lasts a time period between two impulses from rise/fall edge to subsequent rise/fall edge,
   wherein the first magnetic field generating device (12) is configured to assemble a plurality of pulses into a trapezoidal envelope.

6. The magnetic stimulation device according to any preceding claim,
   **characterized in that**

   > the first magnetic field generating device (12) is configured to be coupled to the casing (7) by at least one fastening point (3),
   > wherein the at least one fastening point (3) is flexible, and
   > wherein the at least one fastening point (3) is positioned around at least a part of the circumference of the magnetic field generating device (12).

7. The magnetic stimulation device according to any preceding claim,
   **characterized in that**
   the casing is provided as a chair.

8. The magnetic stimulation device according to any preceding claim,
   **characterized in that**
   the blower is configured to direct the air in a gap in between the magnetic field generating device (12) and the second side of the casing (7).

9. The magnetic stimulation device according to any preceding claim,
   **characterized in that**

   > the stimulation device further comprises a second magnetic field generating device, and
   > wherein each of the first magnetic field device and the second magnetic field generating device is configured to generate time-varying magnetic fields.

10. The magnetic stimulation device according claim 9,
    **characterized in that**
    the time-varying magnetic field generated by the first magnetic field generating device interferes with a second time-varying magnetic field generated by the second magnetic field generating device.

**11.** The magnetic stimulation device according to claim 9,
**characterized in that**
wherein the first magnetic field generating device is located in the first applicator and the second magnetic field generating device is located in a second applicator.

**12.** The magnetic stimulation device according to claim 11,
**characterized in that**
the first applicator and the second applicator are adapted to be independently positionable with respect to each other.

**13.** The magnetic stimulation device according to claim 9 or 12,
**characterized in that**

wherein the first switching device is configured to enable discharging of the first energy storage device to the first magnetic field generating device, and
wherein a second switching device is configured to enable discharging of a second energy storage device to the second magnetic field generating device.

**14.** The magnetic stimulation device according to claim 9 to 12,
**characterized in that**
wherein the first switching device is configured to discharge the first energy storage device to provide electric current to the first magnetic field generating device and to the second magnetic field generating device.

**15.** The magnetic stimulation device according to claim 9 to 12,
**characterized in that**
wherein the stimulation device further comprises a second switching device configured to enable providing electric current from the first energy storage device to the second magnetic field generating device.

Figure 1

Figure 2

Figure 3

Figure 4A

Figure 4B

Figure 5

Figure 6

Figure 7

Figure 8

Figure 9

C ⌒ 41

40

42

PS    SW ⌒ 43    L

Figure 10

Figure 11

Figure 12A

Figure 12B

Figure 12C

Figure 12D

Figure 12E

Figure 12F

Figure 12G

Figure 13

Figure 14

Figure 15

Figure 16

Figure 17

MFD

92

92

92

92

t

Figure 18A

IE

93

t

Figure 18B

Figure 19A

Figure 19B

MFD

92    92    92    92    92

t

Figure 20A

IE

93

t

Figure 20B

Figure 21

Figure 22

MFD

... 92 ...

Figure 23A

IE

101

t

Figure 23B

Figure 24A

Figure 24B

Figure 24C

Figure 25

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2015025299 A
- US 2015157873 A
- US 201264942 W

**Non-patent literature cited in the description**

- **HEISEL**. Jürgen. Physikalische Medizin. Stuttgart. Georg Thieme Verlag, 2005, 159
- **BRONZINO, JOSEPH, D.** The Biomedical Engineering Handbook. CRC Press LLC, 2000, vol. I, 91-1, 91-8